(19) Europäisches Patentamt
European Patent Office
Office européen des brevets

(11) Veröffentlichungsnummer: **0 206 037 B1**

(12) **EUROPÄISCHE PATENTSCHRIFT**

(45) Veröffentlichungstag der Patentschrift: **15.01.92**

(51) Int. Cl.$^5$: **C07H 13/04**, A61K 31/70, A61K 39/39, C07H 13/12, C07H 15/04

(21) Anmeldenummer: **86107665.1**

(22) Anmeldetag: **05.06.86**

(54) **N-(2-Aminoacylamido-2-desoxy-hexosyl)-amide, -carbamate und -harnstoffe, Verfahren zu ihrer Herstellung sowie ihre Verwendung in Arzneimitteln.**

(30) Priorität: **20.06.85 DE 3521994**

(43) Veröffentlichungstag der Anmeldung:
**30.12.86 Patentblatt 86/52**

(45) Bekanntmachung des Hinweises auf die Patenterteilung:
**15.01.92 Patentblatt 92/03**

(84) Benannte Vertragsstaaten:
**AT BE CH DE FR GB IT LI LU NL SE**

(56) Entgegenhaltungen:
**EP-A- 0 091 645**

**CARBOHYDRATE RESEARCH, Band 5, Nr. 1, 1967, Seiten 82-92; Elsevier Publ. Co., BE. J YOSHIMURA et al.: "Studies on 2-amino-2-deoxy-D- glucose derivatives. Part XV. Synthesis of 1-N-acyl-2-acylamido-2-deoxy-beta-D-glucopyranosylamines."**

(73) Patentinhaber: **BAYER AG**

**W-5090 Leverkusen 1 Bayerwerk(DE)**

(72) Erfinder: **Lockhoff, Oswald, Dr.**
**Morgengraben 14**
**W-5000 Köln 80(DE)**
Erfinder: **Hayauchi, Yutaka, Dr.**
**Gustav-Freytag-Strasse 4**
**W-5090 Leverkusen 1(DE)**
Erfinder: **Stadler, Peter, Dr.**
**Am Ideck 2**
**W-5657 Haan 1(DE)**
Erfinder: **Stünkel, Klaus Georg, Dr.**
**Am Eckbusch 55/12**
**W-5600 Wuppertal 1(DE)**
Erfinder: **Streissle, Gert, Dr.**
**Gellertweg 22**
**W-5600 Wuppertal 1(DE)**
Erfinder: **Paessens, Arnold, Dr.**
**Stresemannstrasse 56**
**W-5657 Haan 1(DE)**

EP 0 206 037 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

Erfinder: **Klimetzek, Dr.**
**Kant-Strasse 64**
**W-5620 Velbert-Tönisheide(DE)**
Erfinder: **Zeiler, Hans-Joachim, Dr.**
**Elsbeeker Strasse 46**
**W-5620 Velbert 15(DE)**
Erfinder: **Metzger, Karl Georg, Dr.**
**Pahlkestrasse 75**
**W-5600 Wuppertal 1(DE)**
Erfinder: **Kroll, Hein-Peter, Dr.**
**Pahlkestrasse 96**
**W-5600 Wuppertal 1(DE)**
Erfinder: **Brunner, Prof. Dr.**
**Waldstrasse 10**
**W-4018 Langenfeld(DE)**
Erfinder: **Schaller, Klaus, Dr.**
**Am Sonnenschein 38**
**W-5600 Wuppertal 1(DE)**

## Beschreibung

Die Erfindung betrifft neue N-Glycosylamide, N-Glycosylharnstoffe und N-Glycosylcarbamate, die im Zuckerrest mit einer Aminosäure substituiert sind, sowie Verfahren zu ihrer Herstellung und ihre Verwendung als Arzneimittel.

In Carbohydrate Research 5(1), 1967, Seiten 82-92 sind N-(2-Aminoacylamido-2-desoxy-hexosyl)-amide beschrieben.

Die neuen Verbindungen entsprechen der allgemeinen Formel I

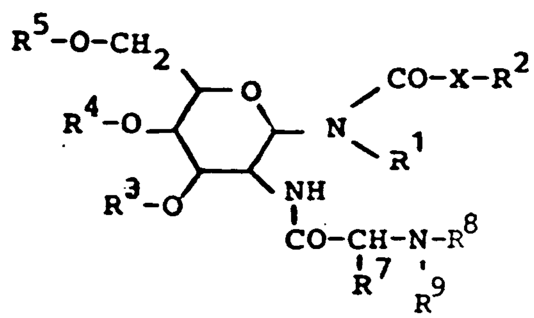

(I)

in der

| | |
|---|---|
| $R^1$ | einen gesättigten oder einfach oder mehrfach ungesättigten Alkylrest mit bis zu 50 Kohlenstoffatomen bedeutet, |
| X | für $-CH_2$, -O- oder -NH- steht, |
| $R^2$ | Wasserstoff oder einen gesättigten oder einfach oder mehrfach ungesättigten Alkylrest mit bis zu 50 Kohlenstoffatomen bedeutet, |
| $R^3$, $R^4$ und $R^5$ | unabhängig voneinander Wasserstoff oder Acyl $-CO-R^6$ bedeutet, wobei unter $R^6$ ein Alkylrest mit einem bis zu 10 Kohlenstoffatomen zu verstehen ist, |
| $R^7$ | Wasserstoff, $C_1$-$C_7$-Alkyl, Hydroxymethyl, 1-Hydroxyethyl, Mercaptomethyl, 2-(Methylthio)-ethyl, 3-Aminopropyl, 3-Ureidopropyl, 3-Guanidylpropyl, 4-Aminobutyl, Carboxymethyl, Carbamoylmethyl, 2-Carboxyethyl, 2-Carbamoylethyl, Benzyl, 4-Hydroxybenzyl, 3-Indolylmethyl oder 4-Imidazolylmethyl bedeutet, und |
| $R^8$ | Wasserstoff oder Methyl, bedeutet und |
| $R^9$ | für Wasserstoff, Acetyl, Benzoyl, Trichloracetyl, Trifluoracetyl, Methoxycarbonyl, t-Butyloxycarbonyl und Benzyloxycarbonyl steht und |
| $R^7$ und $R^8$ | zusammen $-CH_2-CH_2-CH_2-$ bedeuten können. |

Die Stereochemie an dem chiralen Zentrum in der $\alpha$-Aminosäure ist entweder L oder D.

Der Rest $R^1$ stellt bevorzugt einen geradkettigen oder verzweigten, gesättigten oder ungesättigten Alkylrest mit bis zu 20 C-Atomen dar, besonders bevorzugt mit 10 bis 20 C-Atomen.

Beispiele für geradkettige, gesättigte Alkylreste $R^1$ sind Methyl, Ethyl, Propyl, Butyl, Pentyl, Hexyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Eicosyl, Docosyl, Tetracosyl, Hexacosyl, Octacosyl und Triacontyl.

Ungesättigte Reste $R^1$ sind beispielsweise Vinyl, Allyl, 2-Butenyl, 3-Butenyl, 2-Hexenyl, 3-Hexenyl, 4-Hexenyl, 5-Hexenyl, 2-Octenyl, 4-Octenyl, 6-Octenyl, 7-Octenyl, 2-Decenyl, 4-Decenyl, 6-Decenyl, 8-Decenyl, 9-Decenyl, 2-Dodecenyl, 6-Dodecenyl, 10-Dodecenyl, 11-Dodecenyl, 4-Tetradecenyl, 6-Tetradecenyl, 10-Tetradecenyl, 6-Hexadecenyl, 8-Hexadecenyl, 10-Hexadecenyl, 12-Hexadecenyl, 6-Heptadecenyl, 8-Heptadecenyl, 10-Heptadecenyl, 6-Octadecenyl, 8-Octadecenyl, 10-Octadecenyl, 8,11-Heptadecandienyl oder 8,11,14-Heptadecantrienyl.

Im allgemeinen sind unter den ungesättigten Resten die längerkettigen bevorzugt, speziell die einfach oder zweifach ungesättigten mit 10 bis 20 Kohlenstoffatomen.

Die Reste $R^1$ können auch verzweigte, gesättigte oder einfach oder zweifach ungesättigte Alkylreste sein. Hierbei sind als Alkylsubstituenten der Alkyl- oder Alkenylkette solche Alkylreste bevorzugt, die bis zu zwölf Kohlenstoffatome aufweisen.

Beispiele für verzweigte Alkylreste $R^1$ sind 2-Methyldodecyl, 4-Methyldodecyl, 6-Methyldodecyl, 8-Methyldodecyl, 11-Methyldodecyl, 4-Ethyldodecyl, 8-Ethyldodecyl, 2-Methyltetradecyl, 4-Methyltetradecyl, 10-Methyltetradecyl, 13-Methyltetradecyl, 2-Methylhexadecyl, 4-Methylhexadecyl, 8-Methylhexadecyl, 15-Methylhexadecyl, 1-Methyloctadecyl, 2-Methyloctadecyl, 4-Methyloctadecyl, 10-Methyloctadecyl, 16-Methyloctadecyl, 17-Methyloctadecyl, 1-Butyldodecyl, 1-Dodecyldodecyl, 1-Decyltetradecyl und 1-Dodecylhexadecyl.

Der Rest $R^2$ stellt bevorzugt Wasserstoff oder einen geradkettigen oder verzweigten, gesättigten oder ungesättigten Alkylrest mit bis zu 20 C-Atomen dar, besonders bevorzugt mit 8 bis 20 C-Atomen.

Beispiele für die Reste $R^2$ sind die bei $R^1$ genannten Reste.

Wie in Formel I ersichtlich ist, liegt den erfindungsgemäßen Verbindungen eine substituierte 2-Amino-2-desoxyhexose zugrunde. Diese Zucker sind immer über C-1, das anomere Kohlenstoffatom, N-glycosidisch mit der Acylamido-, Carbamido- oder Alkoxycarbonylamidogruppierung

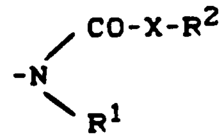

mit den oben angegebenen Bedeutungen für $R^1$, $R^2$ und X verbunden.

Bevorzugte Aminozucker in den erfindungsgemäßen Verbindungen der Formel I sind 2-Amino-2-desoxy-D-glucose und 2-Amino-2-desoxy-D-galactose.

Die 2-Aminogruppe der genannten Aminozucker in den erfindungsgemäßen Verbindungen der Formel I ist amidisch mit einer α-Aminosäure oder einem α-Aminosäurederivat verbunden.

Bevorzugte Aminosäuren sind die natürlichen L-Aminosäuren wie Glycin, Sarcosin, Hippursäure, Alanin, Valin, Leucin, Isoleucin, Serin, Threonin, Cystein, Methionin, Ornithin, Citrullin, Arginin, Asparginsäure, Asparagin, Glutaminsäure, Glutamin,Phenylalanin, Tyrosin, Prolin, Tryptophan, Histidin. Es können aber auch D-Aminosäuren wie D-Alanin oder Aminocarbonsäuren wie α-Aminobuttersäure, α-Aminovaleriansäure, α-Aminocapronsäure oder α-Aminoheptansäure, sowohl in der D- als auch in der L-Form als Substituenten an dem Aminozucker fungieren.

Die Erfindung betrifft auch Verfahren zur Herstellung der Verbindungen gemäß Formel I. Hierbei geht man aus von einem an der Aminogruppe geschützten 2-Amino-2-desoxyglycopyranosederivat II,

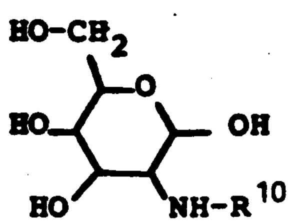

(II)

in dem

$R^{10}$ eine für den Schutz von Aminogruppen aus der Synthese von Peptiden her bekannte und gegebenenfalls selektiv abspaltbare Schutzgruppe darstellt.

Geeignete Schutzgruppen sind z.B. Acylgruppen wie Trifluoracetyl oder Trichloracetyl, o-Nitrophenylsulfenyl, 2,4-Dinitrophenylsulfenyl oder gegebenenfalls substituiertes Niederalkoxycarbonyl wie Methoxycarbonyl, t-Butyloxycarbonyl, Benzyloxycarbonyl, p-Methoxybenzyloxycarbonyl oder 2,2,2-Trichlorethyloxycarbonylgruppen, also allgemein solche Gruppen, die sich in Peptiden selektiv wieder abspalten lassen können. Geeignete N-geschützte Aminohexosederivate 11 sind prinzipiell bekannt (Lit. z.B. M. Bergmann und L. Zervas, Ber. 64, 975 (1931): D. Horton, J. Org. Chem. 29, 1776 (1964): P.H. Gross und R.W. Jeanloz, J.Org. Chem. 32, 2759 (1967): M.L.Wolfrom und H.B.Bhat, J.Org. Chem. 32, 1821 (1967): allgemein: J.F.W. McOmie (Hrsg). Prot. Groups. Org.Chem., Plenum Press (1973): Geiger in "The Peptides" Vol. 3, p 1-99, (1981) Academic Press; und dort zitierte Literatur).

Besonders bevorzugte Aminoschutzgruppen für die Herstellung der Verbindungen gemäß Formel I sind die BOC-Gruppe (tert. Butyloxycarbonyl) oder die Z-Gruppe (Benzyloxycarbonyl).

Die blockierten Aminozuckerderivate II werden in einem ersten Reaktionsschritt mit Aminen III,

$H_2N-R^1$ (III)

wobei

$R^1$ die oben angegebene Bedeutung hat, zu Glycosylaminen IV

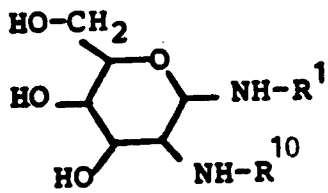

(IV)

umgesetzt.

Derartige Darstellungen von Glycosylaminen sind prinzipiell bekannt (ELLIS, Advances in Carbohydrate Chemistry 10, 95 (1955)) und sind speziell in der DE-OS 3 213 650 beschrieben.

In dem zweiten Reaktionsschritt werden die Glycosylamine IV entweder mit geeigneten Carbonsäurederivaten V wie Carbonsäurehalogeniden oder Carbonsäureanhydriden

$R^{11}$-CO-$CH_2$-$R^2$ (V)

umgesetzt, wobei

$R^2$ die oben angegebene Bedeutung hat und $R^{11}$ für Halogen wie z.B. Chlor oder für -O-CO-$R^2$ mit der oben angegebenen Bedeutung für $R^2$ oder für -O-CO-O-Niederalkyl, steht.

Auf diese Weise gelangt man zu Glycosylamiden VI

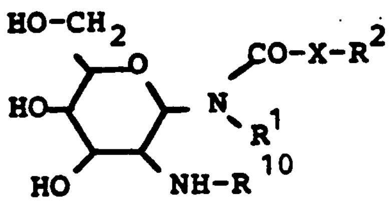

(VI)

in denen

$R^1$, $R^2$ und $R^{10}$ die oben angeführten Bedeutungen aufweisen und X für -$CH_2$- steht.

Die Bedingungen für derartige N-Acylierungen sind in der DE-OS 3 213 650 angegeben.

In einer bevorzugten Ausführungsform werden die Glycosylamine der Formel IV mit einem bis zwei Äquivalenten eines Carbonsäurechlorides V oder mit einem bis zwei Äquivalenten eines gemischten Anhydrides, welches aus der betreffenden Carbonsäure $R^2$-$CH_2$-$CO_2H$ und Chlorameisensäureethylester oder Chlorameisensäureisobutylester in Gegenwart von einer organischen Hilfsbase nach Literaturbekannten Methoden gewonnen worden ist, zum Glycosylamid VI mit X = -$CH_2$- umgesetzt.

Man arbeitet in organischen oder wäßrig-organischen Lösungsmitteln zwischen 0° C und 50° C, gegebenenfalls in Gegenwart einer anorganischen oder organischen Base. Geeignete Verdünnungsmittel sind Alkohole wie Methanol, Ethanol, 1-Propanol oder 2-Propanol, oder Ether wie Diethylether, Tetrahydrofuran oder 1,4-Dioxan, oder Halogenkohlenwasserstoffe wie Dichlormethan, Trichlormethan, 1,2-Dichlorethan oder N,N-Dimethylformamid.

Werden die im ersten Schritt erhaltenen Glycosylamine IV mit Halogenameisensäureester VII

$R^{12}$-CO-O-$R^2$ (VII)

umgesetzt, wobei

$R^{12}$ für Halogen wie z.B. Chlor oder Brom steht und $R^2$ die oben angegebene Bedeutung hat,

so gelangt man zu Glycosylcarbamaten VI, wobei $R^1$, $R^2$ und $R^{10}$ in Formel VI die oben angegebene Bedeutung haben und X in Formel VI für Sauerstoff steht.

In einer bevorzugten Ausführungsform werden die Glycosylamine der Formel IV mit einem bis zwei Äquivalenten eines Chlorkohlensäureesters VII zum Glycosylcarbamat umgesetzt. Man arbeitet bevorzugt in organischen oder wäßrig-organischen Lösungsmitteln bei Temperaturen zwischen 0° C und 50° C, besonders bevorzugt jedoch bei Raumtemperatur. Geeignete Lösungsmittel sind Alkohole, Ether, Halogenkohlenwasserstoffe oder Dimethylformamid, so wie sie oben genannt sind.

Werden die im ersten Schritt erhaltenen Glycosylamine IV mit ein bis zwei Äquivalenten eines organischen Isocyanates VIII

$R^2$-NCO (VIII)

umgesetzt, wobei

$R^2$ die oben genannte Bedeutung hat,

erhält man Glycosylharnstoffe der Formel VI, wobei $R^1$, $R^2$ und $R^{10}$ die oben angeführten Bedeutungen aufweisen und X für -NH- steht.

Diese Acylierungsreaktion wird wie die oben genannten Umsetzungen bevorzugt in organischen Lösungsmitteln durchgeführt, wobei die Reaktionstemperaturen zwischen -20 °C und 60 °C, bevorzugt zwischen 0 °C und 25 °C liegen.

Geeignete Lösungsmittel sind die oben genannten Alkohole, Ether, Halogenkohlenwasserstoffe oder Dimethylformamid.

Die auf diese Weise erhaltenen Glycosylamide (VI, X = $-CH_2-$), Glycosylcarbamate (VI, X = -O-) oder Glycosylharnstoffe (VI, X = -NH-) werden nach an sich bekannten Verfahren in Form von kristallinen oder amorphen Feststoffen isoliert und wenn nötig, durch Umkristallisation, Chromatographie, Extraktion usw. gereinigt.

In vielen Fällen ist es auch günstig, parallel zu oder anstelle der oben angeführten Aufreinigungsschritte eine chemische Derivatisierung vorzunehmen, die zu einem Derivat der Glycosylamide,-carbamate und -harnstoffe VI mit den oben angeführten Bedeutungen für $R^1$, $R^2$ , $R^{10}$ und X führt, das gute Kristallisationseigenschaften hat. Solche chemischen Derivatisierungen sind im Falle der erfindungsgemäßen Glycosylamide, Glycosylcarbamate und Glycosylharnstoffe z. B. Veresterungsreaktionen an den Hydroxylgruppen der Zuckerreste.

Geeignete Estergruppierungen sind z.B. Acetyl-, Benzoyl- oder p-Nitrobenzoylgruppen.

Zur Herstellung der Tri-O-acylderivate der Glycosylamide, Glycosylharnstoffe oder Glycosylcarbamate werden die entsprechenden Triole VI in Gegenwart von anorganischen oder organischen Hilfsbasen mit Acylierungsmitteln umgesetzt. Geeignete Acylierungsmittel sind Säurechloride wie Acetylchlorid, Benzoylchlorid oder p-Nitrobenzoylchlorid oder Anhydride wie z.B. Acetanhydrid. Dabei entstehen die Ester gemäß Formel IX

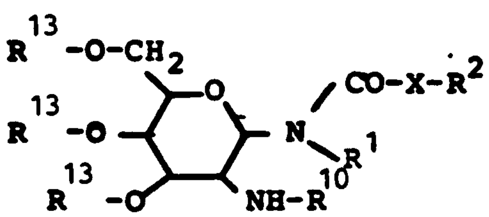

(IX)

wobei

$R^1$, $R^2$, $R^{10}$ und X die oben genannte Bedeutung haben und

$R^{13}$ für Acetyl, Benzoyl oder p-Nitrobenzoyl steht.

Die O-Acylierungsreaktionen werden bevorzugt in organischen, inerten Lösungsmitteln durchgeführt. Als solche seien genannt Halogenkohlenwasserstoffe wie Dichlormethan, Trichlormethan oder 1,2-Dichlorethan, Ether wie Tetrahydrofuran oder 1,4-Dioxan, Ester wie Essigsäureethylester, Amide wie Dimethylformamid. Als geeignete Lösungsmittel können auch die organischen Basen alleine zugegeben werden, wie Triethylamin oder Pyridin.

Als Basen können alle in der organischen Chemie für O-Acylierungen verwendeten Basen benutzt werden. Bevorzugt werden Triethylamin, Pyridin oder das Gemisch Pyridin/4-Dimethylaminopyridin benutzt.

Die Triester IX lassen sich gut aus organischen Lösungsmitteln kristallisieren. Besonders bevorzugt für die Kristallisation sind polare Lösungsmittel wie kurzkettige Alkohole, d.h. Methanol, Ethanol, n-Propanol oder Isopropanol. Andere für die Kristallisation der Triester IX geeignete Lösungsmittel sind Gemische von organischen Lösungsmitteln mit polaren anorganischen oder organischen Lösungsmitteln, z.B. Tetrahydrofuran-Methanol, Tetrahydrofuran-Wasser, Ethanol-Wasser, Isopropanol-Wasser.

Die durch einfach oder gegebenenfalls mehrfache Umkristallisation aufgereinigten Triester IX werden durch Verseifung oder Umesterung der drei O-Acetylgruppen in die Triole VI zurückgeführt.

Esterspaltungen sind in der organischen Chemie in vielfältiger Form bekannt. Zur Darstellung der Triole VI aus den Triestern IX sei die Umesterung der Acylgruppen in Gegenwart von Methanol und katalytischen Mengen Natriummethanolat genannt, die in der organischen Chemie als ZEMPLEN-Verseifung bekannt ist.

Der dritte Reaktionsschritt in der Herstellung der erfindungsgemäßen Verbindungen der Formel I

besteht in der selektiven Spaltung der Schutzgruppe der 2-Aminogruppe am Zucker in den Verbindungen der Formel VI. Bei dieser Reaktion ist besonders dafür Sorge zu tragen, daß nicht auch gleichzeitig die 1-Amido-, bzw. die 1-Carbamido bzw. die 1-(Alkoxycarbonylamido)-Gruppe am Zucker in den Verbindungen der Formel VI abgespalten wird.

Die bevorzugt verwendete Benzyloxycarbonylgruppe an C-2 der Aminohexose läßt sich quantitativ und selektiv unter Erhalt der 1-Amido-, 1-Carbamido- oder 1-Alkoxycarbonylamidofunktion unter hydrogenolytischen Bedingungen spalten. Diese Hydrogenolyse liefert die Glycosylamide, Glycosylharnstoffe bzw. Glycosylcarbamate mit freier 2-Aminogruppe am Zucker mit folgender Strukturformel X

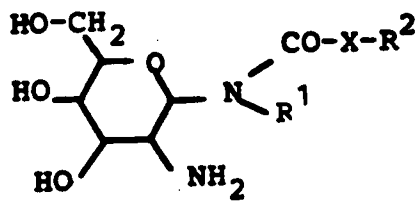

(X)

mit den oben angeführten Bedeutungen von $R^1$, $R^2$ und X.

Geeignete Katalysatoren für die Hydrogenolyse sind z.B. Edelmetalle wie Platin oder Palladium, die auf Aktivkohle aufgezogen sind.

Bevorzugt wird Palladium-Kohle (5 % oder 10 %) verwendet. Die Hydrogenolyse kann bei Normaldruck oder erhöhtem Druck in einem geeigneten Druckgefäß durchgeführt werden. Als Lösungsmittel für die Hydrierung kommen inerte Solventien wie z.B. Alkohole wie Methanol, Ethanol, Propanol, Ether wie Tetrahydrofuran oder 1,4-Dioxan oder Carbonsäuren wie Essigsäure oder Gemische der genannten Lösungsmittel in Frage, gegebenenfalls mit einem Zusatz von Wasser oder verdünnten Säuren wie Salzsäure oder Schwefelsäure. Bei Zusatz von Säuren fallen die 2-Amino-2-desoxy-glycosylamide, -carbamate bzw. -harnstoffe gemäß Formel X naturgemäß als Ammoniumsalze dieser Säuren an.

Die ebenfalls bevorzugt verwendete t-Butyloxycarbonyl-Schutzgruppe in den Verbindungen der Formel VI läßt sich nach literaturbekannten Verfahren durch Mineralsäuren wie Salzsäure oder Schwefelsäure spalten.

Auch in diesem Fall gelangt man selektiv zu den 2-Amino-2-desoxy-glycosylamiden, -carbamaten, bzw. -harnstoffen der Formel X, die dann als Ammoniumsalze der zur Spaltung eingesetzten Säuren anfallen.

Der vierte Reaktionsschritt für die Synthese der erfindungsgemäßen Verbindungen der Formel I besteht in der Verknüpfung der Aminoglycosylamide, -carbamate bzw. -harnstoffe gemäß Formel X bzw. deren Salze mit einem geeigneten Aminosäurederivat.

Geeignete Aminosäurederivate sind N-blockierte Aminosäuren XI

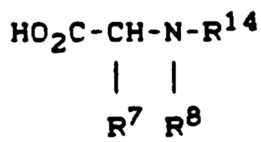

(XI)

wobei

- $R^7$ die oben angegebene Bedeutung hat,
- $R^8$ für Wasserstoff oder Methyl steht und
- $R^{14}$ eine in der Peptidsynthese gewöhnlich verwendete, selektiv unter Erhalt der Peptidbindung wieder abspaltbare Schutzgruppe darstellt.

Bevorzugt verwendete Schutzgruppen für die Aminofunktion in Formel XI sind die oben genannten, besonders bevorzugt ist die Benzyloxycarbonyl- oder die t-Butyloxycarbonylgruppe.

Die Verknüpfung des 2-Amino-2-desoxy-glycosylamids, -carbamats bzw. -harnstoffs der Formel X mit einem Aminosäurederivat der Formel XI kann nach gängigen Methoden der Peptidsynthese erfolgen (E.Wünsch et al.: Synthese von Peptiden, in: Methoden der Org. Chemie (Houben-Weyl) (E. Müller, Hrsg.). Vol XV/I und XV/2, 4. Aufl. Thieme Verlag Stuttgart (1974).

Gängige Verfahren sind z.B. die Kondensation der Aminofunktion in der Verbindung der Formel X mit einem Aminosäurederivat XI in Gegenwart von wasserentziehenden Mitteln, z.B. Dicyclohexylcarbodiimid oder Diisopropylcarbodiimid.

Die Kondensation der Verbindungen der Formel X mit denen der Formel XI kann auch durchgeführt

werden, wenn die Carboxylgruppe aktiviert ist. Eine aktivierte Carboxylgruppe kann z.B. ein Säureanhydrid, bevorzugt ein gemischtes Anhydrid wie ein Acetat der Säure, oder ein Amid der Säure, wie ein Imidazolid, oder ein aktivierter Ester sein. Die aktivierten Ester sind z.B. Cyanomethylester, Pentachlorphenylester, N-Hydroxyphthalimidester. Aktivierte Ester können auch aus der Säure XI und N-Hydroxysuccinimid oder 1-Hydroxybenzthiazol in Gegenwart eines wasserentziehenden Mittels wie Carbodiimid erhalten werden.

Die Derivate der Aminosäuren sind bekannt und können in bekannter Weise hergestellt werden.

Die Kondensation der Aminoverbindung X mit den gegebenenfalls aktivierten Carboxylverbindungen XI ergibt die Peptidoglycolipide der Formel XII

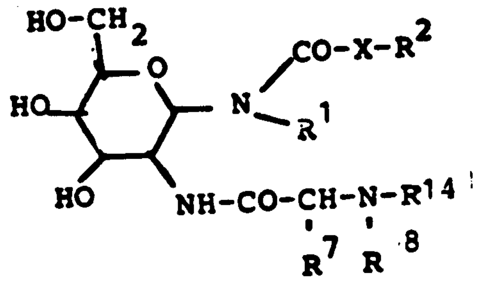

(XII)

mit den oben angegebenen Bedeutungen für $R^1$, $R^2$, $R^7$, $R^8$, $R^{14}$ und X.

In einem letzten Verfahrensschritt zur Herstellung der Verbindungen gemäß Formel I wird die Schutzgruppe $R^{14}$ in den Verbindungen der Formel XII abgespalten.

Hierbei ist darauf zu achten, daß die anderen in den Verbindungen der Formel XII vorhandenen Amid-, Urethan- oder Harnstofffunktionen nicht gespalten werden.

Die bevorzugt verwendeten Schutzgruppen $R^{14}$ in den Verbindungen der Formel XII, die N-Carbobenzoxygruppe und die N-tert.-Butyloxycarbonylgruppe, lassen sich unter Erhalt der Amid-, Urethan- oder Harnstofffunktion abspalten.

Die Carbobenzoxygruppe läßt sich selektiv durch Hydrogenolyse in Gegenwart von Edelmetallen, wie z.B. Palladium auf Kohle, in einem geeigneten Lösungsmittel wie Ethanol, Methanol, Eisessig, Tetrahydrofuran, sei es als reines Lösungsmittel, oder in Kombination untereinander oder auch mit Wasser, abspalten, wobei sowohl bei Normaldruck als auch bei erhöhtem Druck gearbeitet werden kann.

Die tert.-Butyloxycarbonylgruppe $R^{14}$ in den Verbindungen der Formel XII läßt sich mittels acidolytischer Verfahren abspalten. Geeignete Bedingungen sind z.B. die Verwendung von Chlorwasserstoff in geeigneten Lösungsmitteln wie z.B. Eisessig, Diethylether, Dioxan oder Essigsäureethylester bei Raumtemperatur.

Derartige Verfahren zur Spaltung der t-Butylcarbamate sind prinzipiell bekannt.

Die auf diese Weise erhaltenen Peptidoglycosylamide, -carbamate und -harnstoffe der Formel I werden nach an sich bekannten Verfahren in Form von kristallinen oder amorphen Feststoffen isoliert und werden, falls notwendig, durch Umkristallisation, Chromatographie, Extraktion usw. gereinigt.

Die erfindungsgemäßen Verbindungen der Formel I lassen sich mit ähnlich guten Ergebnissen auch auf einem zweiten Syntheseweg herstellen.

Dieser zweite Syntheseweg unterscheidet sich von dem ersten, oben beschriebenen, dadurch, daß die Reihenfolge der Verknüpfung der Synthesebausteine Aminozucker, Aminosäure, Amin $R^1$-$NH_2$ und Carbonsäure $R^2$-$CH_2$-$CO_2$-H bzw. Kohlensäurederivat $R^2$-O-CO-Halogen bzw. $R^2$-NCO mit den oben angeführten Bedeutungen von $R^1$, $R^2$ eine andere ist.

In diesem zweiten Weg werden geeignete 2-N-(Aminoacyl)-aminozucker der Formel XIII

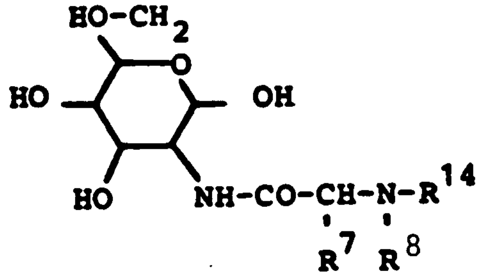

(XIII)

mit der oben angegebenen Bedeutung für $R^7$ und $R^8$, und in der $R^{14}$ eine in der Peptidchemie bekannte Aminoschutzgruppe darstellt, vorzugsweise die Benzyloxycarbonyl- oder die t-Butyloxycarbonylgruppe, als Ausgangskomponente eingesetzt.

Die Darstellung solcher 2-Aminoacyl-aminozucker ist prinzipiell bekannt (z.B. MIYAZEKI et al., Yakugaku Zasshi, 100, (1980) 95).

Die so erhaltenen Verbindungen der Formel XIII werden dann mit Aminoverbindungen der Formel III zu Glycosylaminen der allgemeinen Formel XIV

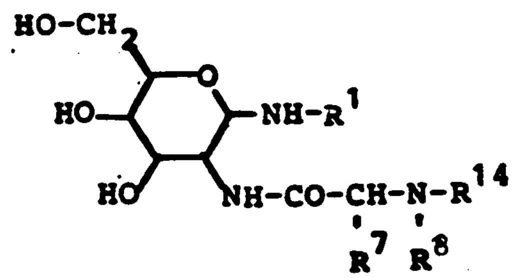

(XIV)

kondensiert, wobei $R^1$, $R^7$, $R^8$ und $R^{14}$ die oben angeführte Bedeutung haben.

Für die Darstellung der Verbindungen der allgemeinen Formel XIV können alle Verfahren, die weiter oben für die Darstellung der Verbindungen der allgemeinen Formel IV beschrieben worden sind, angewendet werden.

Die Verbindungen der Formel XIV werden dann entweder mit den oben genannten Carbonsäurederivaten V oder mit Halogenameisensäureestern VII oder mit organischen Isocyanaten VIII zu den 2-(Aminoacyl)-aminoglycosylamiden der Formel XII (X = $-CH_2-$) oder den -carbamaten der Formel XII (X = -O-) oder den -harnstoffen der Formel XII (X = -NH-) umgesetzt. Diese Acylierungsreaktionen können allgemein nach den weiter oben beschriebenen Verfahren zur Umsetzung von Glycosylaminen mit Carbon- bzw. Kohlensäurederivaten durchgeführt werden.

Die auf diesem Wege erhaltenen Zwischenprodukte XII können durch die oben genannten physikalischen Reinigungsmethoden gereinigt werden. Es ist jedoch zu bevorzugen, die Verbindungen XII durch die oben beschriebenen Methoden der O-Acylierung in die Tri-O-acetate bzw. die Tri-O-benzoate der allgemeinen Formel XV

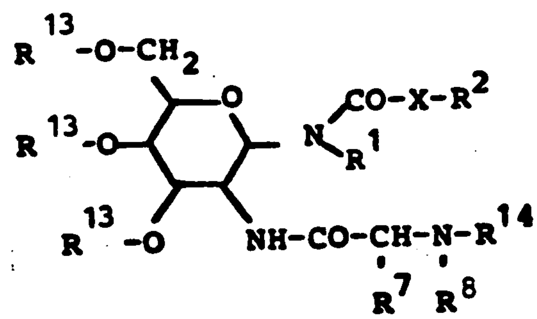

(XV)

mit den oben angeführten Bedeutungen für $R^1$, $R^2$, $R^7$, $R^8$, $R^{13}$, $R^{14}$ und X zu überführen.

Diese Verbindungen lassen sich gut aus bevorzugt polaren Lösungsmitteln wie Methanol oder Ethanol kristallisieren und dadurch reinigen.

Die kristallinen, gereinigten Derivate XV werden dann durch die oben angeführten Methoden der Esterverseifung, die speziell in der Zuckerchemie vielfach angewendet werden, in die Triole XII übergeführt.

Die letztendliche Abspaltung der Schutzgruppen in der Aminosäure in den Verbindungen der Formel XII ist bereits bei der Darstellung der Verbindungen der Formel I weiter oben beschrieben worden.

Die erfindungsgemäßen Verfahren zur Herstellung der Verbindungen der Formel I lassen sich schematisch wie folgt darstellen:

1. Verfahren

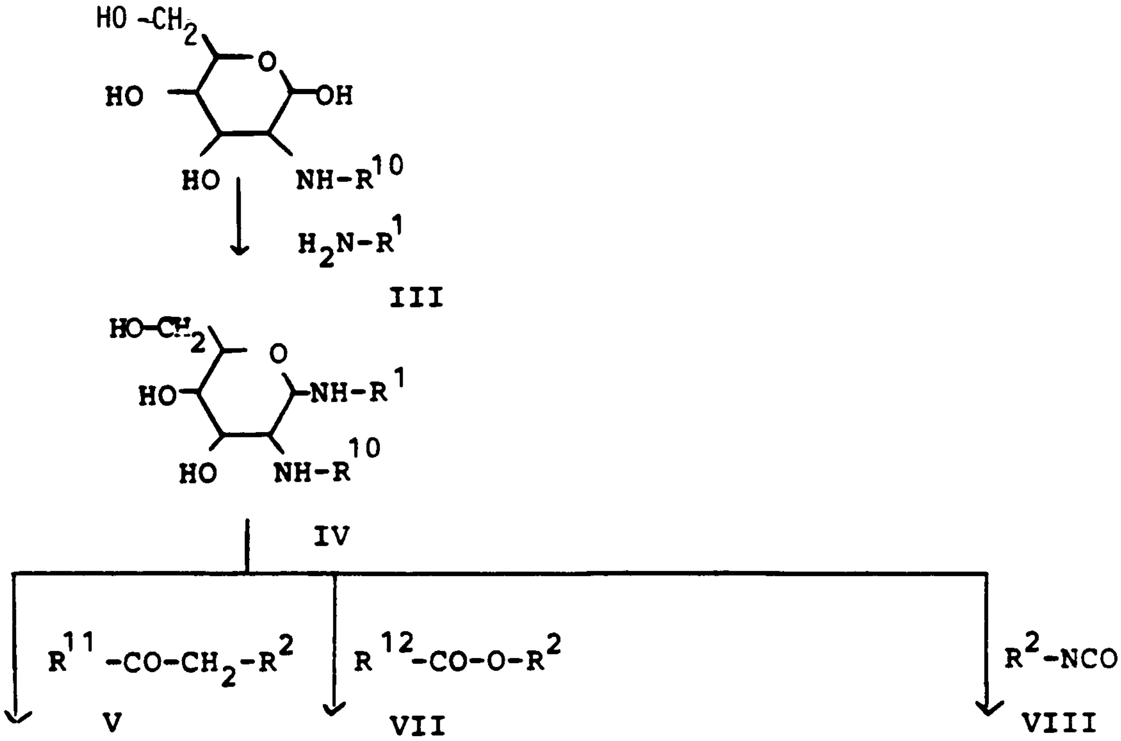

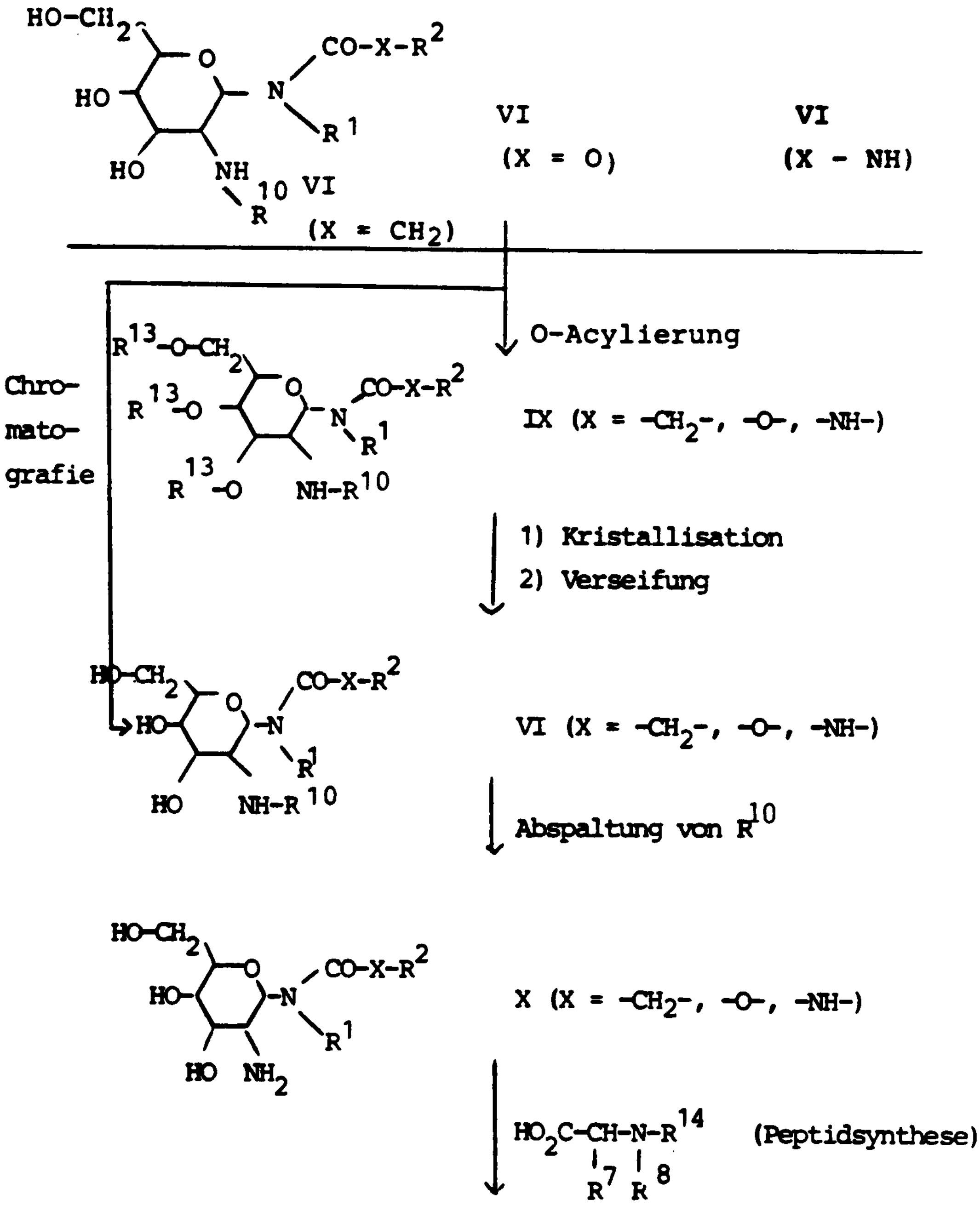
VI
(X = O)
VI
(X - NH)
VI
(X = CH2)
O-Acylierung
Chromatografie
IX (X = -CH2-, -O-, -NH-)
1) Kristallisation
2) Verseifung
VI (X = -CH2-, -O-, -NH-)
Abspaltung von R10
X (X = -CH2-, -O-, -NH-)
(Peptidsynthese)
XI

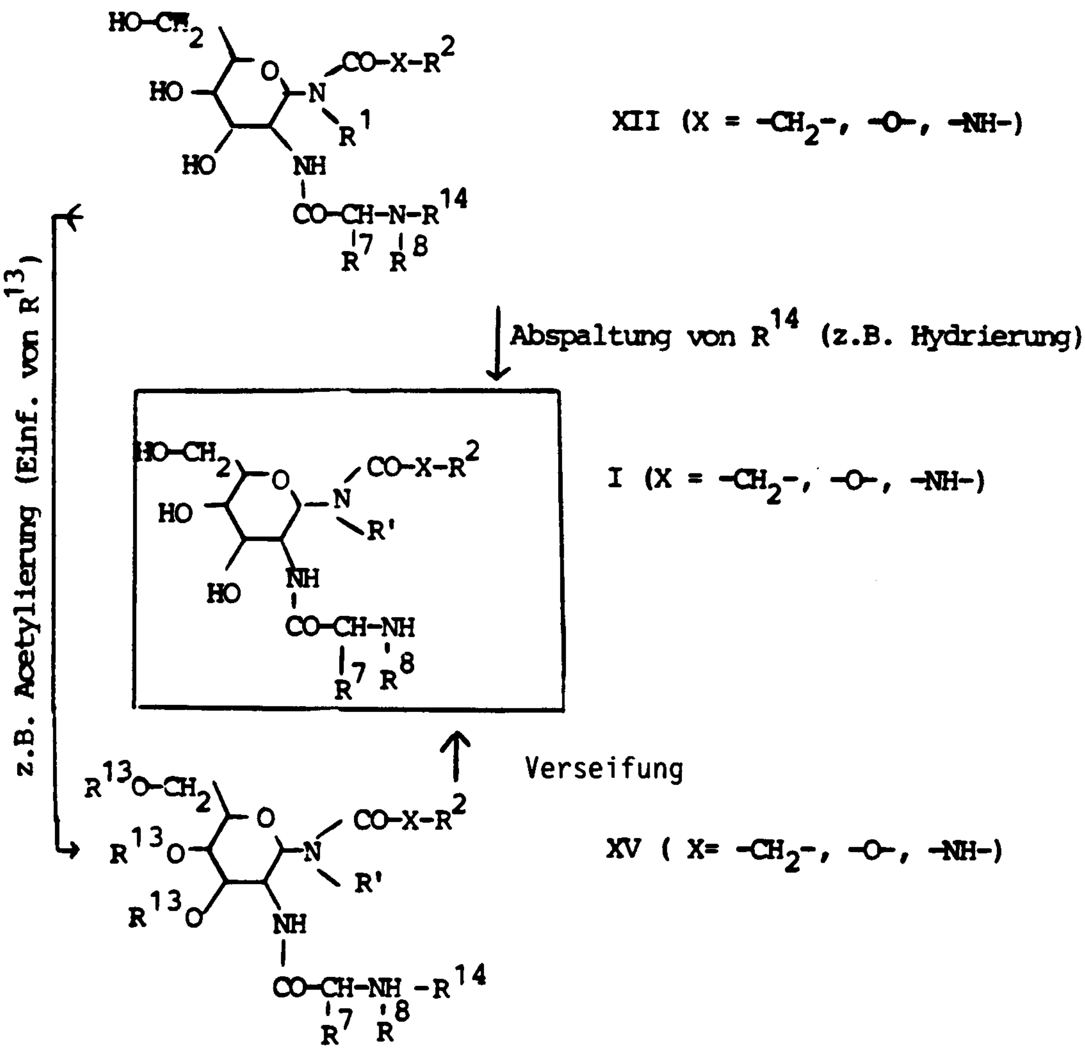


XII (X = $-CH_2-$, $-O-$, $-NH-$)

I (X = $-CH_2-$, $-O-$, $-NH-$)

XV ( X= $-CH_2-$, $-O-$, $-NH-$)

## 2. Verfahren

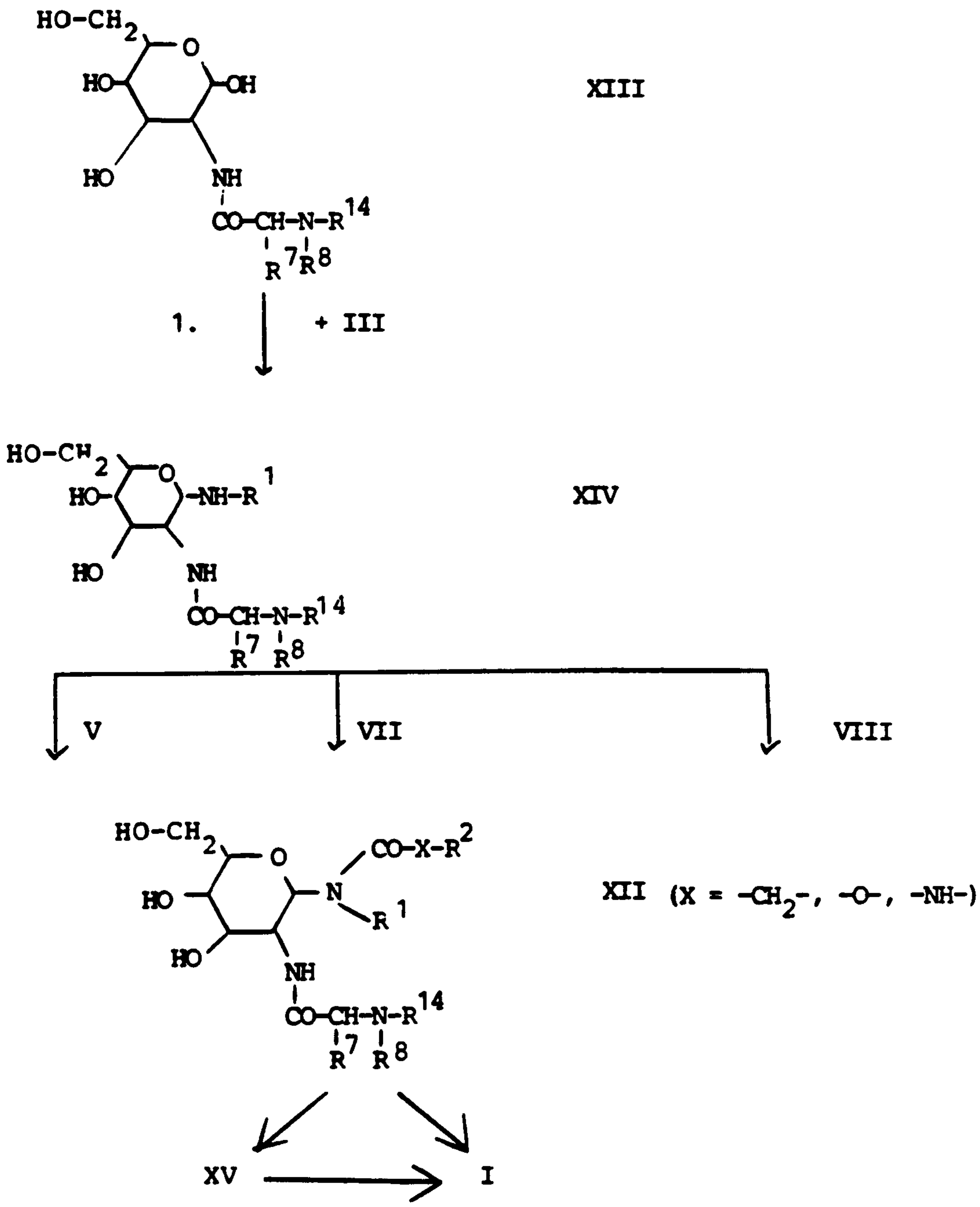


Zum Gegenstand der Erfindung gehören auch Salze der Verbindungen der Formel I. Dabei handelt es sich in erster Linie um üblicherweise pharmazeutisch verwendbare, nicht-toxische Salze, z.B. Chloride, Acetate, Lactate oder auch inerte Salze der Verbindungen der Formel I.

Die Verbindungen der Erfindung weisen eine ausgeprägte Abwehr-steigernde Wirkung auf. Es wurde gefunden, daß die Verbindungsklasse die Antikörpersynthese des Immunsystems Antigen-spezifisch steigert und darüber hinaus die unspezifische wirtseigene Abwehr verstärkt. Diese Ergebnisse wurden anhand der nachfolgenden Versuchsanordnung erhalten.

Steigerung der primären humoralen Immunität <u>in vitro</u> gegen Schaferythrozyten (SE).

Es ist experimentell möglich, die Entwicklung einer humoralen Immunantwort mit heterologen roten Blutzellen durch primäre Immunisation von Maus-Milzzellen in Suspensionskulturen <u>in vitro</u> einzuleiten (R.I.

Mishell und R.W. Dutton, J. Exp. Med. 126, 423 (1967)).

Hierzu werden Balb/c Maus-Milzzellen für 5 Tage in Gegenwart von Antigen (SE) und Testsubstanz kultiviert. Die Zellen werden geerntet, gewaschen und zusammen mit dem Antigen und Komplement in semisolidem Agar ausplattiert und für 2 Stunden bei 37 °C inkubiert (N.K. Jerne, A.A. Nordin und C. Henry, "Cell bound Antibodies", eds. Amos and Koprowski, Wistar Inst. Press, Philadelphia, USA, pp 109 (1963)). Die Antigen-Sensibilisierung von Maus-Lymphozyten in der Primärkultur führt zur Synthese und Freisetzung von Antikörpern. Die spezifischen, sezernierten Antikörper binden sich an das SE-Antigen und lysieren diese Zellen durch die Gegenwart des Komplements (Plaque-Bildung). Substanzen der vorliegenden Verbindungsklasse sind in der Lage, dosisabhängig im Bereich 0,3-100 µg/ml die Zahl der Antikörper-bildenden Zellen zu steigern (Tabelle 1).

**Tabelle 1:** Wirkung verschiedener, ausgewählter Peptidoglykolipid-Analoga der vorliegenden Verbindungsklasse auf die Antikörpersynthese *in vitro*

| Substanzen | Antikörper-sezernierende Zellen/Kultur in Abhängigkeit von der Dosis (ug/ml) | | | | | |
|---|---|---|---|---|---|---|
| Beispiel-Nr. | 0 | 1 | 3 | 10 | 30 | 100 |
| M 61 | 1595 | 2600 | 2190 | 7380 | 10.200 | 9920 |
| M 34 | 1710 | 2450 | 5700 | 8260 | 6240 | 10.640 |
| M 66 | 1710 | 1390 | 3480 | 6160 | 7600 | 8640 |
| M 51 | 1710 | 1260 | 3560 | 5560 | 7600 | n.d. 1) |
| M 54 | 1595 | 1910 | 2230 | 6160 | 6460 | n.d. 1) |

1) nicht durchgeführt

Steigerung der primären humoralen Immunität in vivo gegen das lösliche Antigen Ovalbumin.

NMRI-Mäuse wurden mit einer suboptimalen Antigendosis (1 µg/Tier, Tag 0) subcutan (s.c.) immunisiert. Bei suboptimaler Antigenstimulation wird nur eine geringe Zahl von Lymphozyten der Tiere zur Antikörpersynthese angeregt. Die zusätzliche Behandlung der Tiere mit Verbindungen der genannten Beispiele der vorliegenden Erfindung ist in der Lage, bei einer einmaligen Applikation von 0,3-30 mg/kg subcutan den Antikörpertiter im Serum der Tiere signifikant zu steigern. Die Antikörpertiterbestimmung

erfolgt durch indirekte Hämagglutination am Tag 10. Der Effekt der Behandlung wird durch den geometrischen Mittelwert der $log_2$-Titer ausgedrückt (Tabelle 2).

Der immunstimulierende Effekt der genannten Verbindungen ist im Gegensatz zu anderen, z.B. bakteriellen Immunstimulantien wie LPS aus Gram-negativen Bakterien Antigen-abhängig, d.h. die Substanzen bewirken überraschenderweise nur in Verbindung mit einem antigenen Reiz (hier SE oder Ovalbumin) die Zunahme der Antikörpersynthese. Sie haben im Gegensatz zu den erwähnten konventionellen Immunstimulantien keine mitogenen Eigenschaften.

Die erfindungsgemäßen Verbindungen bewirken in vitro und in vivo auch eine Steigerung des Aktivierungszustandes von Makrophagen. Der gesteigerte Aktivierungszustand ist durch die Erhöhung der antimikrobiellen Leistung der Makrophagen in vitro nachweisbar.

**Tabelle 2:** Adjuvantive Wirkung verschiedener, erfindungsgemäßer Verbindungen in vivo am Beispiel des löslichen Antigens Ovalbumin

| Substanzen Beispiel-Nr. | Hämagglut.-Titer ($log_2$) Dosis (mg/kg) 0 | 3 | 10 | 30 |
|---|---|---|---|---|
| M 17 | 4,2 | 4,4[1] | 5,6 | 6,6 |
| M 34 | 4,8 | 5,6[1] | 6,2 | 7,2 |
| M 1 | 5,4 | 5,4[1] | 6,4 | 7,6 |
| M 41 | 4,2 | 5,6 | 5,4 | 6,6 |
| M 66 | 4,0 | 6,2 | 6,6 | 7,2 |
| M 74 | 4,4 | 5,8 | 6,6 | 7,0 |

1) nicht signifikant;
die übrigen Werte sind signifikant gesteigert ($p \leq 0{,}05$)

Die erfindungsgemäßen Verbindungen haben die Fähigkeit, einerseits bei Mischung mit einem Antigen dessen Immunogenität zu erhöhen, andererseits bei systemischer Applikation die immunologische Reaktivität des behandelten Organismus zu steigern. Dabei sind die genannten Stoffe in der Lage, die für die Antikörperbildung verantwortlichen Lymphocyten zu aktivieren.

Die neuen Verbindungen können somit als Adjuvantien in Mischung mit Impfstoffen dazu benutzt werden, den Impferfolg zu verbessern und den durch Immunität vermittelten Infektionsschutz gegenüber bakteriellen, viralen oder parasitären Erregern zu steigern.

Weiterhin eignen sich die beschriebenen Verbindungen in Mischung mit den verschiedensten Antigenen als Adjuvantien bei der experimentellen und industriellen Herstellung von Antiseren für Therapie und

Diagnostik.

Versuchsbeschreibung

Adjuvantive Wirkung bei Anwendung von Virusvaccinen

Herpesvirus-Vaccine:

Kaninchennierenzellen wurden nach der Methode von N.J. Schmidt (in: Viral, Rickettsial and Chlamydial Infections, E.H. Lennette und N.J Schmidt, Herausgeber, p. 89, American Public Health Association, Inc., Washington, 1979) mit Minimum Essential Medium Eagle (MEME) und 10 % Kälberserum als Nährmedium gezüchtet. Sobald sich ein zusammenhängender Zellrasen gebildet hatte, wurden die Zellkulturen mit Herpes simplex-Virus Typ I (HSV I) infiziert. 48 Stunden nach der Infektion wurden Zellkulturen und Nährmedium bei -80 °C eingefroren, aufgetaut und niedertourig zentrifugiert. Der Zellkulturüberstand, der $10^7$ $ZKID_{50}$ [1]enthielt, wurde abgenommen und bis zur Verwendung bei -80 °C eingefroren.

Die Methode von Spear et al. (Journal of Virology 9, 143-159(1972)) wurde mit Modifikationen zur Reinigung von HSV I eingesetzt.

HSV I-haltige Zellkulturüberstände wurden 1 Stunde bei 40.000 RPM im T145 Rotor durch Ultrazentrifugation pelletiert (4 °C).

Die virushaltigen Pellets wurden in einem kleinen Volumen 0,01 M Trispuffer, pH 7,5, aufgenommen und beschallt (5x, 2 sec; Branson Sonifier).

Die Probe wurde auf 50 % Sucrose (w/w) eingestellt und je 10 ml in 38 ml Zentrifugenröhrchen zur Zentrifugation im SW28 Rotor eingefüllt. Der diskontinuierliche Gradient wurden nach der Methode von Spears (s.o.) aufbereitet, zentrifugiert und geerntet.

Die sich anschließende Konzentrierung des Virus durch Ultrazentrifugation geschah über ein Sucrosekissen, das auf 1 M Harnstoff eingestellt war.

Das resultierende Pellet wurde in 0,01 M Tris-puffer, pH 7,4, aufgenommen, beschallt und auf eine Extinktion $E^{280}$ von ca. 2-3 mg/ml eingestellt.

Anschließend wurde das virale "Envelope"-Antigen (EAG) in Anlehnung an die Methoden von Klein et al. (Archives of Virology 68, 73-80 (1981) aus dem gereinigten Virus isoliert. Gereinigtes Virus mit einer Extinktion von $E^{280}$ = 2,8 mg/ml wurde auf 1 % Triton-x-100 eingestellt und über Nacht bei 4 °C inkubiert. Das Detergenz-behandelte Virus wurde auf einen Kaliumtartrat-Gradienten (5-40 %) in 0,01 M Tris-puffer, pH 7,4, gegeben und im SW28 Rotor bei 28.000 RPM 18 Stunden zentrifugiert.

Proben aus dem oberen Drittel des Gradienten mit einer Absorption bei 280 nm größer 0,1 wurden vereint, mittels Vakuumdialyse eingeengt, gegen PBS dialysiert und bis zur Verwendung bei -80 °C eingefroren.

Das aus den Kaninchennierenzellen gewonnene komplette HSV I wurde 4 Stunden bei 60 °C inaktiviert. Das komplette, inaktvierte HSV I und das EAG wurden zur Immunisierung von Mäusen gegen eine Infektion mit HSV I benutzt.

Pseudorabies-Virusvaccine:

Pseudorabies-Virus wurde nach der Methode von E.A. Rollinson und G. White (Antimicrob. Ag. Chemother, 24, 221-226, 1983) in PK-15-Zellen, einer etablierten Zellinie aus Schweinenieren, die von der American Type Culture Collection, Washington, bezogen wurde, mit MEME und 2 % Serum von neugeborenen Kälbern als Nährmedium gezüchtet. Sobald virusbedingte Zellzerstörungen auftraten, wurden Zellkulturen und Nährmedium bei -80 °C eingefroren, aufgetaut und niedertourig zentrifugiert. Der Zellkulturüberstand, der $10^8$ $ZKID_{50}$ Pseudorabies-Virus enthielt, wurde abgenommen und bei -80 °C eingefroren.

Pseudorabies-Virus wurde nach der Methode von Ben-Porat et al. (Virology 41, 256-64, 1970) konzentriert und gereinigt. Anschließend wurde das virale "Envelope"-Antigen (EAG) nach der Methode von A.S. Kaplan und T. Ben-Porat (Proc. Nat. Acad. Sci., U.S.A. 66, 799-806, 1970) aus dem gereinigten Virus isoliert.

Das aus den PK-15-Zellen gewonnene, komplette Pseudorabies-Virus wurde 4 Stunden bei 60 °C inaktiviert. Das komplette, inaktivierte Pseudorabies-Virus oder des EAG wurden zur Immunisierung von Mäusen gegen eine Infektion mit Pseudorabies-Virus benutzt.

Die Immunisierung von Mäusen wurde in Anlehnung an die von E.N. Kitces et al. (Infection and

[1] zellkulturinfektiöse Dosen

Immunity Vol. 16, 955-960, 1970) beschriebenen Methode durchgeführt. Komplette, hitzeinaktivierte Viruspartikel oder EAG (Subunitvaccine) von HSV I oder Pseudorabies-Virus wurden adulten CF-1-Mäusen intramuskulär injiziert. Der Grad der Immunisierung wurde so eingestellt, daß ein Teil der Tiere eine sonst letal verlaufende Challenge-Infektion mit infektiösem Virus überlebten.

Die Behandlung mit den genannten Verbindungen kann intraperitoneal, subkutan, intramuskulär oder intravenös erfolgen. Dabei können die Verbindungen entweder getrennt oder zusammen mit der Vaccine verabreicht werden.

Die Challenge-Infektion mit infektiösem Virus erfolgte intraperitoneal, intracerebral oder intranasal 7-13 Tage nach der Immunisierung. Tote Tiere wurden täglich registriert. Der Beobachtungszeitraum betrug 14 Tage, da erfahrungsgemäß nach dieser Zeitspanne kein Tier mehr infektionsbedingt stirbt. Unterschiede in der Überlebensrate und Überlebenszeit behandelter und unbehandelter, immunisierter Tiere wurden ermittelt. Die Ergebnisse sind in den Tabellen 3-6 aufgezeigt.

Die erfindungsgemäßen Verbindungen der Formel I weisen somit starke adjuvantive Wirkung bei Anwendung von Virusvaccinen auf. Sie können daher bei menschlichen und tierischen Virusinfektionen eingesetzt werden, die einer Immunprophylaxe zugänglich sind. Die Verwendung der genannten Verbindungen ist besonders angezeigt bei Vaccinen, die nur schwach immunogen sind, z.B. bei gentechnologischen oder auf chemischem Wege hergestellte Subunitvaccinen.

Für die erfindungsgemäßen Verbindungen können als Indikationen beispielsweise folgende einer Immunprophylaxe zugänglichen Virusinfektionen genannt werden:

a) in der Humanmedizin:
Infektionen mit Influenza-, Mumps-, Masern-, Röteln-, Hepatitis- und Herpesviren
b) in der Tiermedizin:
Infektionen mit Pseudorabies-Virus (Rind, Schwein),
Rhinopneumonitis-Virus (Pferd),
sowie Marek-Virus (Huhn),
Maul- und Klauenseuche-Virus (Rind, Schwein) und Rindergrippe

Diese Aufzählung ist beispielhaft und keineswegs beschränkend aufzufassen.

Tabelle 3:

Adjuvantive Wirkung von erfindungsgemäßen Substanzen bei Anwendung einer Herpes simplex-Virusvaccine

Immunisierung: intramuskulär
Substanzgabe: intramuskulär zusammen mit Antigen
Challenge-Infektion: intraperitoneal

| Beispiel-Nr. | Überlebensrate in % (n = 10) | Mittlere Überlebensrate (Tage) |
|---|---|---|
| M 66[1)] | 100 | › 14* |
| M 51 | 90 | 13,3* |
| M 74 | 80 | 12,8* |
| M 34 | 80 | 12,8* |
| unbehandelte Kontrolle | 30 | 9,3 |

1) Dosis: 10 mg/kg
* p = 0,05 - 0,01
Die p-Werte wurden nach dem t-Test ermittelt.

Tabelle 4:

Adjuvantive Wirkung von Substanz M 66 bei Anwendung einer Herpes simplex-Virusvaccine

Immunisierung: intramuskulär
Substanzgabe: intramuskulär zusammen mit Antigen
Challenge-Infektion: intracerebral

| Dosis (mg/kg) | Überlebensrate i. % (n = 10) | Mittlere Überlebensrate (Tage) |
|---|---|---|
| 0 | 0 | 5,6 |
| 12,5 | 40 | 10,1* |
| 25 | 40 | 9,8* |

* p = 0,05 - 0,01
Die p-Werte wurden nach dem t-Test ermittelt.

Tabelle 5:

Adjuvantive Wirkung von Substanz M 66 bei Anwendung einer Pseudorabies-Virusvaccine

Immunisierung: intramuskulär
Substanzgabe: intramuskulär zusammen mit Antigen
Challenge-Infektion: intraperitoneal

| Dosis (mg/kg) | Überlebensrate i. % (n = 10) | Mittlere Übelebensrate (Tage) |
|---|---|---|
| 0 | 60 | 10,5 |
| 1,5 | 90 | 13,1 |
| 3,0 | 100 | > 14,0* |
| 6,0 | 100 | > 14,0* |
| 12,0 | 100 | > 14,0* |

* p = 0,05 - 0,025
Die p-Werte wurden nach dem t-Test ermittelt.

Tabelle 6:

Adjuvantive Wirkung von Substanz M 66 bei Anwendung einer Herpes simplex-Subunit Vakzine

Immunisierung: intramuskulär (80 ug/Maus)
Substanzgabe: intramuskulär zusammen mit dem Antigen
Challenge-Infektion: intranasal

| Dosis mg/kg | % Überlebensrate (n = 10 Mäuse) | Mittlere Überlebensrate (Tage) |
|---|---|---|
| 0 | 50 | 10,6 |
| 6 | 100 | > 14,0* |

* P = 0,01 - 0,005
Die p-Werte wurden nach dem t-Test ermittelt.

Darüber hinaus können die neuen Verbindungen auch ohne gleichzeitige Antigenzufuhr dazu benützt werden, bereits unterschwellig ablaufende Abwehrreaktionen bei Mensch und Tier zu fördern. Die Verbindungen eignen sich demnach besonders für die Stimulation der körpereigenen Abwehr, z.B. bei chronischen und akuten Infektionen oder bei selektiven (antigenspezifischen) immunologischen Defekten, sowie bei angeborenen, aber auch erworbenen allgemeinen (d.h. nicht antigenspezifisch) immunologischen Defektzuständen, wie sie im Alter, im Verlauf schwerer Primärerkrankungen und vor allen nach Therapie mit ionisierenden Strahlen oder mit immunsuppressiv wirkenden Stoffen auftreten. Die genannten Stoffe können somit vorzugsweise auch in Kombination mit antiinfektiven Antibiotika, Chemotherapeutika oder anderen Heilverfahren verabreicht werden, um Schädigungen des Makroorganismen entgegenzuwirken. Schließlich sind die beschriebenen Stoffe auch zur allgemeinen Prophylaxe von Infektionskrankheiten bei Mensch und Tier geeignet.

Die erfindungsgemäßen Verbindungen erhöben die Überlebensrate im Tiermodell der systemischen Mäusecandidose und der akuten bakteriellen Infektion und steigern die körpereigene Abwehr bei chronisch-persistierenden Infektionen.

Versuchsbeschreibung

Mäuse vom Typ SPF-CFW 1 wurden intravenös mit 2-6 x $10^5$ logarithmisch wachsenden Zellen von Candida albicans, suspendiert in physiologischer Kochsalzlösung, infiziert. Beginnend mit dem 3. Tag post infectionem werden bei unbehandelten Kontrolltieren die ersten Krankheitssymptome erkennbar. Bis zum 5. Tag sterben die ersten Tiere an akutem Nierenversagen und bis zum 14. Tag post infectionem sind in der Regel mehr als 80 % der unbehandelten Tiere gestorben. In diesem Test sind die erfindungsgemäßen Verbindungen krankheitsverzögernd wirksam. Eine signifikante krankheitsverzögernde Wirkung wurde beispielsweise mit den Verbindungen gemäß Beispiel M 17, M 61 und M 66 erreicht, wenn die Substanzen 24 Stunden vor der Infektion in Konzentrationen von 1-50 mg/kg Körpergewicht parenteral (i.p. oder s.c.)

verabreicht wurden.

Bei behandelten Tieren wurde eine statistisch signifikante Verlängerung der Überlebenszeit im Vergleich zu den unbehandelten Kontrollen beobachtet. Etwa 50 % der behandelten Tiere überlebten einen Beobachtungszeitraum von 14 Tagen, verglichen mit etwa 20 % unbehandelter Kontrolltiere.

Die erfindungsgemäßen Verbindungen können allein als Prophylaktikum, zur Bekämpfung bestehender Infektionen oder in Kombination mit einer antibiotischen Therapie zur Steigerung der therapeutischen Wirkung von Antibiotika und Chemotherapeutika (z.B. Penicilline, Cephalosporine, Aminoglykoside etc.) bei infizierten Menschen und Tieren verwendet werden.

Es wurde gefunden, daß Infektionen der Maus mit pathogenen Keimen, die innerhalb von 24-48 Stunden zum Tod der Versuchstiere führen, durch eine prophylaktische Behandlung - bevorzugt intraperitoneal - mit 1-80 mg/kg der erfindungsgemäßen Verbindungen therapiert werden können. Dies trifft für eine ganze Reihe grampositiver (z.B. Staphylokokken) und gramnegativer (z.B. E.coli, Klebsiella, Proteus, Pseudomonas) Krankheitserreger zu. Diese Aufzählung ist beispielhaft und keineswegs beschränkend aufzufassen. So überlebten z.B. Mäuse, die mit dem pathogenen Stamm Klebsiella 63 infiziert worden waren, nach Behandlung (z.B. 18 Stunden vor Infektion) mit 10-40 mg/kg der erfindungsgemäßen Verbindungen gemäß den Beispielen M 4, M 5, M 34, M 92 und M 51 zu 40 bis 100 % diese Infektion, während von den unbehandelten Kontrolltieren nur 0 bis 30 % überlebten.

In einem weiteren Versuchsmodell konnte gezeigt werden, daß die therapeutische Wirksamkeit von Antibiotika durch die erfindungsgemäßen Verbindungen gesteigert werden kann. So wurden Mäuse mit dem Stamm Pseudomonas W. infiziert. Diese Infektion führt bei den meisten Kontrolltieren innerhalb 24 Stunden zum Tode. Eine weitere Gruppe wurde mit 4 mg/kg Sisomicin 30 Stunden post infectionem behandelt. Es konnte gezeigt werden, daß in der Versuchsgruppe, die mit den erfindungsgemäßen Verbindungen (Beispiele siehe oben) 18 Stunden vor Infektion behandelt worden waren, die therapeutische Wirksamkeit des Sisomicins entscheidend verbessert werden konnte.

Für die Versuche zur subakuten Infektion wurden als Versuchstiere, $CFW_1$ Mäuse eingesetzt. Die Gruppen wurden mit jeweils 0,1 ml der in 3 % Ethanol formulierten Substanzen, in den Kontrollgruppen mit der Leerformulierung (ohne Substanz), jeweils 3 mal i.m. behandelt. Die Behandlung erfolgte 24 Stunden und 1 Stunde vor Infektion und 24 Stunden nach Infektion. Die i.p.-Infektion mit Salmonella typhimurium Stamm LT2 und ca. $5x10^5$ Keimen/Maus in 0,25 ml entsprach einer LD50. Der Infektionsverlauf der Kontrollgruppe zeichnete sich durch eine 4-tägige, initiale Phase der Infektion aus, an denen die Tiere nicht starben. Diese initiale Phase der Infektion bietet den Tieren die Möglichkeit, zelluläre Immunmechanismen zu aktivieren und stimuliert somit die unspezifische Abwehr einer latenten oder chronischen Infektion. Vom Tag 4 bis 12 nach Infektion starben ca. 50 % der Kontrolltiere. Nach einer Beobachtungszeit von 21 Tagen wurde der Versuch beendet.

Die Auswertung der Versuche erfolgte durch Vergleich der Kontrollgruppen mit den behandelten Gruppen. Hierbei wurde für die Wirksamkeit der Substanzen sowohl die reduzierte Absterberate als auch die Verzögerung des Beginns der Absterbephase als Kriterium herangezogen.

Die Verbindungen M 1, M 92, M 17 und M 54 zeigten sowohl eine Verlängerung des Zeitraums bis zum Beginn des Absterbens der Tiere, als auch eine deutliche Erhöhung der Überlebensrate. Die Effekte wurden im Konzentrationsbereich von 1 bis 10 mg/kg Körpergewicht beobachtet.

Weitere Versuche an Inzuchtmäusen (CBA/J mit normaler Infektionsabwehr ($Ity^r$) gegenüber Salmonella typhimurium zeigen nach subcutaner oder intraperitonealer Infektion mit $10^4$ - $10^5$ kolonienbildenden Einheiten einen chronischen Verlauf der Erkrankung mit Auftreten der Keime im Blut und Absiedlung in Leber und Milz. Die Bakterien sind 6-8 Wochen lang in den Organen nachweisbar, d.h. die Infektion verläuft chronisch-persistierend.

Mäuse wurden randomisiert in Gruppen zu 5 bzw. 10 Tieren eingeteilt und beispielsweise mit unterschiedlichen Dosen der Substanzen M 52 und M 54 (1x täglich) behandelt. Eine mit Lösungsmittel behandelte Gruppe von Mäusen diente als Kontrolle. Bei prophylaktischer Gabe (intraperitoneal oder subcutan) von M 52 an den Tagen -4, -3, -2, -1 vor der Erregerinokulation zeigte sich 21 Tage nach der Infektion eine Verminderung der Keimzahlen in der Leber um 90 % im Vergleich zu den Kontrollen.

Die Substanz M 54 bewirkte z.B. während der Infektion an den Tagen +3, +4, +5, +6, intraperitoneal gegeben, eine Verminderung der Keimzahlen in der Leber am 7. Tag post infectionem um ebenfalls ca. 90 %.

Unbehandelte, Salmonella-infizierte Mäuse zeigen ab der 2. Woche nach Erregerapplikation eine Suppression der T-Zell-vermittelten Immunität, nachweisbar an der verminderten Einbaurate von $^3H$-Thymidin in die Desoxyribonukleinsäure ihrer Milzlymphozyten unter Einwirkung der Mitogene Phytohämagglutinin (PHA) bzw. Concanavalin A (Con A). Nach prophylaktischer Behandlung der Tiere mit einer der erfindungsgemäßen Substanzen, z.B. M 52, war die infektionsbedingte Suppression der T-Zell-vermittelten

Immunität deutlich geringer als bei Kontrolltieren. Die Stimulierbarkeit der Milzlymphozyten erreichte Werte, wie sie bei nichtinfizierten Tieren beobachtet werden. Diese Wirkungen wurden bei einer Dosis von 5 mg/kg KG beobachtet. Ohne Infektion wurde mit M 52 keine Steigerung der Proliferation von Milzlymphozyten nachgewiesen.

Obwohl Verbindungen der beschriebenen Art ihre potenzierende Wirkung an der Maus beispielsweise bereits nach einer Einzeldosis von 10 mg/kg i.p., oder peroral entfalten, werden auch bei Applikation von 100 mg/kg keine toxischen Effekte beobachtet. Die genannten Stoffe verfügen deshalb über eine gute Verträglichkeit.

Die pharmazeutischen Präparate der vorliegenden Erfindung sind vorzugsweise Tabletten oder Gelatinekapseln, welche die Wirkstoffe zusammen mit Verdünnungsmitteln, z.B. Lactose, Dextrose, Saccharose, Mannit, Sorbit, Cellulose und/oder Schmiermitteln, z.B. Kieselerde, Talk, Stearinsäure oder Salze davon, wie Magnesium- oder Calciumstearat, und/oder Polyethylenglykol, enthalten. Tabletten enthalten ebenfalls Bindemittel, z.B. Magnesium-aluminiumsilikat, Stärken, wie Mais-, Weizen-, Reis- oder Pfeilwurzstärke, Gelatine, Traganth, Methylcellulose, Natriumcarboxymethylcellulose und/oder Polyvinylpyrrolidon, und wenn erwünscht, Sprengmittel, z.B. Stärken, Agar, Alginsäure oder ein Salz davon, wie Natriumalginat und/oder Brausemischungen, oder Adsorptionsmittel, Farbstoffe, Geschmackstoffe und Süßmittel. Injizierbare Präparate sind vorzugsweise isotonische wäßrige Lösungen oder Suspensionen. Suppositorien, Salben oder Cremen sind in erster Linie Fettemulsionen oder -suspensionen. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wertvolle Stoffe enthalten können, werden in an sich bekannter Weise, z.B. mittels konventioneller Misch-, Granulier- oder Dragierverfahren, hergestellt und enthalten von etwa 0,1 % bis etwa 75 % insbesondere von etwa 1 % bis 50 % der genannten Aktivstoffe.

Die oral applizierten Präparate der vorliegenden Erfindung können auch mit einem gegen Magensaft beständigen Überzug versehen werden.

Die erfindungsgemäßen Verbindungen können als abwehrsteigernde und immunpotenzierende Mittel zur Behandlung von chronischen und akuten Infektionen (z.B. bakterielle, virale und parasitäre) und malignen Tumoren verwendet werden. Sie können ebenfalls als Adjuvantien bei der Vakzinierung, zur Stimulierung von Phagocytose, zur Modulierung des Abwehr- und Immunsystems verwendet werden.

Einfluß bei Langzeitbehandlung auf immunologisch-vermittelte Prozesse am Model der Adjuvans-Arthritis.

In einem 30-tägigen Versuch wurde die Wirkung von Substanzen der vorliegenden Verbindungsklasse am Modell der Adjuvans-Arthritis bei der Ratte (Pearson, C.M. und F.D. Wood, Arthr. Rheu. 2, 440 (1959)), einem chronischen Entzündungsmodell, nach neuesten Mitteilungen mit einer ausgeprägten zellulären (T-Lymphozyten) Komponente, untersucht.

Bei täglicher subkutaner Applikation über einen Zeitraum von 20 Tagen supprimieren die Substanzen der vorliegenden erfindungsgemäßen Verbindungen deutlich die Läsionen der mit komplettem Freund'schen Adjuvans (CFA) injizierten Pfote (Primärläsion); als Beispiel für die Peptidoglykolipid-Analoga sei die Wirksubstanz M66 aufgeführt.

Die nach 10 Tagen erfolgende Systemisierung der Erkrankung, gemessen an der nicht-belasteten Rattenpfote (Sekundärläsion), konnte von M66 signifikant unterdrückt werden und hielt auch über einen Zeitraum von 10 Tagen nach Absetzen der Behandlung ein.

Die Verbindu8ngen weisen demnach Eigenschaften auf, die sie für Behandlungen von immunologisch bedingten Prozessen bei chronischen Entzündungen (z.B. Erkrankungen des rheumatischen Formenkreises) und immunologischen Dysregulationen (bestimmte Formen der Immundefizienz) wertvoll machen.

Beispiele

A. Allgemeine Methode zur Herstellung von N-(2-Benzyloxycarbonylamino-2-desoxy-β-D-glucopyranosyl)-alkylamin IV

Die Mischung von 10 mmol 2-Benzyloxycarbonylamino-2-desoxy-D-glucopyranose (II) (Herst.: E. Chargaff und M. Borarnick, J. Biol. Chem. 118 (1937) 421) und 20 mmol Alkylamin III wurde in 60 ml Methanol in der Wärme gelöst und 3h unter Rückfluß gerührt. Nach dem Abkühlen auf Raumtemperatur wurde das Lösungsmittel im Vakuum abgezogen. Der verbleibende Rückstand wurde in 60 ml Dimethylformamid gelöst und 5 mal mit je 20 ml n-Hexan extrahiert. Die DMF-Lösung wurde ohne weitere Manipulationen für die N-Acylierungen eingesetzt.

Nach der gleichen Methode wurde auch die N-(2-Benzyloxycarbonylamino-2-desoxy-β-D-galactopyranosyl)-alkylamine (IV) hergestellt.

B. Allgemeine Methode zur Herstellung von N-(2-Benzyloxycarbonylamino-2-desoxy-β-D-glucopyranosyl)-N-alkyl-carbonsäureamiden (VI) (X = $CH_2$)

11 mmol Carbonsäure (V, $R^{11}$ = -OH) wurden in 15 ml abs. Tetrahydrofuran gelöst und nach Zugabe von 10,5 mmol Chlorameisensäureethylester und 10,5 mmol Triethylamin 1h bei Raumtemperatur gerührt. Das gebildete Ammoniumsalz wurde abgesaugt und 2 mal mit je 3 ml Tetrahydrofuran nachgewaschen. Die vereinigten Filtrate wurden zu der Lösung des nach dem Verfahren A hergestellten Glycosylamins (IV) gegeben. Die vereinigten Lösungen wurden 4h bei Raumtemperatur gerührt. Der Ansatz wurde im Hochvakuum eingedampft und der erhaltene Rückstand durch Säulenchromatographie (Laufmittel Dichlormethan-Methanol = 20 : 1) gereinigt.

Nach der gleichen Methoden wurden, ausgehend von den N-(2-Benzyloxycarbonylamino-2-desoxy-β-D-galactopyranosyl)-alkylaminen (IV), die entsprechenden N-(2-Benzyloxycarbonylamino-2-desoxy-β-D-galactopyranosyl)-N-alkyl-carbonsäureamide VI (X = $CH_2$) hergestellt.

C: Allgemeine Methode zur Herstellung der N-(3,4,6-Tri-O-acetyl-2-benzyloxycarbonylamino-2-desoxy-β-D-glucopyranosyl)-N-alkyl-carbonsäureamide IX (X = $-CH_2-$)

Die nach der allgemeinen Methode B hergestellten N-(2-Benzyloxycarbonylamino-2-desoxy-β-D-glucopyranosyl)-N-alkyl-carbonsäureamide VI (X = $CH_2$) wurden ohne vorherige chromatographische Reinigung in 30 ml Pyridin gelöst und nach Zugabe von 20 ml Acetanhydrid für 30 min auf 50° C erwärmt. Nach dem Abkühlen auf Raumtemperatur wurde der Ansatz im Vakuum eingedampft. Der Rückstand wurde mehrfach in Toluol aufgenommen und eingedampft. Der Rückstand wurde in 50 ml Dichlormethan gelöst und über 5 g Kieselgel 60 (MERCK) filtriert. Das Filtrat wurde im Vakuum eingedampft.

Der Rückstand wurde heiß in Methanol gelöst und kristallisierte bei Raumtemperatur. Die erhaltenen Kristalle wurden aus Methanol umkristallisiert.

Analog wurden aus den N-(2-Benzyloxycarbonylamino-2-desoxy-β-D-galactopyranosyl)-N-alkyl-carbonsäureamiden VI (X = $CH_2$) durch Umsetzung mit Acetanhydrid und Pyridin die N-(3,4,6-Tri-O-acetyl-2-benzyloxycarbonylamino-2-desoxy-β-D-galactopyranosyl)-N-alkyl-carbonsäureamide IX (X = $-CH_2-$) hergestellt.

D. Allgemeine Methode zur Herstellung der N-(2-Benzyloxycarbonylamino-2-desoxy-β-D-glucopyranosyl)-N-alkyl-carbonsäureamide VI (X = $-CH_2-$) aus den entsprechenden Tri-O-acetaten IX (X = $-CH_2-$)

10 mmol der N-(3,4,6-Tri-O-acetyl-2-benzyloxycarbonylamino-2-desoxy-β-D-glucopyranosyl)-N-alkyl-carbonsäureamide IX (X = $-CH_2-$) wurden in 50 ml abs. Methanol gelöst und mit 0,5 ml 1 N Natriummethanolat versetzt. Die Mischung wurde 30 min auf 50° C erwärmt, danach auf Raumtemperatur abgekühlt und mit Ionenaustauscherharz Lewatit ® SC 108 ($H^+$-Form) neutralisiert. Das Ionenaustauscherharz wurde abfiltriert und das Filtrat zum Sirup eingedampft.

Analog wurden aus den N-(3,4,6-Tri-O-acetyl-2-benzyloxycarbonylamino-2-desoxy-β-D-galactopyranosyl)-N-alkyl-carbonsäureamiden IX (X = $-CH_2-$) durch Umesterung die entsprechenden N-(2-Benzyloxycarbonylamino-2-des- oxy-β-D-galactopyranosyl)-N-alkyl-carbonsäureamide VI (X = $-CH_2-$) hergestellt.

E. Allgemeine Methode zur Herstellung der N-(2-Benzyloxycarbonylamino-2-desoxy-β-D-glucopyranosyl)-N-alkyl-O-alkylcarbamate VI (X = O)

10 mmol des nach Methode A hergestellten N-(2-Benzyloxycarbonylamino-2-desoxy-β-D-glucopyranosyl)-alkylamins IV wurden in 20 ml abs. Tetrahydrofuran gelöst und mit 10 mmol Kaliumcarbonat versetzt.

Unter Rühren wurden 10 mmol Chlorameisensäurealkylester VII, gelöst in 10 ml abs. Tetrahydrofuran, dazugetropft. Der Ansatz wurde bis 1h nachgerührt und dann filtriert und das Filtrat im Vakuum eingedampft. Der Rückstand wurde säulenchromatographisch gereinigt: (Laufmittel Dichlormethan-Methanol = 10 : 1).

Analog wurden aus den N-(2-Benzyloxycarbonylamino-2-desoxy-β-D-galactopyranosyl)-alkylaminen IV die entsprechenden N-(2-Benzyloxycarbonylamino-2-desoxy-β-D-galactopyranosyl)-N-alkyl-O-alkylcarbamate VI (X = O) hergestellt.

F. Allgemeine Methode zur Herstellung der N-(2-Benzyloxycarbonylamino-2-desoxy-β-D-glucopyranosyl)-N-alkyl-N'-alkylharnstoffe VI (X = NH)

10 mmol des nach Methode A hergestellten N-(2-Benzyloxycarbonylamino-2-desoxy-β-D-glucopyranosyl)-alkylamins IV wurden in 20 ml abs. Tetrahydrofuran und 5 ml Methanol gelöst. Dazu wurde unter Rühren eine Lösung von 10 mmol Alkylisocyanat VIII in 10 ml Tetrahydrofuran getropft. Der Ansatz wurde 2h bei Raumtemperatur gerührt und dann im Vakuum eingedampft. Der erhaltene Sirup wurde chromatographisch auf Kieselgel gereinigt. (Laufmittel Dichlormethan-Methanol = 15 : 1).

Analog wurden aus den N-(2-Benzyloxycarbonylamino-2-desoxy-β-D-galactopyranosyl)-alkylaminen IV die entsprechenden N-(2-Benzyloxycarbonylamino-2-desoxy-β-D-galactopyranosyl)-N-alkyl-N'-alkylharnstoffe VI (X = -NH-) hergestellt.

G. Allgemeine Methode zur Herstellung der N-(2-Amino-2-desoxy-β-D-glucopyranosyl)-N-alkyl-carbonsäureamide X (X = $-CH_2-$)

10 mmol der N-(2-Benzyloxycarbonylamino-2-desoxy-β-D-glucopyranosyl)-N-alkylcarbonsäureamide VI (X = $CH_2$) wurden in 20 ml Tetrahydrofuran, 20 ml Methanol und 4 ml Eisessig gelöst und in Gegenwart von 500 mg 10 %iger Palladiumkohle bei Normaldruck hydriert. Nach beendeter Wasserstoffaufnahme wurde vom Katalysator abfiltriert und im Vakuum eingedampft. Das erhaltene Produkt fiel als Essigsäuresalz an.

Analog wurden aus den N-(2-Benzyloxycarbonylamino-2-desoxy-β-D-galactopyranosyl)-N-alkyl-carbonsäureamiden VI (X = $-CH_2-$) durch Hydrierung die entsprechenden N-(2-Amino-2-desoxy-β-D-galactopyranosyl)-N-alkylcarbonsäureamide X (X = $-CH_2-$) gewonnen.

H Allgemeine Methode zur Herstellung der N-(2-Amino-2-desoxy-β-D-glucopyranosyl)-N-alkyl-O-alkylcarbamate X (X = -O-)

Die Hydrierung von 10 mmol N-(2-Benzyloxycarbonylamino-2-desoxy-β-D-glucopyranosyl)-N-alkyl-O-alkylcarbamat VI (X = O) wurde wie bei G beschrieben durchgeführt.

Analog wurden aus den N-(2-Benzyloxycarbonylamino-2-desoxy-β-D-galactopyranosyl)-N-alkyl-O-alkyl-carbamaten VI (X = O) durch Hydrierung die entsprechenden N-(2-Amino-2-desoxy-β-D-galactopyranosyl)-N-alkyl-O-alkyl-carbamate X (X = -O-) hergestellt.

I. Allgemeine Methode zur Herstellung der N-(2-Amino-2-desoxy-β-D-glucopyranosyl)-N-alkyl-N'-alkylharnstoffe X (X = -NH-)

Die Hydrierung von 10 mmol N-(2-Benzyloxycarbonylamino-2-desoxy-β-D-glucopyranosyl)-N-alkyl-N'-alkylharnstoff VII (X = -NH-) wurde wie bei H beschrieben durchgeführt.

Analog wurden aus den N-(2-Benzyloxycarbonylamino-2-desoxy-β-D-galactopyranosyl)-N-alkyl-N'-alkyl-harnstoffen VI (X = -NH-) die entsprechenden N-(2-Amino-2-desoxy-β-D-galactopyranosyl)-N-alkyl-N'-alkylharnstoffe X (X = -NH-) hergestellt.

Allgemeine Methode zur Herstellung von N-(2-(2-Benzyloxycarbonylamino-acylamido)-2-desoxy-β-D-hexopyranosyl)-N-alkyl-carbonsäureamiden XII (X = $-CH_2-$), -O-alkyl-carbamaten XII (X = -O-) und N'-alkylharnstoffen XII (X = -NH-).

10,0 mmol der N-Benzyloxycarbonylaminosäure XI wurden in 30 ml abs. Tetrahydrofuran gelöst, mit 12,5 mmol N-Hydroxysuccinimid versetzt und auf 0 °C gekühlt. Nach Zugabe von 10,0 mmol Dicyclohexylcarbodiimid wurde der Ansatz für 3h bei 0 °C und anschließend 1h bei Raumtemperatur gerührt. Der ausgefallene Harnstoff wurde abgesaugt, das Filtrat wurde bei 0 °C zu einer Lösung von 9,5 mmol Aminoverbindung X in 50 ml abs. Tetrahydrofuran und 9,5 mmol Triethylamin gegeben.

Die Mischung wurde langsam auf Raumtemperatur erwärmt und 2h bei Raumtemperatur gerührt. Der Ansatz wurde im Vakuum eingedampft, der sirupöse Rückstand wurde in 200 ml Dichlormethan und 40 ml 2-Propanol gelöst und mehrfach mit je 60 ml 5 %iger wäßriger Kochsalzlösung extrahiert.

Die organische Phase wurde über Magnesiumsulfat getrocknet und im Vakuum zum Sirup eingedampft. Der Sirup wurde auf Kieselgel 60 säulenchromatographisch getrennt (Laufmittel Dichlormethan-Methanol-Ammoniakwasser = 20:1/5:1).

K. Allgemeine Methode zur Herstellung der N-(3,4,6-Tri-O-acetyl-2-(2-benzyloxycarbonylaminoacylamido)-2-desoxy-β-D-hexopyranosyl)-N-alkyl-carbonsäureamide XV (X = $CH_2$), -O-alkylcarbamate XV (X = O) und -N'-alkylharnstoffe XV (X = -NH-)

Die nach der allgemeinen Methode J hergestellten Glycopyranosylamide, -carbamate bzw. -harnstoffe XII wurden vor der dort beschriebenen chromatographischen Reinigungsstufe in 50 ml Pyridin und 30 ml Acetanhydrid gelöst und 1h auf 40 °erwärmt. Der Ansatz wurde mit 100 ml Eiswasser versetzt. Die organische Substanz wurde mit 150 ml Dichlormethan extrahiert, anschließend wurde die Dichlormethan-Phase erschöpfend mit 1 N Salzsäure, dann mit gesättigter wäßriger Natriumhydrogencarbonat und schließlich mit Wasser extrahiert und über Magnesiumsulfat getrocknet. Die Dichlormethan-Phase wurde eingedampft, der verbleibende Sirup wurde heiß in Methanol gelöst.

Beim langsamen Abkühlen auf Raumtemperatur oder auf 10 °C kristallisierten die Tri-O-acetate XV.

L. Allgemeine Methode zur Herstellung der Verbindungen XII durch O-Entacetylierung der Tri-O-acetate XV:

10 mmol der Tri-O-acetyl-glycopyranosylamide XV (X = $-CH_2-$), -carbamate XV (X = -O-) oder -harnstoffe XV (X = NH) wurden in 20 ml abs. Tetrahydrofuran und 30 ml abs. Methanol gelöst und nach Zugabe von 0,2 ml 1 N Natriummethanolat-Lösung 1h auf 50 °erwärmt. Die Aufarbeitung der Reaktionsansätze erfolgte wie bei der Methode D beschrieben.

M. Allgemeine Methode zur Herstellung der N-(2-(2-Aminoacylamido)-2-desoxy-β-D-hexopyranosyl)-N-alkyl-carbonsäureamide I (X = $-CH_2-$, $R^3$ = $R^4$ = $R^5$ = -H), -O-alkyl-carbamate I (X = -O-, $R^3$ = $R^4$ = $R^5$ = -H) und -N'-alkylharnstoffe I (X = NH, $R^3$ = $R^4$ = $R^5$ = -H).

10 mmol der N-(2-(2-Benzyloxycarbonylamino)acylamido-2-desoxy-β-D-hexopyranosyl)-N-alkyl-carbonsäureamide XII (X = $-CH_2-$), -O-alkylcarbamate XII (X = -O-) oder -N'-alkylharnstoffe XII (X = -NH-) wurden in 50 ml Tetrahydrofuran, 50 ml Methanol und 10 ml Eisessig gelöst und in Gegenwart von 1,0 g 10 %iger Palladiumkohle bei Normaldruck hydriert. Aufarbeitung der Ansätze erfolgte wie bei Methode G.

Nach der unter A angeführten Methode wurden die folgenden Verbindungen der allgemeinen Struktur IV hergestellt:

A.1 N-(2-Benzyloxycarbonylamino-2-desoxy-β-D-glucopyranosyl)-octylamin
A.2 N-(2-Benzyloxycarbonylamino-2-desoxy-β-D-glucopyranosyl)-decylamin
A.3 N-(2-Benzyloxycarbonylamino-2-desoxy-β-D-glucopyranosyl)-dodecylamin
A.4 N-(2-Benzyloxycarbonylamino-2-desoxy-β-D-glucopyranosyl)-tetradecylamin
A.5 N-(2-Benzyloxycarbonylamino-2-desoxy-β-D-glucopyranosyl)-hexadecylamin
A.6 N-(2-Benzyloxycarbonylamino-2-desoxy-β-D-glucopyranosyl)-octadecylamin
A.7 N-(2-Benzyloxycarbonylamino-2-desoxy-β-D-glucopyranosyl)-eicosylamin
A.8 N-(2-Benzyloxycarbonylamino-2-desoxy-β-D-galactopyranosyl)-decylamin
A.9 N-(2-Benzyloxycarbonylamino-2-desoxy-β-D-galactopyranosyl)-dodecylamin
A.10 N-(2-Benzyloxycarbonylamino-2-desoxy-β-D-galactopyranosyl)-tetradecylamin
A.11 N-(2-Benzyloxycarbonylamino-2-desoxy-β-D-galactopyranosyl)-hexadecylamin
A.12 N-(2-Benzyloxycarbonylamino-2-desoxy-β-D-galactopyranosyl)-octadecylamin

Nach der unter B angeführten Methode wurden die folgenden Verbindungen der allgemeinen Struktur VI (X = $-CH_2-$) erhalten.

B.1 N-(2-Benzyloxycarbonylamino-2-desoxy-β-D-glucopyranosyl)-N-octyl-tetradecansäureamid
B.2 N-(2-Benzyloxycarbonylamino-2-desoxy-β-D-glucopyranosyl)-N-octyl-octandecansäureamid
B.3 N-(2-Benzyloxycarbonylamino-2-desoxy-β-D-glucopyranosyl)-N-decyl-dodecansäureamid
B.4 N-(2-Benzyloxycarbonylamino-2-desoxy-β-D-glucopyranosyl)-N-decyl-tetradecansäureamid
B.5 N-(2-Benzyloxycarbonylamino-2-desoxy-β-D-glucopyranosyl)-N-decyl-octadecansäureamid
B.6 N-(2-Benzyloxycarbonylamino-2-desoxy-β-D-glucopyranosyl)-N-dodecyl-dodecansäureamid
B.7 N-(2-Benzyloxycarbonylamino-2-desoxy-β-D-glucopyranosyl)-N-dodecyl-tetradecansäureamid
B.8 N-(2-Benzyloxycarbonylamino-2-desoxy-β-D-glucopyranosyl)-N-dodecyl-hexadecansäureamid
B.9 N-(2-Benzyloxycarbonylamino-2-desoxy-β-D-glucopyranosyl)-N-dodecyl-octadecansäureamid
B.10 N-(2-Benzyloxycarbonylamino-2-desoxy-β-D-glucopyranosyl)-N-tetradecyl-dodecansäureamid
B.11 N-(2-Benzyloxycarbonylamino-2-desoxy-β-D-glucopyranosyl)-N-tetradecyl-tetradecansäureamid
B.12 N-(2-Benzyloxycarbonylamino-2-desoxy-β-D-glucopyranosyl)-N-tetradecyl-hexadecansäureamid
B.13 N-(2-Benzyloxycarbonylamino-2-desoxy-β-D-glucopyranosyl)-N-tetradecyl-octadecansäureamid
B.14 N-(2-Benzyloxycarbonylamino-2-desoxy-β-D-glucopyranosyl)-N-tetradecyl-eicosansäureamid
B.15 N-(2-Benzyloxycarbonylamino-2-desoxy-β-D-glucopyranosyl)-N-hexadecyl-decansäureamid
B.16 N-(2-Benzyloxycarbonylamino-2-desoxy-β-D-glucopyranosyl)-N-hexadecyl-dodecansäureamid
B.17 N-(2-Benzyloxycarbonylamino-2-desoxy-β-D-glucopyranosyl)-N-hexadecyl-tetradecansäureamid
B.18 N-(2-Benzyloxycarbonylamino-2-desoxy-β-D-glucopyranosyl)-N-hexadecyl-hexadecansäureamid
B.19 N-(2-Benzyloxycarbonylamino-2-desoxy-β-D-glucopyranosyl)-N-hexadecyl-octandecansäureamid
B.20 N-(2-Benzyloxycarbonylamino-2-desoxy-β-D-glucopyranosyl)-N-octadecyl-dodecansäureamid
B.21 N-(2-Benzyloxycarbonylamino-2-desoxy-β-D-glucopyranosyl)-N-octadecyl-tetradecansäureamid
B.22 N-(2-Benzyloxycarbonylamino-2-desoxy-β-D-glucopyranosyl)-N-octadecyl-hexadecansäureamid
B.23 N-(2-Benzyloxycarbonylamino-2-desoxy-β-D-glucopyranosyl)-N-octadecyl-octadecansäureamid
B.24 N-(2-Benzyloxycarbonylamino-2-desoxy-β-D-glucopyranosyl)-N-octadecyl-eicosansäureamid
B.25 N-(2-Benzyloxycarbonylamino-2-desoxy-β-D-galactopyranosyl)-N-dodecyl-dodecansäureamid
B.26 N-(2-Benzyloxycarbonylamino-2-desoxy-β-D-galactopyranosyl)-N-dodecyl-tetradecansäureamid
B.27 N-(2-Benzyloxycarbonylamino-2-desoxy-β-D-galactopyranosyl)-N-dodecyl-octadecansäureamid
B.28 N-(2-Benzyloxycarbonylamino-2-desoxy-β-D-galactopyranosyl)-N-tetradecyl-dodecansäureamid
B.29 N-(2-Benzyloxycarbonylamino-2-desoxy-β-D-galactopyranosyl)-N-tetradecyl-tetradecansäureamid
B.30 N-(2-Benzyloxycarbonylamino-2-desoxy-β-D-galactopyranosyl)-N-tetradecyl-octadecansäureamid
B.31 N-(2-Benzyloxycarbonylamino-2-desoxy-β-D-galactopyranosyl)-N-octadecyl-dodecansäureamid

B.32 N-(2-Benzyloxycarbonylamino-2-desoxy-β-D-galactopyranosyl)-N-octadecyl-tetradecansäureamid

B.33 N-(2-Benzyloxycarbonylamino-2-desoxy-β-D-galactopyranosyl)-N-octadecyl-octadecansäureamid

Nach der unter C angeführten Methode wurden die folgenden Verbindungen der allgemeinen Struktur IX (X = $CH_2$) erhalten.

C.1 N-(3,4,6-Tri-O-acetyl-2-benzyloxycarbonylamino-2-desoxy-β-D-glucopyranosyl)-N-dodecyl-dodecansäureamid

C.2 N-(3,4,6-Tri-O-acetyl-2-benzyloxycarbonylamino-2-desoxy-β-D-glucopyranosyl)-N-dodecyl-tetradecansäureamid

C.3 N-(3,4,6-Tri-O-acetyl-2-benzyloxycarbonylamino-2-desoxy-β-D-glucopyranosyl)-N-dodecyl-octadecansäureamid

C.4 N-(3,4,6-Tri-O-acetyl-2-benzyloxycarbonylamino-2-desoxy-β-D-glucopyranosyl)-N-tetradecyl-dodecansäureamid

C.5 N-(3,4,6-Tri-O-acetyl-2-benzyloxycarbonylamino-2-desoxy-β-D-glucopyranosyl)-N-tetradecyl-tetradecansäureamid

C.6 N-(3,4,6-Tri-O-acetyl-2-benzyloxycarbonylamino-2-desoxy-β-D-glucopyranosyl)-N-tetradecyl-octadecansäureamid

C.7 N-(3,4,6-Tri-O-acetyl-2-benzyloxycarbonylamino-2-desoxy-β-D-glucopyranosyl)-N-octadecyl-dodecansäureamid

C.8 N-(3,4,6-Tri-O-acetyl-2-benzyloxycarbonylamino-2-desoxy-β-D-glucopyranosyl)-N-octadecyl-tetradecansäureamid

C.9 N-(3,4,6-Tri-O-acetyl-2-benzyloxycarbonylamino-2-desoxy-β-D-glucopyranosyl)-N-octadecyl-octadecansäureamid

C.10 N-(3,4,6-Tri-O-acetyl-2-benzyloxycarbonylamino-2-desoxy-β-D-galactopyranosyl)-N-dodecyl-octadecansäureamid

C.11 N-(3,4,6-Tri-O-acetyl-2-benzyloxycarbonylamino-2-desoxy-β-D-galactopyranosyl)-N-octadecyl-dodecansäureamid

C.12 N-(3,4,6-Tri-O-acetyl-2-benzyloxycarbonylamino-2-desoxy-β-D-galactopyranosyl)-N-octadecyl-tetradecansäureamid

C.13 N-(3,4,6-Tri-O-acetyl-2-benzyloxycarbonylamino-2-desoxy-β-D-galactopyranosyl)-N-octadecyl-octadecansäureamid

Nach der unter E angeführten Methode wurden die folgenden Verbindungen der allgemeinen Struktur VI (X = -O-) erhalten.

E.1 N-(2-Benzyloxycarbonylamino-2-desoxy-β-D-glucopyranosyl)-N-dodecyl-O-dodecyl-carbamat

E.2 N-(2-Benzyloxycarbonylamino-2-desoxy-β-D-glucopyranosyl)-N-dodecyl-O-tetradecyl-carbamat

E.3 N-(2-Benzyloxycarbonylamino-2-desoxy-β-D-glucopyranosyl)-N-dodecyl-O-octadecyl-carbamat

E.4 N-(2-Benzyloxycarbonylamino-2-desoxy-β-D-glucopyranosyl)-N-tetradecyl-O-dodecyl-carbamat

E.5 N-(2-Benzyloxycarbonylamino-2-desoxy-β-D-glucopyranosyl)-N-tetradecyl-O-octadecyl-carbamat

E.6 N-(2-Benzyloxycarbonylamino-2-desoxy-β-D-glucopyranosyl)-N-octadecyl-O-dodecyl-carbamat

E.7 N-(2-Benzyloxycarbonylamino-2-desoxy-β-D-glucopyranosyl)-N-octadecyl-O-tetradecyl-carbamat

E.8 N-(2-Benzyloxycarbonylamino-2-desoxy-β-D-glucopyranosyl)-N-octadecyl-O-octadecyl-carbamat

E.9 N-(2-Benzyloxycarbonylamino-2-desoxy-β-D-galactopyranosyl)-N-dodecyl-O-dodecyl-carbamat

E.10 N-(2-Benzyloxycarbonylamino-2-desoxy-β-D-galactopyranosyl)-N-dodecyl-O-tetradecyl-carbamat

E.11 N-(2-Benzyloxycarbonylamino-2-desoxy-β-D-galactopyranosyl)-N-dodecyl-O-octadecyl-carbamat

E.12 N-(2-Benzyloxycarbonylamino-2-desoxy-β-D-galactopyranosyl)-N-tetradecyl-O-dodecyl-carbamat

E.13 N-(2-Benzyloxycarbonylamino-2-desoxy-β-D-galactopyranosyl)-N-tetradecyl-O-tetradecyl-carbamat

E.14 N-(2-Benzyloxycarbonylamino-2-desoxy-β-D-galactopyranosyl)-N-tetradecyl-O-hexadecyl-carbamat

E.15 N-(2-Benzyloxycarbonylamino-2-desoxy-β-D-galactopyranosyl)-N-tetradecyl-O-octadecyl-carbamat

E.16 N-(2-Benzyloxycarbonylamino-2-desoxy-β-D-galactopyranosyl)-N-octadecyl-O-dodecyl-carbamat

E.17 N-(2-Benzyloxycarbonylamino-2-desoxy-β-D-galactopyranosyl)-N-octadecyl-O-tetradecyl-carbamat

E.18 N-(2-Benzyloxycarbonylamino-2-desoxy-β-D-galactopyranosyl)-N-octadecyl-O-octadecyl-carbamat

Nach der unter F angeführten Methode wurden die folgenden Verbindungen der allgemeinen Struktur VI (X = -NH-) erhalten.

F.1 N-(2-Benzyloxycarbonylamino-2-desoxy-β-D-glucopyranosyl)-N-dodecyl-N'-dodecyl-harnstoff
F.2 N-(2-Benzyloxycarbonylamino-2-desoxy-β-D-glucopyranosyl)-N-dodecyl-N'-tetradecyl-harnstoff
F.3 N-(2-Benzyloxycarbonylamino-2-desoxy-β-D-glucopyranosyl)-N-dodecyl-N'-octadecyl-harnstoff
F.4 N-(2-Benzyloxycarbonylamino-2-desoxy-β-D-glucopyranosyl)-N-tetradecyl-N'-dodecyl-harnstoff
F.5 N-(2-Benzyloxycarbonylamino-2-desoxy-β-D-glucopyranosyl)-N-tetradecyl-N'-octadecyl-harnstoff
F.6 N-(2-Benzyloxycarbonylamino-2-desoxy-β-D-glucopyranosyl)-N-octadecyl-N'-dodecyl-harnstoff
F.7 N-(2-Benzyloxycarbonylamino-2-desoxy-β-D-glucopyranosyl)-N-octadecyl-N'-tetradecyl-harnstoff
F.8 N-(2-Benzyloxycarbonylamino-2-desoxy-β-D-glucopyranosyl)-N-octadecyl-N'-octadecyl-harnstoff
F.9 N-(2-Benzyloxycarbonylamino-2-desoxy-β-D-galactopyranosyl)-N-dodecyl-N'-dodecyl-harnstoff
F.10 N-(2-Benzyloxycarbonylamino-2-desoxy-β-D-galactopyranosyl)-N-dodecyl-N'-tetradecyl-harnstoff
F.11 N-(2-Benzyloxycarbonylamino-2-desoxy-β-D-galactopyranosyl)-N-dodecyl-N'-octadecyl-harnstoff
F.12 N-(2-Benzyloxycarbonylamino-2-desoxy-β-D-galactopyranosyl)-N-tetradecyl-N'-dodecyl-harnstoff
F.13 N-(2-Benzyloxycarbonylamino-2-desoxy-β-D-galactopyranosyl)-N-tetradecyl-N'-tetradecyl-harnstoff
F.14 N-(2-Benzyloxycarbonylamino-2-desoxy-β-D-galactopyranosyl)-N-tetradecyl-N'-octadecyl-harnstoff
F.15 N-(2-Benzyloxycarbonylamino-2-desoxy-β-D-galactopyranosyl)-N-octadecyl-N'-dodecyl-harnstoff
F.16 N-(2-Benzyloxycarbonylamino-2-desoxy-β-D-galactopyranosyl)-N-octadecyl-N'-tetradecyl-harnstoff
F.17 N-(2-Benzyloxycarbonylamino-2-desoxy-β-D-galactopyranosyl)-N-octadecyl-N'-octadecyl-harnstoff

Noch der unter G angeführten Methode wurden die folgenden Verbindungen der allgemeinen Struktur X (X = $-CH_2-$) in Form ihrer Essigsäure-Salze erhalten.

G.1 N-(2-Amino-2-desoxy-β-D-glucopyranosyl)-N-dodecyl-dodecansäureamid
G.2 N-(2-Amino-2-desoxy-β-D-glucopyranosyl)-N-dodecyl-tetradecansäureamid
G.3 N-(2-Amino-2-desoxy-β-D-glucopyranosyl)-N-dodecyl-hexadecansäureamid
G.4 N-(2-Amino-2-desoxy-β-D-glucopyranosyl)-N-dodecyl-octadecansäureamid
G.5 N-(2-Amino-2-desoxy-β-D-glucopyranosyl)-N-tetradecyl-dodecansäureamid
G.6 N-(2-Amino-2-desoxy-β-D-glucopyranosyl)-N-tetradecyl-tetradecansäureamid
G.7 N-(2-Amino-2-desoxy-β-D-glucopyranosyl)-N-tetradecyl-hexadecansäureamid
G.8 N-(2-Amino-2-desoxy-β-D-glucopyranosyl)-N-tetradecyl-octadecansäureamid
G.9 N-(2-Amino-2-desoxy-β-D-glucopyranosyl)-N-hexadecyl-dodecansäureamid
G.10 N-(2-Amino-2-desoxy-β-D-glucopyranosyl)-N-hexadecyl-tetradecansäureamid
G.11 N-(2-Amino-2-desoxy-β-D-glucopyranosyl)-N-hexadecyl-hexadecansäureamid
G.12 N-(2-Amino-2-desoxy-β-D-glucopyranosyl)-N-hexadecyl-octadecansäureamid
G.13 N-(2-Amino-2-desoxy-β-D-glucopyranosyl)-N-octadecyl-dodecansäureamid
G.14 N-(2-Amino-2-desoxy-β-D-glucopyranosyl)-N-octadecyl-tetradecansäureamid
G.15 N-(2-Amino-2-desoxy-β-D-glucopyranosyl)-N-octadecyl-hexadecansäureamid
G.16 N-(2-Amino-2-desoxy-β-D-glucopyranosyl)-N-octadecyl-octadecansäueramid
G.17 N-(2-Amino-2-desoxy-β-D-galactopyranosyl)-N-dodecyl-dodecansäureamid
G.18 N-(2-Amino-2-desoxy-β-D-galactopyranosyl)-N-dodecyl-tetradecansäureamid
G.19 N-(2-Amino-2-desoxy-β-D-galactopyranosyl)-N-dodecyl-octadecansäureamid
G.20 N-(2-Amino-2-desoxy-β-D-galactopyranosyl)-N-tetradecyl-dodecansäureamid
G.21 N-(2-Amino-2-desoxy-β-D-galactopyranosyl)-N-tetradecyl-octadecansäureamid
G.22 N-(2-Amino-2-desoxy-β-D-galactopyranosyl)-N-octadecyl-dodecansäureamid
G.23 N-(2-Amino-2-desoxy-β-D-galactopyranosyl)-N-octadecyl-tetradecansäureamid
G.24 N-(2-Amino-2-desoxy-β-D-galactopyranosyl)-N-octadecyl-octadecansäureamid

Nach der unter H angeführten Methode wurden die folgenden Verbindungen der allgemeinen Struktur X (X = -O-) in Form ihrer Essigsäure-Salze erhalten.

H.1 N-(2-Amino-2-desoxy-β-D-glucopyranosyl)-N-dodecyl-O-dodecyl-carbamat
H.2 N-(2-Amino-2-desoxy-β-D-glucopyranosyl)-N-dodecyl-O-tetradecyl-carbamat
H.3 N-(2-Amino-2-desoxy-β-D-glucopyranosyl)-N-dodecyl-O-octadecyl-carbamat
H.4 N-(2-Amino-2-desoxy-β-D-glucopyranosyl)-N-tetradecyl-O-dodecyl-carbamat
H.5 N-(2-Amino-2-desoxy-β-D-glucopyranosyl)-N-tetradecyl-O-octadecyl-carbamat
H.6 N-(2-Amino-2-desoxy-β-D-glucopyranosyl)-N-octadecyl-O-dodecyl-carbamat

H.7 N-(2-Amino-2-desoxy-β-D-glucopyranosyl)-N-octadecyl-O-tetradecyl-carbamat
H.8 N-(2-Amino-2-desoxy-β-D-glucopyranosyl)-N-octadecyl-O-octadecyl-carbamat
H.9 N-(2-Amino-2-desoxy-β-D-galactopyranosyl)-N-dodecyl-O-dodecyl-carbamat
H.10 N-(2-Amino-2-desoxy-β-D-galactopyranosyl)-N-dodecyl-O-tetradecyl-carbamat
H.11 N-(2-Amino-2-desoxy-β-D-galactopyranosyl)-N-dodecyl-O-octadecyl-carbamat
H.12 N-(2-Amino-2-desoxy-β-D-galactopyranosyl)-N-tetradedecyl-O-dodecyl-carbamat
H.13 N-(2-Amino-2-desoxy-β-D-galactopyranosyl)-N-tetradedecyl-O-octadecyl-carbamat
H.14 N-(2-Amino-2-desoxy-β-D-galactopyranosyl)-N-octadecyl-O-dodecyl-carbamat
H.15 N-(2-Amino-2-desoxy-β-D-galactopyranosyl)-N-octadecyl-O-tetradecyl-carbamat
H.16 N-(2-Amino-2-desoxy-β-D-galactopyranosyl)-N-octadecyl-O-octadecyl-carbamat

Nach der unter I angeführten Methode wurden die folgenden Verbindungen der allgemeinen Struktur X (X = -NH-) in Form ihrer Essigsäure-Salze erhalten.

I.1 N-(2-Amino-2-desoxy-β-D-glucopyranosyl)-N-dodecyl-N'-dodecyl-harnstoff
I.2 N-(2-Amino-2-desoxy-β-D-glucopyranosyl)-N-dodecyl-N'-tetradecyl-harnstoff
I.3 N-(2-Amino-2-desoxy-β-D-glucopyranosyl)-N-dodecyl-N'-octadecyl-harnstoff
I.4 N-(2-Amino-2-desoxy-β-D-glucopyranosyl)-N-tetradecyl-N'-dodecyl-harnstoff
I.5 N-(2-Amino-2-desoxy-β-D-glucopyranosyl)-N-tetradecyl-N'-octadecyl-harnstoff
I.6 N-(2-Amino-2-desoxy-β-D-glucopyranosyl)-N-octadecyl-N'-dodecyl-harnstoff
I.7 N-(2-Amino-2-desoxy-β-D-glucopyranosyl)-N-octadecyl-N'-tetradecyl-harnstoff
I.8 N-(2-Amino-2-desoxy-β-D-glucopyranosyl)-N-octadecyl-N'-octadecyl-harnstoff
I.9 N-(2-Amino-2-desoxy-β-D-galactopyranosyl)-N-dodecyl-N'-dodecyl-harnstoff
I.10 N-(2-Amino-2-desoxy-β-D-galactopyranosyl)-N-dodecyl-N'-tetradecyl-harnstoff
I.11 N-(2-Amino-2-desoxy-β-D-galactopyranosyl)-N-dodecyl-N'-octadecyl-harnstoff
I.12 N-(2-Amino-2-desoxy-β-D-galactopyranosyl)-N-tetradecyl-N'-dodocyl-harnstoff
I.13 N-(2-Amino-2-desoxy-β-D-galactopyranosyl)-N-tetradecyl-N'-octadecyl-harnstoff
I.14 N-(2-Amino-2-desoxy-β-D-galactopyranosyl)-N-octadecyl-N'-dodecyl-harnstoff
I.15 N-(2-Amino-2-desoxy-β-D-galactopyranosyl)-N-octadecyl-N'-tetradecyl-harnstoff
I.16 N-(2-Amino-2-desoxy-β-D-galactopyranosyl)-N-octadecyl-N'-octadecyl-harnstoff

Nach der unter J angeführten Methode wurden die folgenden Verbindungen der allgemeinen Struktur XII (X = $-CH_2-$) erhalten.

J.1 N-(2-(N-Benzyloxycarbonyl-glycinamido)-2-desoxy-β-D-glucopyranosyl)-N-dodecyl-dodecansäureamid
J.2 N-(2-(N-Benzyloxycarbonyl-glycinamido)-2-desoxy-β-D-glucopyranosyl)-N-dodecyl-tetradecansäureamid
J.3 N-(2-(N-Benzyloxycarbonyl-glycinamido)-2-desoxy-β-D-glucopyranosyl)-N-dodecyl-hexadecansäureamid
J.4 N-(2-(N-Benzyloxycarbonyl-glycinamido)-2-desoxy-β-D-glucopyranosyl)-N-dodecyl-octadecansäureamid
J.5 N-(2-(N-Benzyloxycarbonyl-glycinamido)-2-desoxy-β-D-glucopyranosyl)-N-tetradecyl-dodecansäureamid
J.6 N-(2-(N-Benzyloxycarbonyl-glycinamido)-2-desoxy-β-D-glucopyranosyl)-N-tetradecyl-tetradecansäureamid
J.7 N-(2-(N-Benzyloxycarbonyl-glycinamido)-2-desoxy-β-D-glucopyranosyl)-N-tetradecyl-hexadecansäureamid
J.8 N-(2-(N-Benzyloxycarbonyl-glycinamido)-2-desoxy-β-D-glucopyranosyl)-N-tetradecyl-octadecansäureamid
J.9 N-(2-(N-Benzyloxycarbonyl-glycinamido)-2-desoxy-β-D-glucopyranosyl)-N-hexadecyl-dodecansäureamid
J.10 N-(2-(N-Benzyloxycarbonyl-glycinamido)-2-desoxy-β-D-glucopyranosyl)-N-hexadecyl-tetradecansäureamid
J.11 N-(2-(N-Benzyloxycarbonyl-glycinamido)-2-desoxy-β-D-glucopyranosyl)-N-hexadecyl-hexadecansäureamid
J.12 N-(2-(N-Benzyloxycarbonyl-glycinamido)-2-desoxy-β-D-glucopyranosyl)-N-hexadecyl-octadecansäureamid
J.13 N-(2-(N-Benzyloxycarbonyl-glycinamido)-2-desoxy-β-D-glucopyranosyl)-N-octadecyl-dodecansäureamid
J.14 N-(2-(N-Benzyloxycarbonyl-glycinamido)-2-desoxy-β-D-glucopyranosyl)-N-octadecyl-tetradecansäureamid

J.15 N-(2-(N-Benzyloxycarbonyl-glycinamido)-2-desoxy-β-D-glucopyranosyl)-N-octadecyl-hexadecansäureamid

J.16 N-(2-(N-Benzyloxycarbonyl-glycinamido)-2-desoxy-β-D-glucopyranosyl)-N-octadecyl-octadecansäureamid

J.17 N-(2-(N-Benzyloxycarbonyl-L-alaninamido)-2-desoxy-β-D-glucopyranosyl)-N-dodecyl-dodecansäureamid

J.18 N-(2-(N-Benzyloxycarbonyl-L-alaninamido)-2-desoxy-β-D-glucopyranosyl)-N-dodecyl-tetradecansäureamid

J.19 N-(2-(N-Benzyloxycarbonyl-L-alaninamido)-2-desoxy-β-D-glucopyranosyl)-N-dodecyl-hexadecansäureamid

J.20 N-(2-(N-Benzyloxycarbonyl-L-alaninamido)-2-desoxy-β-D-glucopyranosyl)-N-dodecyl-octadecansäureamid

J.21 N-(2-(N-Benzyloxycarbonyl-L-alaninamido)-2-desoxy-β-D-glucopyranosyl)-N-tetradecyl-dodecansäureamid

J.22 N-(2-(N-Benzyloxycarbonyl-L-alaninamido)-2-desoxy-β-D-glucopyranosyl)-N-tetradecyl-tetradecansäureamid

J.23 N-(2-(N-Benzyloxycarbonyl-L-alaninamido)-2-desoxy-β-D-glucopyranosyl)-N-tetradecyl-hexadecansäureamid

J.24 N-(2-(N-Benzyloxycarbonyl-L-alaninamido)-2-desoxy-β-D-glucopyranosyl)-N-tetradecyl-octadecansäureamid

J.25 N-(2-(N-Benzyloxycarbonyl-L-alaninamido)-2-desoxy-β-D-glucopyranosyl)-N-hexadecyl-dodecansäureamid

J.26 N-(2-(N-Benzyloxycarbonyl-L-alaninamido)-2-desoxy-β-D-glucopyranosyl)-N-hexadecyl-tetradecansäureamid

J.27 N-(2-(N-Benzyloxycarbonyl-L-alaninamido)-2-desoxy-β-D-glucopyranosyl)-N-hexadecyl-hexadecansäureamid

J.28 N-(2-(N-Benzyloxycarbonyl-L-alaninamido)-2-desoxy-β-D-glucopyranosyl)-N-hexadecyl-octadecansäureamid

J.29 N-(2-(N-Benzyloxycarbonyl-L-alaninamido)-2-desoxy-β-D-glucopyranosyl)-N-octadecyl-dodecansäureamid

J.30 N-(2-(N-Benzyloxycarbonyl-L-alaninamido)-2-desoxy-β-D-glucopyranosyl)-N-octadecyl-tetradecansäureamid

J.31 N-(2-(N-Benzyloxycarbonyl-L-alaninamido)-2-desoxy-β-D-glucopyranosyl)-N-octadecyl-hexadecansäureamid

J.32 N-(2-(N-Benzyloxycarbonyl-L-alaninamido)-2-desoxy-β-D-glucopyranosyl)-N-octadecyl-octadecansäureamid

J.33 N-(2-(N-Benzyloxycarbonyl-D-alaninamido)-2-desoxy-β-D-glucopyranosyl)-N-dodecyl-dodecansäureamid

J.34 N-(2-(N-Benzyloxycarbonyl-D-alaninamido)-2-desoxy-β-D-glucopyranosyl)-N-dodecyl-octadecansäureamid

J.35 N-(2-(N-Benzyloxycarbonyl-D-alaninamido)-2-desoxy-β-D-glucopyranosyl)-N-tetradecyl-dodecansäureamid

J.36 N-(2-(N-Benzyloxycarbonyl-D-alaninamido)-2-desoxy-β-D-glucopyranosyl)-N-tetradecyl-octadecansäureamid

J.37 N-(2-(N-Benzyloxycarbonyl-D-alaninamido)-2-desoxy-β-D-glucopyranosyl)-N-octadecyl-dodecansäureamid

J.38 N-(2-(N-Benzyloxycarbonyl-D-alaninamido)-2-desoxy-β-D-glucopyranosyl)-N-octadecyl-tetradecansäureamid

J.39 N-(2-(N-Benzyloxycarbonyl-D-alaninamido)-2-desoxy-β-D-glucopyranosyl)-N-octadecyl-octadecansäureamid

J.40 N-(2-(N-Benzyloxycarbonyl-L-phenylalaninamido)-2-desoxy-β-D-glucopyranosyl)-N-dodecyl-dodecansäureamid

J.41 N-(2-(N-Benzyloxycarbonyl-L-phenylalaninamido)-2-desoxy-β-D-glucopyranosyl)-N-dodecyl-octadecansäureamid

J.42 N-(2-(N-Benzyloxycarbonyl-L-phenylalaninamido)-2-desoxy-β-D-glucopyranosyl)-N-tetradecyl-dodecansäureamid

J.43 N-(2-(N-Benzyloxycarbonyl-L-phenylalaninamido)-2-desoxy-β-D-glucopyranosyl)-N-tetradecyl-octadecansäureamid

J.44 N-(2-(N-Benzyloxycarbonyl-L-phenylalaninamido)-2-desoxy-β-D-glucopyranosyl)-N-octadecyl-dodecansäureamid

J.45 N-(2-(N-Benzyloxycarbonyl-L-phenylalaninamido)-2-desoxy-β-D-glucopyranosyl)-N-octadecyl-tetradecansäureamid

J.46 N-(2-(N-Benzyloxycarbonyl-L-phenylalaninamido)-2-desoxy-β-D-glucopyranosyl)-N-octadecyl-octandecansäureamid

J.47 N-(2-(N-Benzyloxycarbonyl-L-valinamido)-2-desoxy-β-D-glucopyranosyl)-N-dodecyl-dodecansäureamid

J.48 N-(2-(N-Benzyloxycarbonyl-L-valinamido)-2-desoxy-β-D-glucopyranosyl)-N-dodecyl-octadecansäureamid

J.49 N-(2-(N-Benzyloxycarbonyl-L-valinamido)-2-desoxy-β-D-glucopyranosyl)-N-tetradecyl-dodecansäureamid

J.50 N-(2-(N-Benzyloxycarbonyl-L-valinamido)-2-desoxy-β-D-glucopyranosyl)-N-tetradecyl-octadecansäureamid

J.51 N-(2-(N-Benzyloxycarbonyl-L-valinamido)-2-desoxy-β-D-glucopyranosyl)-N-octadecyl-dodecansäureamid

J.52 N-(2-(N-Benzyloxycarbonyl-L-valinamido)-2-desoxy-β-D-glucopyranosyl)-N-octadecyl-tetradecansäureamid

J.53 N-(2-(N-Benzyloxycarbonyl-L-valinamido)-2-desoxy-β-D-glucopyranosyl)-N-octadecyl-octadecansäureamid

J.54 N-(2-(N-Benzyloxycarbonyl-L-leucinamido)-2-desoxy-β-D-glucopyranosyl)-N-dodecyl-dodecansäureamid

J.55 N-(2-(N-Benzyloxycarbonyl-L-leucinamido)-2-desoxy-β-D-glucopyranosyl)-N-dodecyl-tetradecansäureamid

J.56 N-(2-(N-Benzyloxycarbonyl-L-leucinamido)-2-desoxy-β-D-glucopyranosyl)-N-dodecyl-hexadecansäureamid

J.57 N-(2-(N-Benzyloxycarbonyl-L-leucinamido)-2-desoxy-β-D-glucopyranosyl)-N-dodecyl-octadecansäureamid

J.58 N-(2-(N-Benzyloxycarbonyl-L-leucinamido)-2-desoxy-β-D-glucopyranosyl)-N-tetradecyl-dodecansäureamid

J.59 N-(2-(N-Benzyloxycarbonyl-L-leucinamido)-2-desoxy-β-D-glucopyranosyl)-N-tetradecyl-tetradecansäureamid

J.60 N-(2-(N-Benzyloxycarbonyl-L-leucinamido)-2-desoxy-β-D-glucopyranosyl)-N-tetradecyl-hexadecansäureamid

J.61 N-(2-(N-Benzyloxycarbonyl-L-leucinamido)-2-desoxy-β-D-glucopyranosyl)-N-tetradecyl-octadecansäureamid

J.62 N-(2-(N-Benzyloxycarbonyl-L-leucinamido)-2-desoxy-β-D-glucopyranosyl)-N-hexadecyl-dodecansäureamid

J.63 N-(2-(N-Benzyloxycarbonyl-L-leucinamido)-2-desoxy-β-D-glucopyranosyl)-N-hexadecyl-tetradecansäureamid

J.64 N-(2-(N-Benzyloxycarbonyl-L-leucinamido)-2-desoxy-β-D-glucopyranosyl)-N-hexadecyl-hexadecansäureamid

J.65 N-(2-(N-Benzyloxycarbonyl-L-leucinamido)-2-desoxy-β-D-glucopyranosyl)-N-hexadecyl-octadecansäureamid

J.66 N-(2-(N-Benzyloxycarbonyl-L-leucinamido)-2-desoxy-β-D-glucopyranosyl)-N-octadecyl-dodecansäureamid

J.67 N-(2-(N-Benzyloxycarbonyl-L-leucinamido)-2-desoxy-β-D-glucopyranosyl)-N-octadecyl-tetradecansäureamid

J.68 N-(2-(N-Benzyloxycarbonyl-L-leucinamido)-2-desoxy-β-D-glucopyranosyl)-N-octadecyl-hexadecansäureamid

J.69 N-(2-(N-Benzyloxycarbonyl-L-leucinamido)-2-desoxy-β-D-glucopyranosyl)-N-octadecyl-octadecansäureamid

J.70 N-(2-(N-Benzyloxycarbonyl-sarcosinamido)-2-desoxy-β-D-glucopyranosyl)-N-dodecyl-dodecansäureamid

J.71 N-(2-(N-Benzyloxycarbonyl-sarcosinamido)-2-desoxy-β-D-glucopyranosyl)-N-dodecyl-octadecansäureamid

J.72 N-(2-(N-Benzyloxycarbonyl-sarcosinamido)-2-desoxy-β-D-glucopyranosyl)-N-tetradecyl-dodecansäureamid

J.73 N-(2-(N-Benzyloxycarbonyl-sarcosinamido)-2-desoxy-$\beta$-D-glucopyranosyl)-N-tetradecyl-octadecansäureamid

J.74 N-(2-(N-Benzyloxycarbonyl-sarcosinamido)-2-desoxy-$\beta$-D-glucopyranosyl)-N-octadecyl-dodecansäureamid

J.75 N-(2-(N-Benzyloxycarbonyl-sarcosinamido)-2-desoxy-$\beta$-D-glucopyranosyl)-N-octadecyl-tetradecansäureamid

J.76 N-(2-(N-Benzyloxycarbonyl-sarcosinamido)-2-desoxy-$\beta$-D-glucopyranosyl)-N-octadecyl-octadecansäureamid

J.77 N-(2-(N-Benzyloxycarbonyl-O-benzyl-L-serinamido)-2-desoxy-$\beta$-D-glucopyranosyl)-N-dodecyl-dodecansäureamid

J.78 N-(2-(N-Benzyloxycarbonyl-O-benzyl-L-serinamido)-2-desoxy-$\beta$-D-glucopyranosyl)-N-dodecyl-octadecansäureamid

J.79 N-(2-(N-Benzyloxycarbonyl-O-benzyl-L-serinamido)-2-desoxy-$\beta$-D-glucopyranosyl)-N-tetradecyl-dodecansäureamid

J.80 N-(2-(N-Benzyloxycarbonyl-O-benzyl-L-serinamido)-2-desoxy-$\beta$-D-glucopyranosyl)-N-tetradecyl-octadecansäureamid

J.81 N-(2-(N-Benzyloxycarbonyl-O-benzyl-L-serinamido)-2-desoxy-$\beta$-D-glucopyranosyl)-N-octadecyl-dodecansäureamid

J.82 N-(2-(N-Benzyloxycarbonyl-O-benzyl-L-serinamido)-2-desoxy-$\beta$-D-glucopyranosyl)-N-octadecyl-tetradecansäureamid

J.83 N-(2-(N-Benzyloxycarbonyl-O-benzyl-L-serinamido)-2-desoxy-$\beta$-D-glucopyranosyl)-N-octadecyl-octadecansäure amid

J.84 N-(2-(2,5-Di-N-benzyloxycarbonyl-L-lysinamido)-2-desoxy-$\beta$-D-glucopyranosyl)-N-dodecyl-dodecansäureamid

J.85 N-(2-(2,5-Di-N-benzyloxycarbonyl-L-lysinamido)-2-desoxy-$\beta$-D-glucopyranosyl)-N-dodecyl-octadecansäureamid

J.86 N-(2-(2,5-Di-N-benzyloxycarbonyl-L-lysinamido)-2-desoxy-$\beta$-D-glucopyranosyl)-N-tetradecyl-dodecansäureamid

J.87 N-(2-(2,5-Di-N-benzyloxycarbonyl-L-lysinamido)-2-desoxy-$\beta$-D-glucopyranosyl)-N-tetradecyl-octadecansäureamid

J.88 N-(2-(2,5-Di-N-benzyloxycarbonyl-L-lysinamido)-2-desoxy-$\beta$-D-glucopyranosyl)-N-octadecyl-dodecansäureamid

J.89 N-(2-(2,5-Di-N-benzyloxycarbonyl-L-lysinamido)-2-desoxy-$\beta$-D-glucopyranosyl)-N-octadecyl-tetradecansäureamid

J.90 N-(2-(2,5-Di-N-benzyloxycarbonyl-L-lysinamido)-2-desoxy-$\beta$-D-glucopyranosyl)-N-octadecyl-octadecansäureamid

J.91 N-(2-(Benzyl-2-N-Benzyloxycarbonyl-L-aspartoylamido-2-desoxy-$\beta$-D-glucopyranosyl)-N-dodecyl-dodecansäureamid

J.92 N-(2-(Benzyl-2-N-Benzyloxycarbonyl-L-aspartoylamido)-2- desoxy-$\beta$-D-glucopyranosyl)-N-dodecyl-octadecansäureamid

J.93 N-(2-(Benzyl-2-N-Benzyloxycarbonyl-L-aspartoylamido)-2- desoxy-$\beta$-D-glucopyranosyl)-N-tetradecyl-dodecansäureamid

J.94 N-(2-(Benzyl-2-N-Benzyloxycarbonyl-L-aspartoylamido)-2-desoxy-$\beta$-D-glucopyranosyl)-N-tetradecyl-octadecansäureamid

J.95 N-(2-(Benzyl-2-N-Benzyloxycarbonyl-L-aspartoylamido)-2- desoxy-$\beta$-D-glucopyranosyl)-N-octadecyl-dodecansäureamid

J.96 N-(2-(Benzyl-2-N-Benzyloxycarbonyl-L-aspartoylamido)-2-desoxy-$\beta$-D-glucopyranosyl)-N-octadecyl-tetradecansäureamid

J.97 N-(2-(Benzyl-2-N-Benzyloxycarbonyl-L-aspartoylamido)-2-desoxy-$\beta$-D-glucopyranosyl)-N-octadecyl-octadecansäureamid

J.98 N-(2-(2-N-Benzyloxycarbonyl-5-O-benzyl-L-glutaminamido)-2-desoxy-$\beta$-D-glucopyranosyl)-N-dodecyl-dodecansäureamid

J.99 N-(2-(2-N-Benzyloxycarbonyl-5-O-benzyl-L-glutaminamido)-2-desoxy-$\beta$-D-glucopyranosyl)-N-dodecyl-octadecansäureamid

J.100 N-(2-(2-N-Benzyloxycarbonyl-5-O-benzyl-L-glutaminamido)-2-desoxy-$\beta$-D-glucopyranosyl)-N-tetradecyl-dode cansäureamid

J.101 N-(2-(2-N-Benzyloxycarbonyl-5-O-benzyl-L-glutaminamido)-2-desoxy-$\beta$-D-glucopyranosyl)-N-tetradecyl-octadecansäureamid

J.102 N-(2-(2-N-Benzyloxycarbonyl-5-O-benzyl-L-glutaminamido)-2-desoxy-$\beta$-D-glucopyranosyl)-N-octadecyl-dodecansäureamid

J.103 N-(2-(2-N-Benzyloxycarbonyl-5-O-benzyl-L-glutaminamido)-2-desoxy-$\beta$-D-glucopyranosyl)-N-octadecyl-tetradecansäureamid

J.104 N-(2-(2-N-Benzyloxycarbonyl-5-O-benzyl-L-glutaminamido)-2-desoxy-$\beta$-D-glucopyranosyl)-N-octadecyl-octadecansäureamid

J.105 N-(2-(N-Benzyloxycarbonyl-glycinamido)-2-desoxy-$\beta$-D-galactopyranosyl)-N-dodecyl-dodecansäureamid

J.106 N-(2-(N-Benzyloxycarbonyl-glycinamido)-2-desoxy-$\beta$-D-galactopyranosyl)-N-dodecyl-octadecansäureamid

J.107 N-(2-(N-Benzyloxycarbonyl-glycinamido)-2-desoxy-$\beta$-D-galactopyranosyl)-N-tetradecyl-dodecansäureamid

J.108 N-(2-(N-Benzyloxycarbonyl-glycinamido)-2-desoxy-$\beta$-D-galactopyranosyl)-N-tetradecyl-octadecansäureamid

J.109 N-(2-(N-Benzyloxycarbonyl-glycinamido)-2-desoxy-$\beta$-D-galactopyranosyl)-N-octadecyl-dodecansäureamid

J.110 N-(2-(N-Benzyloxycarbonyl-glycinamido)-2-desoxy-$\beta$-D-galactopyranosyl)-N-octadecyl-tetradecansäureamid

J.111 N-(2-(N-Benzyloxycarbonyl-glycinamido)-2-desoxy-$\beta$-D-galactopyranosyl)-N-octadecyl-octadecansäureamid

J.112 N-(2-(N-Benzyloxycarbonyl-L-alaninamido)-2-desoxy-$\beta$-D-galactopyranosyl)-N-dodecyl-dodecansäureamid

J.113 N-(2-(N-Benzyloxycarbonyl-L-alaninamido)-2-desoxy-$\beta$-D-galactopyranosyl)-N-dodecyl-octadecansäureamid

J.114 N-(2-(N-Benzyloxycarbonyl-L-alaninamido)-2-desoxy-$\beta$-D-galactopyranosyl)-N-tetradecyl-dodecansäureamid

J.115 N-(2-(N-Benzyloxycarbonyl-L-alaninamido)-2-desoxy-$\beta$-D-galactopyranosyl)-N-tetradecyl-octadecansäureamid

J.116 N-(2-(N-Benzyloxycarbonyl-L-alaninamido)-2-desoxy-$\beta$-D-galactopyranosyl)-N-octadecyl-dodecansäureamid

J.117 N-(2-(N-Benzyloxycarbonyl-L-alaninamido)-2-desoxy-$\beta$-D-galactopyranosyl)-N-octadecyl-tetradecansäureamid

J.118 N-(2-(N-Benzyloxycarbonyl-L-alaninamido)-2-desoxy-$\beta$-D-galactopyranosyl)-N-octadecyl-octadecansäureamid

J.119 N-(2-(N-Benzyloxycarbonyl-L-leucinamido)-2-desoxy-$\beta$-D-galactopyranosyl)-N-dodecyl-dodecansäureamid

J.120 N-(2-(N-Benzyloxycarbonyl-L-leucinamido)-2-desoxy-$\beta$-D-galactopyranosyl)-N-dodecyl-octadecansäureamid

J.121 N-(2-(N-Benzyloxycarbonyl-L-leucinamido)-2-desoxy-$\beta$-D-galactopyranosyl)-N-tetradecyl-dodecansäureamid

J.122 N-(2-(N-Benzyloxycarbonyl-L-leucinamido)-2-desoxy-$\beta$-D-galactopyranosyl)-N-tetradecyl-octadecansäureamid

J.123 N-(2-(N-Benzyloxycarbonyl-L-leucinamido)-2-desoxy-$\beta$-D-galactopyranosyl)-N-octadecyl-dodecansäureamid

J.124 N-(2-(N-Benzyloxycarbonyl-L-leucinamido)-2-desoxy-$\beta$-D-galactopyranosyl)-N-octadecyl-tetradecansäureamid

J.125 N-(2-(N-Benzyloxycarbonyl-L-leucinamido)-2-desoxy-$\beta$-D-galactopyranosyl)-N-octadecyl-octadecansäureamid

Nach der unter J angeführten Methode wurden die folgenden Verbindungen der allgemeinen Struktur XII (X = O) erhalten.

J.126 N-(2-(N-Benzyloxycarbonyl-glycinamido)-2-desoxy-$\beta$-D-glucopyranosyl)-N-dodecyl-O-dodecyl-carbamat

J.127 N-(2-(N-Benzyloxycarbonyl-glycinamido)-2-desoxy-$\beta$-D-glucopyranosyl)-N-dodecyl-O-tetradecyl-carbamat

J.128 N-(2-(N-Benzyloxycarbonyl-glycinamido)-2-desoxy-$\beta$-D-glucopyranosyl)-N-dodecyl-O-octadecyl-carbamat

J.129 N-(2-(N-Benzyloxycarbonyl-glycinamido)-2-desoxy-$\beta$-D-glucopyranosyl)-N-tetradecyl-O-dodecyl-carbamat

J.130 N-(2-(N-Benzyloxycarbonyl-glycinamido)-2-desoxy-β-D-glucopyranosyl)-N-tetradecyl-O-octadecyl-carbamat

J.131 N-(2-(N-Benzyloxycarbonyl-glycinamido)-2-desoxy-β-D-glucopyranosyl)-N-octadecyl-O-dodecyl-carbamat

J.132 N-(2-(N-Benzyloxycarbonyl-glycinamido)-2-desoxy-β-D-glucopyranosyl)-N-octadecyl-O-tetradecyl-carbamat

J.133 N-(2-(N-Benzyloxycarbonyl-glycinamido)-2-desoxy-β-D-glucopyranosyl)-N-octadecyl-O-octadecyl-carbamat

J.134 N-(2-(N-Benzyloxycarbonyl-L-alaninamido)-2-desoxy-β-D-glucopyranosyl)-N-dodecyl-O-dodecyl-carbamat

J.135 N-(2-(N-Benzyloxycarbonyl-L-alaninamido)-2-desoxy-β-D-glucopyranosyl)-N-dodecyl-O-tetradecyl-carbamat

J.136 N-(2-(N-Benzyloxycarbonyl-L-alaninamido)-2-desoxy-β-D-glucopyranosyl)-N-dodecyl-O-octadecyl-carbamat

J.137 N-(2-(N-Benzyloxycarbonyl-L-alaninamido)-2-desoxy-β-D-glucopyranosyl)-N-tetradecyl-O-dodecyl-carbamat

J.138 N-(2-(N-Benzyloxycarbonyl-L-alaninamido)-2-desoxy-β-D-glucopyranosyl)-N-tetradecyl-O-octadecyl-carbamat

J.139 N-(2-(N-Benzyloxycarbonyl-L-alaninamido)-2-desoxy-β-D-glucopyranosyl)-N-octadecyl-O-dodecyl-carbamat

J.140 N-(2-(N-Benzyloxycarbonyl-L-alaninamido)-2-desoxy-β-D-glucopyranosyl)-N-octadecyl-O-tetradecyl-carbamat

J.141 N-(2-(N-Benzyloxycarbonyl-L-alaninamido)-2-desoxy-β-D-glucopyranosyl)-N-octadecyl-O-octadecyl-carbamat

J.142 N-(2-(N-Benzyloxycarbonyl-L-leucinamido)-2-desoxy-β-D-glucopyranosyl)-N-dodecyl-O-dodecyl-carbamat

J.143 N-(2-(N-Benzyloxycarbonyl-L-leucinamido)-2-desoxy-β-D-glucopyranosyl)-N-dodecyl-O-tetradecyl-carbamat

J.144 N-(2-(N-Benzyloxycarbonyl-L-leucinamido)-2-desoxy-β-D-glucopyranosyl)-N-dodecyl-O-octadecyl-carbamat

J.145 N-(2-(N-Benzyloxycarbonyl-L-leucinamido)-2-desoxy-β-D-glucopyranosyl)-N-tetradecyl-O-dodecyl-carbamat

J.146 N-(2-(N-Benzyloxycarbonyl-L-leucinamido)-2-desoxy-β-D-glucopyranosyl)-N-tetradecyl-O-octadecyl-carbamat

J.147 N-(2-(N-Benzyloxycarbonyl-L-leucinamido)-2-desoxy-β-D-glucopyranosyl)-N-octadecyl-O-dodecyl-carbamat

J.148 N-(2-(N-Benzyloxycarbonyl-L-leucinamido)-2-desoxy-β-D-glucopyranosyl)-N-octadecyl-O-tetradecyl-carbamat

J.149 N-(2-(N-Benzyloxycarbonyl-L-leucinamido)-2-desoxy-β-D-glucopyranosyl)-N-octadecyl-O-octadecyl-carbamat

J.150 N-(2-(N-Benzyloxycarbonyl-glycinamido)-2-desoxy-β-D-galactopyranosyl)-N-dodecyl-O-dodecyl-carbamat

J.151 N-(2-(N-Benzyloxycarbonyl-glycinamido)-2-desoxy-β-D-galactopyranosyl)-N-dodecyl-tetradecyl-carbamat

J.152 N-(2-(N-Benzyloxycarbonyl-glycinamido)-2-desoxy-β-D-galactopyranosyl)-N-dodecyl-O-octadecyl-carbamat

J.153 N-(2-(N-Benzyloxycarbonyl-glycinamido)-2-desoxy-β-D-galactopyranosyl)-N-tetradecyl-O-dodecyl-carbamat

J.154 N-(2-(N-Benzyloxycarbonyl-glycinamido)-2-desoxy-β-D-galactopyranosyl)-N-tetradecyl-O-octadecyl-carbamat

J.155 N-(2-(N-Benzyloxycarbonyl-glycinamido)-2-desoxy-β-D-galactopyranosyl)-N-octadecyl-O-dodecyl-carbamat

J.156 N-(2-(N-Benzyloxycarbonyl-glycinamido)-2-desoxy-β-D-galactopyranosyl)-N-octadecyl-O-tetradecyl-carbamat

J.157 N-(2-(N-Benzyloxycarbonyl-glycinamido)-2-desoxy-β-D-galactopyranosyl)-N-octadecyl-O-octadecyl-carbamat

Nach der unter J angeführten Methode wurden die folgenden Verbindungen der allgemeinen Struktur XII (X = -NH-) erhalten.

J.158 N-(2-(N-Benzyloxycarbonyl-glycinamido)-2-desoxy-β-D-glucopyranosyl)-N-dodecyl-N'-dodecyl-harnstoff

J.159 N-(2-(N-Benzyloxycarbonyl-glycinamido)-2-desoxy-β-D-glucopyranosyl)-N-dodecyl-N'-tetradecyl-harnstoff

J.160 N-(2-(N-Benzyloxycarbonyl-glycinamido)-2-desoxy-β-D-glucopyranosyl)-N-dodecyl-N'-octadecyl-harnstoff

J.161 N-(2-(N-Benzyloxycarbonyl-glycinamido)-2-desoxy-β-D-glucopyranosyl)-N-tetradecyl-N'-dodecyl-harnstoff

J.162 N-(2-(N-Benzyloxycarbonyl-glycinamido)-2-desoxy-β-D-glucopyranosyl)-N-tetradecyl-N'-octadecyl-harnstoff

J.163 N-(2-(N-Benzyloxycarbonyl-glycinamido)-2-desoxy-β-D-glucopyranosyl)-N-octadecyl-N'-dodecyl-harnstoff

J.164 N-(2-(N-Benzyloxycarbonyl-glycinamido)-2-desoxy-β-D-glucopyranosyl)-N-octadecyl-N'-tetradecyl-harnstoff

J.165 N-(2-(N-Benzyloxycarbonyl-glycinamido)-2-desoxy-β-D-glucopyranosyl)-N-octadecyl-N'-octadecyl-harnstoff

J.166 N-(2-(N-Benzyloxycarbonyl-L-alaninamido)-2-desoxy-β-D-glucopyranosyl)-N-dodecyl-N'-dodecyl-harnstoff

J.167 N-(2-(N-Benzyloxycarbonyl-L-alaninamido)-2-desoxy-β-D-glucopyranosyl)-N-dodecyl-N'-tetradecyl-harnstoff

J.168 N-(2-(N-Benzyloxycarbonyl-L-alaninamido)-2-desoxy-β-D-glucopyranosyl)-N-dodecyl-N'-octadecyl-harnstoff

J.169 N-(2-(N-Benzyloxycarbonyl-L-alaninamido)-2-desoxy-β-D-glucopyranosyl)-N-tetradecyl-N'-dodecyl-harnstoff

J.170 N-(2-(N-Benzyloxycarbonyl-L-alaninamido)-2-desoxy-β-D-glucopyranosyl)-N-tetradecyl-N'-octadecyl-harnstoff

J.171 N-(2-(N-Benzyloxycarbonyl-L-alaninamido)-2-desoxy-β-D-glucopyranosyl)-N-octadecyl-N'-dodecyl-harnstoff

J.172 N-(2-(N-Benzyloxycarbonyl-L-alaninamido)-2-desoxy-β-D-glucopyranosyl)-N-octadecyl-N'-tetradecyl-harnstoff

J.173 N-(2-(N-Benzyloxycarbonyl-L-alaninamido)-2-desoxy-β-D-glucopyranosyl)-N-octadecyl-N'-octadecyl-harnstoff

J.174 N-(2-(N-Benzyloxycarbonyl-L-leucinamido)-2-desoxy-β-D-glucopyranosyl)-N-dodecyl-N'-dodecyl-harnstoff

J.175 N-(2-(N-Benzyloxycarbonyl-L-leucinamido)-2-desoxy-β-D-glucopyranosyl)-N-dodecyl-N'-tetradecyl-harnstoff

J.176 N-(2-(N-Benzyloxycarbonyl-L-leucinamido)-2-desoxy-β-D-glucopyranosyl)-N-dodecyl-N'-octadecyl-harnstoff

J.177 N-(2-(N-Benzyloxycarbonyl-L-leucinamido)-2-desoxy-β-D-glucopyranosyl)-N-tetradecyl-N'-dodecyl-harnstoff

J.178 N-(2-(N-Benzyloxycarbonyl-L-leucinamido)-2-desoxy-β-D-glucopyranosyl)-N-tetradecyl-N'-octadecyl-harnstoff

J.179 N-(2-(N-Benzyloxycarbonyl-L-leucinamido)-2-desoxy-β-D-glucopyranosyl)-N-octadecyl-N'-dodecyl-harnstoff

J.180 N-(2-(N-Benzyloxycarbonyl-L-leucinamido)-2-desoxy-β-D-glucopyranosyl)-N-octadecyl-N'-tetradecyl-harnstoff

J.181 N-(2-(N-Benzyloxycarbonyl-L-leucinamido)-2-desoxy-β-D-glucopyranosyl)-N-octadecyl-N'-octadecyl-harnstoff

J.182 N-(2-(N-Benzyloxycarbonyl-glycinamido)-2-desoxy-β-D-galactopyranosyl)-N-dodecyl-N'-dodecyl-harnstoff

J.183 N-(2-(N-Benzyloxycarbonyl-glycinamido)-2-desoxy-β-D-galactopyranosyl)-N-dodecyl-N'-tetradecyl-harnstoff

J.184 N-(2-(N-Benzyloxycarbonyl-glycinamido)-2-desoxy-β-D-galactopyranosyl)-N-dodecyl-N'-octadecyl-harnstoff

J.185 N-(2-(N-Benzyloxycarbonyl-glycinamido)-2-desoxy-β-D-galactopyranosyl)-N-tetradecyl-N'-dodecyl-harnstoff

J.186 N-(2-(N-Benzyloxycarbonyl-glycinamido)-2-desoxy-β-D-galactopyranosyl)-N-tetradecyl-N'-octadecyl-harnstoff

J.187 N-(2-(N-Benzyloxycarbonyl-glycinamido)-2-desoxy-β-D-galactopyranosyl)-N-octadecyl-N'-dodecyl-harnstoff
J.188 N-(2-(N-Benzyloxycarbonyl-glycinamido)-2-desoxy-β-D-galactopyranosyl)-N-octadecyl-N'-tetradecyl-harnstoff
J.189 N-(2-(N-Benzyloxycarbonyl-glycinamido)-2-desoxy-β-D-galactopyranosyl)-N-octadecyl-N'-octadecyl-harnstoff

Nach der unter M angeführten Methode wurden die folgenden Verbindungen der allgemeinen Sturktur I (X = $-CH_2-$) in Form ihrer Essigsäure-Salze erhalten.

M.1 N-(2-Glycinamido-2-desoxy-β-D-glucopyranosyl)-N-dodecyl-dodecansäureamid
M.2 N-(2-Glycinamido-2-desoxy-β-D-glucopyranosyl)-N-dodecyl-tetradecansäureamid
M.3 N-(2-Glycinamido-2-desoxy-β-D-glucopyranosyl)-N-dodecyl-hexadecansäureamid
M.4 N-(2-Glycinamido-2-desoxy-β-D-glucopyranosyl)-N-dodecyl-octadecansäureamid
M.5 N-(2-Glycinamido-2-desoxy-β-D-glucopyranosyl)-N-tetradecyl-dodecansäureamid
M.6 N-(2-Glycinamido-2-desoxy-β-D-glucopyranosyl)-N-tetradecyl-tetradecansäureamid
M.7 N-(2-Glycinamido-2-desoxy-β-D-glucopyranosyl)-N-tetradecyl-hexadecansäureamid
M.8 N-(2-Glycinamido-2-desoxy-β-D-glucopyranosyl)-N-tetradecyl-octadecansäureamid
M.9 N-(2-Glycinamido-2-desoxy-β-D-glucopyranosyl)-N-hexadecyl-dodecansäureamid
M.10 N-(2-Glycinamido-2-desoxy-β-D-glucopyranosyl)-N-hexadecyl-tetradecansäureamid
M.11 N-(2-Glycinamido-2-desoxy-β-D-glucopyranosyl)-N-hexadecyl-hexadecansäureamid
M.12 N-(2-Glycinamido-2-desoxy-β-D-glucopyranosyl)-N-hexadecyl-octadecansäureamid
M.13 N-(2-Glycinamido-2-desoxy-β-D-glucopyranosyl)-N-octadecyl-dodecansäureamid
M.14 N-(2-Glycinamido-2-desoxy-β-D-glucopyranosyl)-N-octadecyl-tetradecansäureamid
M.15 N-(2-Glycinamido-2-desoxy-β-D-glucopyranosyl)-N-octadecyl-hexadecansäureamid
M.16 N-(2-Glycinamido-2-desoxy-β-D-glucopyranosyl)-N-octadecyl-octadecansäureamid
M.17 N-(2-L-Alaninamido-2-desoxy-β-D-glucopyranosyl)-N-dodecyl-dodecansäureamid
M.18 N-(2-L-Alaninamido-2-desoxy-β-D-glucopyranosyl)-N-dodecyl-tetradecansäureamid
M.19 N-(2-L-Alaninamido-2-desoxy-β-D-glucopyranosyl)-N-dodecyl-hexadecansäureamid
M.20 N-(2-L-Alaninamido-2-desoxy-β-D-glucopyranosyl)-N-dodecyl-octadecansäureamid
M.21 N-(2-L-Alaninamido-2-desoxy-β-D-glucopyranosyl)-N-tetradecyl-dodecansäureamid
M.22 N-(2-L-Alaninamido-2-desoxy-β-D-glucopyranosyl)-N-tetradecyl-tetradecansäureamid
M.23 N-(2-L-Alaninamido-2-desoxy-β-D-glucopyranosyl)-N-tetradecyl-hexadecansäureamid
M.24 N-(2-L-Alaninamido-2-desoxy-β-D-glucopyranosyl)-N-tetradecyl-octadecansäureamid
M.25 N-(2-L-Alaninamido-2-desoxy-β-D-glucopyranosyl)-N-hexadecyl-dodecansäureamid
M.26 N-(2-L-Alaninamido-2-desoxy-β-D-glucopyranosyl)-N-hexadecyl-tetradecansäureamid
M.27 N-(2-L-Alaninamido-2-desoxy-β-D-glucopyranosyl)-N-hexadecyl-hexadecansäureamid
M.28 N-(2-L-Alaninamido-2-desoxy-β-D-glucopyranosyl)-N-hexadecyl-octadecansäureamid
M.29 N-(2-L-Alaninamido-2-desoxy-β-D-glucopyranosyl)-N-octadecyl-dodecansäureamid
M.30 N-(2-L-Alaninamido-2-desoxy-β-D-glucopyranosyl)-N-octadecyl-tetradecansäureamid
M.31 N-(2-L-Alaninamido-2-desoxy-β-D-glucopyranosyl)-N-octadecyl-hexadecansäureamid
M.32 N-(2-L-Alaninamido-2-desoxy-β-D-glucopyranosyl)-N-octadecyl-octadecansäureamid
M.33 N-(2-D-Alaninamido-2-desoxy-β-D-glucopyranosyl)-N-dodecyl-dodecansäureamid
M.34 N-(2-D-Alaninamido-2-desoxy-β-D-glucopyranosyl)-N-dodecyl-octadecansäureamid
M.33 N-(2-D-Alaninamido-2-desoxy-β-D-glucopyranosyl)-N-tetradecyl-dodecansäureamid
M.36 N-(2-D-Alaninamido-2-desoxy-β-D-glucopyranosyl)-N-tetradecyl-octadecansäureamid
M.37 N-(2-D-Alaninamido-2-desoxy-β-D-glucopyranosyl)-N-octadecyl-dodecansäureamid
M.38 N-(2-D-Alaninamido-2-desoxy-β-D-glucopyranosyl)-N-octadecyl-tetradecansäureamid
M.39 N-(2-D-Alaninamido-2-desoxy-β-D-glucopyranosyl)-N-octadecyl-octadecansäureamid
M.40 N-(2-L-Phenylalaninamido-2-desoxy-β-D-glucopyranosyl)-N-dodecyl-dodecansäureamid
M.41 N-(2-L-Phenylalaninamido-2-desoxy-β-D-glucopyranosyl)-N-dodecyl-octadecansäureamid
M.42 N-(2-L-Phenylalaninamido-2-desoxy-β-D-glucopyranosyl)-N-tetradecyl-dodecansäureamid
M.43 N-(2-L-Phenylalaninamido-2-desoxy-β-D-glucopyranosyl)-N-tetradecyl-octadecansäureamid
M.44 N-(2-L-Phenylalaninamido-2-desoxy-β-D-glucopyranosyl)-N-octadecyl-dodecansäureamid
M.45 N-(2-L-Phenylalaninamido-2-desoxy-β-D-glucopyranosyl)-N-octadecyl-tetradecansäureamid
M.46 N-(2-L-Phenylalaninamido-2-desoxy-β-D-glucopyranosyl)-N-octadecyl-octadecansäureamid
M.47 N-(2-L-Valinamido-2-desoxy-β-D-glucopyranosyl)-N-dodecyl-dodecansäureamid
M.48 N-(2-L-Valinamido-2-desoxy-β-D-glucopyranosyl)-N-dodecyl-octadecansäureamid
M.49 N-(2-L-Valinamido-2-desoxy-β-D-glucopyranosyl)-N-tetradecyl-dodecansäureamid
M.50 N-(2-L-Valinamido-2-desoxy-β-D-glucopyranosyl)-N-tetradecyl-octadecansäureamid

M.51 N-(2-L-Valinamido-2-desoxy-β-D-glucopyranosyl)-N-octadecyl-dodecansäureamid
M.52 N-(2-L-Valinamido-2-desoxy-β-D-glucopyranosyl)-N-octadecyl-tetradecansäureamid
M.53 N-(2-L-Valinamido-2-desoxy-β-D-glucopyranosyl)-N-octadecyl-octadecansäureamid
M.54 N-(2-L-Leucinamido-2-desoxy-β-D-glucopyranosyl)-N-dodecyl-dodecansäureamid
M.55 N-(2-L-Leucinamido-2-desoxy-β-D-glucopyranosyl)-N-dodecyl-tetradecansäureamid
M.56 N-(2-L-Leucinamido-2-desoxy-β-D-glucopyranosyl)-N-dodecyl-hexadecansäureamid
M.57 N-(2-L-Leucinamido-2-desoxy-β-D-glucopyranosyl)-N-dodecyl-octadecansäureamid
M.58 N-(2-L-Leucinamido-2-desoxy-β-D-glucopyranosyl)-N-tetradecyl-dodecansäureamid
M.59 N-(2-L-Leucinamido-2-desoxy-β-D-glucopyranosyl)-N-tetradecyl-tetradecansäureamid
M.60 N-(2-L-Leucinamido-2-desoxy-β-D-glucopyranosyl)-N-tetradecyl-hexadecansäureamid
M.61 N-(2-L-Leucinamido-2-desoxy-β-D-glucopyranosyl)-N-tetradecyl-octadecansäureamid
M.62 N-(2-L-Leucinamido-2-desoxy-β-D-glucopyranosyl)-N-hexadecyl-dodecansäureamid
M.63 N-(2-L-Leucinamido-2-desoxy-β-D-glucopyranosyl)-N-hexadecyl-tetradecansäureamid
M.64 N-(2-L-Leucinamido-2-desoxy-β-D-glucopyranosyl)-N-hexadecyl-hexadecansäureamid
M.65 N-(2-L-Leucinamido-2-desoxy-β-D-glucopyranosyl)-N-hexadecyl-octadecansäureamid
M.66 N-(2-L-Leucinamido-2-desoxy-β-D-glucopyranosyl)-N-octadecyl-dodecansäureamid
M.67 N-(2-L-Leucinamido-2-desoxy-β-D-glucopyranosyl)-N-octadecyl-tetradecansäureamid
M.68 N-(2-L-Leucinamido-2-desoxy-β-D-glucopyranosyl)-N-octadecyl-hexadecansäureamid
M.69 N-(2-L-Leucinamido-2-desoxy-β-D-glucopyranosyl)-N-octadecyl-octadecansäureamid
M.70 N-(2-Sarcosinamido-2-desoxy-β-D-glucopyranosyl)-N-dodecyl-dodecansäureamid
M.71 N-(2-Sarcosinamido-2-desoxy-β-D-glucopyranosyl)-N-dodecyl-octadecansäureamid
M.72 N-(2-Sarcosinamido-2-desoxy-β-D-glucopyranosyl)-N-tetradecyl-dodecansäureamid
M.73 N-(2-Sarcosinamido-2-desoxy-β-D-glucopyranosyl)-N-tetradecyl-octadecansäureamid
M.74 N-(2-Sarcosinamido-2-desoxy-β-D-glucopyranosyl)-N-octadecyl-dodecansäureamid
M.75 N-(2-Sarcosinamido-2-desoxy-β-D-glucopyranosyl)-N-octadecyl-tetradecansäureamid
M.76 N-(2-Sarcosinamido-2-desoxy-β-D-glucopyranosyl)-N-octadecyl-octadecansäureamid
M.77 N-(2-L-Serinamido-2-desoxy-β-D-glucopyranosyl)-N-dodecyl-dodecansäureamid
M.78 N-(2-L-Serinamido-2-desoxy-β-D-glucopyranosyl)-N-dodecyl-octadecansäureamid
M.79 N-(2-L-Serinamido-2-desoxy-β-D-glucopyranosyl)-N-tetradecyl-dodecansäureamid
M.80 N-(2-L-Serinamido-2-desoxy-β-D-glucopyranosyl)-N-tetradecyl-octadecansäureamid
M.81 N-(2-L-Serinamido-2-desoxy-β-D-glucopyranosyl)-N-octadecyl-dodecansäureamid
M.82 N-(2-L-Serinamido-2-desoxy-β-D-glucopyranosyl)-N-octadecyl-tetradecansäureamid
M.83 N-(2-L-Serinamido-2-desoxy-β-D-glucopyranosyl)-N-octadecyl-octadecansäureamid
M.84 N-(2-L-Lysinamido-2-desoxy-β-D-glucopyranosyl)-N-dodecyl-dodecansäureamid
M.85 N-(2-L-Lysinamido-2-desoxy-β-D-glucopyranosyl)-N-dodecyl-octadecansäureamid
M.86 N-(2-L-Lysinamido-2-desoxy-β-D-glucopyranosyl)-N-tetradecyl-dodecansäureamid
M.87 N-(2-L-Lysinamido-2-desoxy-β-D-glucopyranosyl)-N-tetradecyl-octadecansäureamid
M.88 N-(2-L-Lysinamido-2-desoxy-β-D-glucopyranosyl)-N-octadecyl-dodecansäureamid
M.89 N-(2-L-Lysinamido-2-desoxy-β-D-glucopyranosyl)-N-octadecyl-tetradecansäureamid
M.90 N-(2-L-Lysinamido-2-desoxy-β-D-glucopyranosyl)-N-octadecyl-octadecansäureamid
M.91 N-(2-L-Asparaginamido-2-desoxy-β-D-glucopyranosyl)-N-dodecyl-dodecansäureamid
M.92 N-(2-L-Asparaginamido-2-desoxy-β-D-glucopyranosyl)-N-dodecyl-octadecansäueramid
M.93 N-(2-L-Asparaginamido-2-desoxy-β-D-glucopyranosyl)-N-tetradecyl-dodecansäureamid
M.94 N-(2-L-Asparaginamido-2-desoxy-β-D-glucopyranosyl)-N-tetradecyl-octadecansäureamid
M.95 N-(2-L-Asparaginamido-2-desoxy-β-D-glucopyranosyl)-N-octadecyl-dodecansäureamid
M.96 N-(2-L-Asparaginamido-2-desoxy-β-D-glucopyranosyl)-N-octadecyl-tetradecansäureamid
M.97 N-(2-L-Asparaginamido-2-desoxy-β-D-glucopyranosyl)-N-octadecyl-octadecansäueamid
M.98 N(-2-L-Glutaminamido-2-desoxy-β-D-glucopyranosyl)-N-dodecyl-dodecansäureamid
M.99 N(-2-L-Glutaminamido-2-desoxy-β-D-glucopyranosyl)-N-dodecyl-octadecansäureamid
M.100 N(-2-L-Glutaminamido-2-desoxy-β-D-glucopyranosyl)-N-tetradecyl-dodecansäureamid
M.101 N(-2-L-Glutaminamido-2-desoxy-β-D-glucopyranosyl)-N-tetradecyl-octadecansäureamid
M.102 N(-2-L-Glutaminamido-2-desoxy-β-D-glucopyranosyl)-N-octadecyl-dodecansäureamid
M.103 N(-2-L-Glutaminamido-2-desoxy-β-D-glucopyranosyl)-N-octadecyl-tetradecansäureamid
M.104 N(-2-L-Glutaminamido-2-desoxy-β-D-glucopyranosyl)-N-octadecyl-octadecansäureamid
M.105 N-(2-Glycinamido-2-desoxy-β-D-galactopyranosyl)-N-dodecyl-dodecansäureamid
M.106 N-(2-Glycinamido-2-desoxy-β-D-galactopyranosyl)-N-dodecyl-octadecansäureamid
M.107 N-(2-Glycinamido-2-desoxy-β-D-galactopyranosyl)-N-tetradecyl-dodecansäureamid
M.108 N-(2-Glycinamido-2-desoxy-β-D-galactopyranosyl)-N-tetradecyl-octadecansäureamid

M.109 N-(2-Glycinamido-2-desoxy-β-D-galactopyranosyl)-N-octadecyl-dodecansäureamid
M.110 N-(2-Glycinamido-2-desoxy-β-D-galactopyranosyl)-N-octadecyl-tetradecansäureamid
M.111 N-(2-Glycinamido-2-desoxy-β-D-galactopyranosyl)-N-octadecyl-octadecansäureamid
M.112 N-(2-L-Alaninamido-2-desoxy-β-D-galactopyranosyl)-N-dodecyl-dodecansäureamid
M.113 N-(2-L-Alaninamido-2-desoxy-β-D-galactopyranosyl)-N-dodecyl-octadecansäureamid
M.114 N-(2-L-Alaninamido-2-desoxy-β-D-galactopyranosyl)-N-tetradecyl-dodecansäureamid
M.115 N-(2-L-Alaninamido-2-desoxy-β-D-galactopyranosyl)-N-tetradecyl-octadecansäureamid
M.116 N-(2-L-Alaninamido-2-desoxy-β-D-galactopyranosyl)-N-octadecyl-dodecansäureamid
M.117 N-(2-L-Alaninamido-2-desoxy-β-D-galactopyranosyl)-N-octadecyl-tetradecansäureamid
M.118 N-(2-L-Alaninamido-2-desoxy-β-D-galactopyranosyl)-N-octadecyl-octadecansäureamid
M.119 N-(2-L-Leucinamido-2-desoxy-β-D-galactopyranosyl)-N-dodecyl-dodecansäureamid
M.120 N-(2-L-Leucinamido-2-desoxy-β-D-galactopyranosyl)-N-dodecyl-octadecansäureamid
M.121 N-(2-L-Leucinamido-2-desoxy-β-D-galactopyranosyl)-N-tetradecyl-dodecansäureamid
M.122 N-(2-L-Leucinamido-2-desoxy-β-D-galactopyranosyl)-N-tetradecyl-octadecansäureamid
M.123 N-(2-L-Leucinamido-2-desoxy-β-D-galactopyranosyl)-N-octadecyl-dodecansäureamid
M.124 N-(2-L-Leucinamido-2-desoxy-β-D-galactopyranosyl)-N-octadecyl-tetradecansäueramid
M.125 N-(2-L-Leucinamido-2-desoxy-β-D-galactopyranosyl)-N-octadecyl-octadecansäureamid

Nach der unter M angeführten Methode wurden die folgenden Verbindungen der allgemeinen Struktur I (X = O) erhalten.

M.126 N-(2-Glycinamido-2-desoxy-β-D-glucopyranosyl)-N-dodecyl-O-dodecyl-carbamat
M.127 N-(2-Glycinamido-2-desoxy-β-D-glucopyranosyl)-N-dodecyl-O-tetradecyl-carbamat
M.128 N-(2-Glycinamido-2-desoxy-β-D-glucopyranosyl)-N-dodecyl-O-octadecyl-carbamat
M.129 N-(2-Glycinamido-2-desoxy-β-D-glucopyranosyl)-N-tetradecyl-O-dodecyl-carbamat
M.130 N-(2-Glycinamido-2-desoxy-β-D-glucopyranosyl)-N-tetradecyl-O-octadecyl-carbamat
M.131 N-(2-Glycinamido-2-desoxy-β-D-glucopyranosyl)-N-octadecyl-O-dodecyl-carbamat
M.132 N-(2-Glycinamido-2-desoxy-β-D-glucopyranosyl)-N-octadecyl-O-tetradecyl-carbamat
M.133 N-(2-Glycinamido-2-desoxy-β-D-glucopyranosyl)-N-octadecyl-O-octadecyl-carbamat
M.134 N-(2-L-Alaninamido-2-desoxy-β-D-glucopyranosyl)-N-dodecyl-O-dodecyl-carbamat
M.135 N-(2-L-Alaninamido-2-desoxy-β-D-glucopyranosyl)-N-dodecyl-O-tetradecyl-carbamat
M.136 N-(2-L-Alaninamido-2-desoxy-β-D-glucopyranosyl)-N-dodecyl-O-octadecyl-carbamat
M.137 N-(2-L-Alaninamido-2-desoxy-β-D-glucopyranosyl)-N-tetradecyl-O-dodecyl-carbamat
M.138 N-(2-L-Alaninamido-2-desoxy-β-D-glucopyranosyl)-N-tetradecyl-O-octadecyl-carbamat
M.139 N-(2-L-Alaninamido-2-desoxy-β-D-glucopyranosyl)-N-octadecyl-O-dodecyl-carbamat
M.140 N-(2-L-Alaninamido-2-desoxy-β-D-glucopyranosyl)-N-octadecyl-O-tetradecyl-carbamat
M.141 N-(2-L-Alaninamido-2-desoxy-β-D-glucopyranosyl)-N-octadecyl-O-octadecyl-carbamat
M.142 N-(2-L-Leucinamido-2-desoxy-β-D-glucopyranosyl)-N-dodecyl-O-dodecyl-carbamat
M.143 N-(2-L-Leucinamido-2-desoxy-β-D-glucopyranosyl)-N-dodecyl-O-tetradecyl-carbamat
M.144 N-(2-L-Leucinamido-2-desoxy-β-D-glucopyranosyl)-N-dodecyl-O-octadecyl-carbamat
M.145 N-(2-L-Leucinamido-2-desoxy-β-D-glucopyranosyl)-N-tetradecyl-O-dodecyl-carbamat
M.146 N-(2-L-Leucinamido-2-desoxy-β-D-glucopyranosyl)-N-tetradecyl-O-octadecyl-carbamat
M.147 N-(2-L-Leucinamido-2-desoxy-β-D-glucopyranosyl)-N-octadecyl-O-dodecyl-carbamat
M.148 N-(2-L-Leucinamido-2-desoxy-β-D-glucopyranosyl)-N-octadecyl-O-tetradecyl-carbamat
M.149 N-(2-L-Leucinamido-2-desoxy-β-D-glucopyranosyl)-N-octadecyl-O-octadecyl-carbamat
M.150 N-(2-Glycinamido-2-desoxy-β-D-galactopyranosyl)-N-dodecyl-O-dodecyl-carbamat
M.151 N-(2-Glycinamido-2-desoxy-β-D-galactopyranosyl)-N-dodecyl-O-tetradecyl-carbamat
M.152 N-(2-Glycinamido-2-desoxy-β-D-galactopyranosyl)-N-dodecyl-O-octadecyl-carbamat
M.153 N-(2-Glycinamido-2-desoxy-β-D-galactopyranosyl)-N-tetradecyl-O-dodecyl-carbamat
M.154 N-(2-Glycinamido-2-desoxy-β-D-galactopyranosyl)-N-tetradecyl-O-octadecyl-carbamat
M.155 N-(2-Glycinamido-2-desoxy-β-D-galactopyranosyl)-N-octadecyl-O-dodecyl-carbamat
M.156 N-(2-Glycinamido-2-desoxy-β-D-galactopyranosyl)-N-octadecyl-O-tetradecyl-carbamat
M.157 N-(2-Glycinamido-2-desoxy-β-D-galactopyranosyl)-N-octadecyl-O-octadecyl-carbamat

Nach der unter M angeführten Methode wurden die folgenden Verbindungen der allgemeinen Struktur I (X = -NH-) erhalten.

M.158 N-(2-Glycinamido-2-desoxy-β-D-glucopyranosyl)-N-dodecyl-N'-dodecyl-harnstoff
M.159 N-(2-Glycinamido-2-desoxy-β-D-glucopyranosyl)-N-dodecyl-N'-tetradecyl-harnstoff
M.160 N-(2-Glycinamido-2-desoxy-β-D-glucopyranosyl)-N-dodecyl-N'-octadecyl-harnstoff
M.161 N-(2-Glycinamido-2-desoxy-β-D-glucopyranosyl)-N-tetradecyl-N'-dodecyl-harnstoff
M.162 N-(2-Glycinamido-2-desoxy-β-D-glucopyranosyl)-N-tetradecyl-N'-octadecyl-harnstoff

| | |
|---|---|
| M.163 | N-(2-Glycinamido-2-desoxy-β-D-glucopyranosyl)-N-octadecyl-N'-dodecyl-harnstoff |
| M.164 | N-(2-Glycinamido-2-desoxy-β-D-glucopyranosyl)-N-octadecyl-N'-tetradecyl-harnstoff |
| M.165 | N-(2-Glycinamido-2-desoxy-β-D-glucopyranosyl)-N-octadecyl-N'-octadecyl-harnstoff |
| M.166 | N-(2-L-Alaninamido-2-desoxy-β-D-glucopyranosyl)-N-dodecyl-N'-dodecyl-harnstoff |
| M.167 | N-(2-L-Alaninamido-2-desoxy-β-D-glucopyranosyl)-N-dodecyl-N'-tetradecyl-harnstoff |
| M.168 | N-(2-L-Alaninamido-2-desoxy-β-D-glucopyranosyl)-N-tetradecyl-N'-octadecyl-harnstoff |
| M.169 | N-(2-L-Alaninamido-2-desoxy-β-D-glucopyranosyl)-N-tetradecyl-N'-dodecyl-harnstoff |
| M.170 | N-(2-L-Alaninamido-2-desoxy-β-D-glucopyranosyl)-N-tetradecyl-N'-octadecyl-harnstoff |
| M.171 | N-(2-L-Alaninamido-2-desoxy-β-D-glucopyranosyl)-N-octadecyl-N'-dodecyl-harnstoff |
| M.172 | N-(2-L-Alaninamido-2-desoxy-β-D-glucopyranosyl)-N-octadecyl-N'-tetradecyl-harnstoff |
| M.173 | N-(2-L-Alaninamido-2-desoxy-β-D-glucopyranosyl)-N-octadecyl-N'-octadecyl-harnstoff |
| M.174 | N-(2-L-Leucinamido-2-desoxy-β-D-glucopyranosyl)-N-dodecyl-N'-dodecyl-harnstoff |
| M.175 | N-(2-L-Leucinamido-2-desoxy-β-D-glucopyranosyl)-N-dodecyl-N'-tetradecyl-harnstoff |
| M.176 | N-(2-L-Leucinamido-2-desoxy-β-D-glucopyranosyl)-N-dodecyl-N'-octadecyl-harnstoff |
| M.177 | N-(2-L-Leucinamido-2-desoxy-β-D-glucopyranosyl)-N-tetradecyl-N'-dodecyl-harnstoff |
| M.178 | N-(2-L-Leucinamido-2-desoxy-β-D-glucopyranosyl)-N-tetradecyl-N'-octadecyl-harnstoff |
| M.179 | N-(2-L-Leucinamido-2-desoxy-β-D-glucopyranosyl)-N-octadecyl-N'-dodecyl-harnstoff |
| M.180 | N-(2-L-Leucinamido-2-desoxy-β-D-glucopyranosyl)-N-octadecyl-N'-tetradecyl-harnstoff |
| M.181 | N-(2-L-Leucinamido-2-desoxy-β-D-glucopyranosyl)-N-octadecyl-N'-octadecyl-harnstoff |
| M.182 | N-(2-Glycinamido-2-desoxy-β-D-galactopyranosyl)-N-dodecyl-N'-dodecyl-harnstoff |
| M.183 | N-(2-Glycinamido-2-desoxy-β-D-galactopyranosyl)-N-dodecyl-N'-tetradecyl-harnstoff |
| M.184 | N-(2-Glycinamido-2-desoxy-β-D-galactopyranosyl)-N-dodecyl-N'-octadecyl-harnstoff |
| M.185 | N-(2-Glycinamido-2-desoxy-β-D-galactopyranosyl)-N-tetradecyl-N'-dodecyl-harnstoff |
| M.186 | N-(2-Glycinamido-2-desoxy-β-D-galactopyranosyl)-N-tetradecyl-N'-octadecyl-harnstoff |
| M.187 | N-(2-Glycinamido-2-desoxy-β-D-galactopyranosyl)-N-octadecyl-N'-dodecyl-harnstoff |
| M.188 | N-(2-Glycinamido-2-desoxy-β-D-galactopyranosyl)-N-octadecyl-N'-tetradecyl-harnstoff |
| M.189 | N-(2-Glycinamido-2-desoxy-β-D-galactopyranosyl)-N-octadecyl-N'-octadecyl-harnstoff |

## Physikalische Daten

Alle chemischen Reaktionen wurden dünnschichtchromatografisch verfolgt. Die angegebenen $R_F$-Werte wurden auf Kieselgel 60 - Dünnschichtplatten (E.Merck, Darmstadt) ermittelt. Die Zusammensetzung der Laufmittelgemische ist in Volumenteilen angegeben.

Optische Drehwerte wurden mit einem Polarimeter, Typ 241, der Firma Perkin-Elmer in 1 dm-Küvetten bei 589 nm (Na-D-Linie) bestimmt. Die Konzentration C der Substanz im Lösungsmittel ist in Prozent (Gewicht/Volumen) angegebenen. Die Elementaranalysen in den angegebenen Fällen ergaben befriedigende Werte für C, H und N mit folgenden Fehlergrenzen:

C + 0,38 %
H + 0,31 %
N + 0,52 %

## Daten für die nach der Methode A hergestellten Glycosylamine der allgemeinen Struktur IV

$R_F$-Werte in Dichlormethan/Methanol = 5:1
2-Benzyloxycarbonylamino-2-desoxy-D-glucopyranose
$R_F$ = 0,31
2-Benzyloxycarbonylamino-2-desoxy-D-galactopyranose
$R_F$ = 0,29
Die hergestellten Glycosylamine der Struktur IV wiesen im o.g. Laufmittel sehr ähnliche $R_F$- Werte auf, die im Bereich von 0,46 bis 0,49 lagen. Dabei hatten die Glycosylamine mit kürzeren Alkylketten geringere $R_F$-Werte als die Glycosylamine mit längeren Alkylketten.
Beispiele:

| Verb. | $R_F$ |
|---|---|
| A 2 | 0,47 |
| A 3 | 0,47 |
| A 4 | 0,47 |
| A 6 | 0,49 |
| A 9 | 0,46 |
| A 12 | 0,48 |

Daten für die nach der Methode B hergestellten Verbindungen der allgemeinen Struktur VI (X = $-CH_2-$)

$R_F$-Werte in Dichlormethan/Methanol = 10:1

| Verb. | $R_F$ | Elementaranalyse |
|---|---|---|
| B 3 | 0,37 | |
| B 6 | 0,38 | $C_{38}H_{66}N_2O_7$ |
| B 8 | 0,38 | |
| B 9 | 0,39 | $C_{44}H_{78}N_2O_7$ |
| B 10 | 0,38 | $C_{40}H_{70}N_2O_7$ |
| B 12 | 0,38 | $C_{44}H_{78}N_2O_7$ |
| B 13 | 0,38 | $C_{46}H_{82}N_2O_7$ |
| B 16 | 0,38 | $C_{42}H_{74}N_2O_7$ |

| Verb. | $R_F$ | Elementaranalyse |
|---|---|---|
| B 20 | 0,38 | $C_{44}H_{78}N_2O_7$ |
| B 21 | 0,38 | $C_{46}H_{82}N_2O_7$ |
| B 23 | 0,38 | $C_{50}H_{90}N_2O_7$ |
| B 25 | 0,34 | $C_{38}H_{66}N_2O_7$ |
| B 26 | 0,35 | $C_{40}H_{70}N_2O_7$ |
| B 27 | 0,36 | $C_{44}H_{78}N_2O_7$ |
| B 30 | 0,36 | $C_{46}H_{82}N_2O_7$ |
| B 31 | 0,36 | |
| B 33 | 0,37 | $C_{50}H_{90}N_2O_7$ |

Daten für die nach der Methode C hergestellten Verbindungen der allgemeinen Struktur IX (X = $-CH_2-$)

$R_F$-Werte in Toluol/Ethanol = 20:1

| Verb. | $R_F$ | Elementar-analyse | Schmelz-punkt | $[\alpha]_D$ |
|---|---|---|---|---|
| C 1 | 0,36 | $C_{44}H_{72}N_2O_{10}$ | | + 15,6° (c=1,0; THF) |
| C 2 | 0,36 | $C_{46}H_{76}N_2O_{10}$ | 73° | + 14,1° (c=1,0; THF) |
| C 6 | 0,36 | $C_{52}H_{88}N_2O_{10}$ | | |
| C 7 | 0,36 | $C_{50}H_{84}N_2O_{10}$ | 65° | +13,5° (c=1,0; $CH_2Cl_2$) |
| C 9 | 0,36 | | | |
| C 10 | 0,33 | $C_{50}H_{84}N_2O_{10}$ | | |
| C 12 | 0,33 | $C_{52}H_{88}N_2O_{10}$ | | |

Daten für die nach der Methode E hergestellten Verbindungen der allgemeinen Struktur VI (X = -O-)

$R_F$-Werte in Dichlormethan/Methanol = 10 : 1

| Verb. | $R_F$ | Elementaranalyse |
|---|---|---|
| E 1 | 0,36 | $C_{39}H_{68}N_2O_8$ |
| E 3 | 0,36 | $C_{45}H_{80}N_2O_8$ |
| E 4 | 0,36 | |
| E 6 | 0,36 | $C_{44}H_{80}N_2O_8$ |
| E 8 | 0,36 | $C_{50}H_{92}N_2O_8$ |
| E 9 | 0,33 | $C_{39}H_{68}N_2O_8$ |
| E 11 | 0,33 | $C_{45}H_{80}N_2O_8$ |
| E 15 | 0,34 | $C_{47}H_{84}N_2O_8$ |
| E 17 | 0,34 | $C_{47}H_{84}N_2O_8$ |

Daten für die nach der Methode F hergestellten Verbindungen der allgemeinen Struktur VI (X = -NH-)

$R_F$-Werte in Dichlormethan/Methanol = 10 : 1

| Verb. | $R_F$ | Elementaranalyse |
|---|---|---|
| F 1 | 0,30 | $C_{39}H_69N_3O_7$ |
| F 3 | 0,30 | $C_{45}H_{81}N_3O_7$ |
| F 5 | 0,30 | $C_{47}H_{85}N_3O_7$ |
| F 6 | 0,30 | $C_{45}H_{81}N_3O_7$ |
| F 8 | 0,31 | $C_{51}H_{93}N_3O_7$ |
| F 9 | 0,28 | |
| F 14 | 0,28 | $C_{47}H_{85}N_3O_7$ |
| F 16 | 0,28 | $C_{47}H_{85}N_3O_7$ |

Daten für die nach der allgemeinen Methode G hergestellten Verbindungen der allgemeinen Struktur (X = $-CH_2-$)

$R_F$-Werte in Dichlormethan / Methanol / 25 % wäßr. Ammoniak = 10 : 1,5 : 0,1

| Verb. | $R_F$ | Elementaranalyse | $[\alpha]_D$ |
|---|---|---|---|
| G 1 | 0,31 | $C_{30}H_{60}N_2O_5$ x $C_2H_4O_2$ | + 12,0°; C = 1,0; THF |
| G 2 | 0,31 | $C_{32}H_{64}N_2O_5$ x $C_2H_4O_2$ | |
| G 3 | 0,32 | | |
| G 4 | 0,33 | $C_{36}H_{72}N_2O_5$ x $C_2H_4O_2$ | |
| G 5 | 0,31 | $C_{32}H_{64}N_2O_5$ x $C_2H_4O_2$ | + 11,3°; C = 1,0; THF |
| G 8 | 0,32 | $C_{38}H_{76}N_2O_5$ x $C_2H_4O_2$ | + 8,5°; C = 1,0; THF |
| G 10 | 0,33 | $C_{36}H_{72}N_2O_5$ x $C_2H_4O_2$ | |
| G 13 | 0,32 | $C_{36}H_{72}N_2O_5$ x $C_2H_4O_2$ | + 9,7°; C = 1,0: THF |
| G 14 | 0,33 | $C_{38}H_{76}N_2O_5$ x $C_2H_4O_2$ | |
| G 15 | 0,34 | $C_{40}H_{80}N_2O_5$ x $C_2H_4O_2$ | |
| G 16 | 0,34 | $C_{42}H_{84}N_2O_5$ x $C_2H_4O_2$ | + 7,0°; C = 1,0; THF |
| G 17 | 0,29 | $C_{30}H_{60}N_2O_5$ x $C_2H_4O_2$ | + 24,0°; C = 1,0; THF |
| G 19 | 0,29 | $C_{36}H_{72}N_2O_5$ x $C_2H_4O_2$ | + 21,8°; C = 1,0; THF |
| G 22 | 0,29 | $C_{36}H_{72}N_2O_5$ x $C_2H_4O_2$ | |
| G 24 | 0,31 | $C_{42}H_{84}N_2O_5$ x $C_2H_4O_2$ | + 17,3°; C = 1,0; THF |

Daten für die nach der allgemeinen Methode H hergestellten Verbindungen der allgemeinen Struktur X (X = -O-)

$R_F$-Werte in Dichlormethan / Methanol / 25 % wäßr. Ammoniak = 10 : 1,5 : 0,1

| Verb. | $R_F$ | Elementaranalyse | $[\alpha]_D$ |
|---|---|---|---|
| H 2 | 0,30 | $C_{33}H_{66}N_2O_6$ x $C_2H_4O_2$ | + 8,4°; C = 1,0, THF |
| H 6 | 0,30 | | |
| H 8 | 0,32 | $C_{43}H_{86}N_2O_6$ x $C_2H_4O_2$ | |
| H 11 | 0,26 | | |

Daten für die nach der allgemeinen Methode 1 hergestellten Verbindungen der allgemeinen Struktur X (X = -NH-)

$R_F$-Werte in Dichlormethan / Methanol / 25 % wäßr. Ammoniak = 10 : 1,5 : 0,1

| Verb. | $R_F$ | Elementaranalyse |
|---|---|---|
| I 1 | 0,26 | $C_{31}H_{63}N_3O_5$ x $C_2H_4O_2$ |
| I 3 | 0,28 | |
| I 5 | 0,28 | $C_{39}H_{79}N_3O_5$ x $C_2H_4O_2$ |
| I 11 | 0,24 | $C_{37}H_{75}N_3O_5$ x $C_2H_4O_2$ |
| I 13 | 0,24 | |

Daten für die nach der allgemeinen Methode J hergestellten Verbindungen der allgemeinen Struktur XII (X = $-CH_2-$)

$R_F$-Werte in Dichlormethan / Methanol / 25 % wäßr. Ammoniak = 10 : 1,5 : 0,1

| Verb | $R_F$ | Elementaranalyse |
|---|---|---|
| J 1 | 0,33 | $C_{40}H_{69}N_3O_8$ |
| J 3 | 0,35 | $C_{44}H_{77}N_3O_8$ |
| J 4 | 0,35 | $C_{46}H_{81}N_3O_8$ |
| J 5 | 0,35 | $C_{42}H_{73}N_3O_8$ |
| J 7 | 0,35 | $C_{46}H_{81}N_3O_8$ |
| J 8 | 0,36 | $C_{48}H_{85}N_3O_8$ |
| J 10 | 0,36 | $C_{46}H_{81}N_3O_8$ |
| J 12 | 0,37 | $C_{50}H_{89}N_3O_8$ |
| J 13 | 0,36 | $C_{46}H_{81}N_3O_8$ |
| J 16 | 0,38 | $C_{52}H_{93}N_3O_8$ |
| J 17 | 0,34 | $C_{41}H_{71}N_3O_8$ |
| J 18 | 0,34 | $C_{43}H_{75}N_3O_8$ |
| J 20 | 0,35 | $C_{47}H_{83}N_3O_8$ |
| J 21 | 0,34 | $C_{43}H_{75}N_3O_8$ |
| J 23 | 0,35 | $C_{47}H_{83}N_3O_8$ |
| J 25 | 0,36 | $C_{45}H_{79}N_3O_8$ |
| J 29 | 0,37 | $C_{47}H_{83}N_3O_8$ |
| J 31 | 0,38 | $C_{51}H_{91}N_3O_8$ |
| J 34 | 0,43 | $C_{47}H_{83}N_3O_8$ |
| J 36 | 0,44 | $C_{49}H_{76}N_3O_8$ |
| J 37 | 0,45 | $C_{47}H_{83}N_3O_8$ |
| J 38 | 0,45 | $C_{49}H_{87}N_3O_8$ |
| J 41 | 0,55 | $C_{53}H_{87}N_3O_8$ |
| J 43 | 0,56 | $C_{55}H_{91}N_3O_8$ |
| J 45 | 0,55 | $C_{55}H_{91}N_3O_8$ |

| Verb | $R_F$ | Elementaranalyse |
|---|---|---|
| J 47 | 0,48 | $C_{43}H_{75}N_3O_8$ |
| J 50 | 0,51 | $C_{51}H_{91}N_3O_8$ |
| J 51 | 0,51 | $C_{49}H_{87}N_3O_8$ |
| J 54 | 0,54 | $C_{44}H_{77}N_3O_8$ |
| J 55 | 0,56 | $C_{46}H_{81}N_3O_8$ |
| J 57 | 0,57 | $C_{50}H_{89}N_3O_8$ |
| J 58 | 0,54 | $C_{46}H_{81}N_3O_8$ |
| J 60 | 0,57 | $C_{50}H_{89}N_3O_8$ |
| J 61 | 0,58 | $C_{52}H_{93}N_3O_8$ |
| J 63 | 0,57 | $C_{50}H_{89}N_3O_8$ |
| J 66 | 0,58 | $C_{50}H_{89}N_3O_8$ |
| J 69 | 0,59 | $C_{56}H_{101}N_3O_8$ |
| J 71 | 0,42 | $C_{47}H_{83}N_3O_8$ |
| J 74 | 0,42 | $C_{47}H_{83}N_3O_8$ |
| J 75 | 0,42 | $C_{49}H_{87}N_3O_8$ |
| J 77 | 0,35 | $C_{48}H_{77}N_3O_9$ |
| J 79 | 0,35 | $C_{50}H_{81}N_3O_9$ |
| J 80 | 0,37 | $C_{56}H_{93}N_3O_9$ |
| J 84 | 0,54 | $C_{52}H_{88}N_4O_{10}$ |
| J 86 | 0,55 | $C_{54}H_{88}N_4O_{10}$ |
| J 88 | 0,55 | $C_{58}H_{96}N_4O_{10}$ |
| J 90 | 0,57 | $C_{64}H_{108}N_4O_{10}$ |
| J 91 | 0,58 | $C_{49}H_{77}N_3O_{10}$ |
| J 92 | 0,58 | $C_{55}H_{89}N_3O_{10}$ |
| J 96 | 0,60 | $C_{57}H_{93}N_3O_{10}$ |
| J 98 | 0,48 | $C_{50}H_{79}N_3O_{10}$ |
| J 99 | 0,50 | $C_{56}H_{91}N_3O_{10}$ |
| J 102 | 0,51 | $C_{56}H_{91}N_3O_{10}$ |
| J 103 | 0,51 | $C_{58}H_{95}N_3O$ |

| Verb. | $R_F$ | Elementaranalyse |
|---|---|---|
| J 105 | 0,28 | $C_{40}H_{69}N_3O_8$ |
| J 109 | 0,30 | $C_{46}H_{81}N_3O_8$ |
| J 116 | 0,32 | $C_{47}H_{83}N_3O_8$ |
| J 122 | 0,54 | $C_{52}H_{93}N_3O_8$ |
| J 123 | 0,54 | $C_{50}H_{89}N_3O_8$ |
| J 124 | 0,54 | $C_{52}H_{93}N_3O_8$ |

Verbindungen der allgemeinen Struktur XII (X = -O-)

| Verb. | $R_F$ | Elementaranalyse |
|---|---|---|
| J 127 | 0,31 | $C_{43}H_{75}N_3O_9$ |
| J 131 | 0,34 | $C_{47}H_{83}N_3O_9$ |
| J 133 | 0,35 | $C_{53}H_{95}N_3O_9$ |
| J 135 | 0,32 | $C_{44}H_{77}N_3O_9$ |
| J 139 | 0,34 | $C_{48}H_{85}N_3O_9$ |
| J 143 | 0,53 | $C_{47}H_{83}N_3O_9$ |
| J 147 | 0,55 | $C_{51}H_{91}N_3O_9$ |
| J 149 | 0,58 | $C_{57}H_{103}N_3O_9$ |
| J 151 | 0,27 | $C_{43}H_{75}N_3O_9$ |
| J 152 | 0,28 | $C_{47}H_{83}N_3O_9$ |

Verbindungen der allgemeinen Struktur XII (X = -NH-)

| Verb. | $R_F$ | Elementaranalyse |
|---|---|---|
| J 158 | 0,29 | $C_{41}H_{72}N_4O_8$ |
| J 160 | 0,32 | $C_{47}H_{84}N_4O_8$ |
| J 162 | 0,32 | $C_{49}H_{88}N_4O_8$ |
| J 170 | 0,32 | $C_{50}H_{90}N_4O_8$ |
| J 174 | 0,50 | $C_{45}H_{80}N_4O_8$ |
| J 176 | 0,53 | $C_{51}H_{92}N_4O_8$ |
| J 178 | 0,53 | $C_{53}H_{96}N_4O_8$ |
| J 184 | 0,26 | $C_{47}H_{84}N_4O_8$ |
| J 186 | 0,26 | $C_{49}H_{88}N_4O_8$ |

Daten für die nach der allgemeinen Methode M hergestellten Verbindungen der allgemeinen Struktur I (X = $-CH_2-$)

$R_F$-Werte in Dichlormethan / Methanol / 25 % wäßr. Ammoniak = 10 : 3 : 0,4

| Verb | $R_F$ | Elementaranalyse | $[\alpha]_D$ |
|---|---|---|---|
| M 1 | 0,23 | $C_{32}H_{63}N_3O_6 \times C_2H_4O_2$ | + 19,9°; C=1,0, THF |
| M 3 | | $C_{36}H_{71}N_3O_6 \times C_2H_4O_2$ | |
| M 4 | | $C_{38}H_{75}N_3O_6 \times C_2H_4O_2$ | + 12,4°; C=1,0, THF |
| M 5 | 0,21 | $C_{34}H_{67}N_3O_6 \times C_2H_4O_2$ | |
| M 7 | | $C_{38}H_{75}N_3O_6 \times C_2H_4O_2$ | |
| M 8 | 0,26 | $C_{49}H_{79}N_3O_6 \times C_2H_4O_2$ | + 12,5°; C=1,0, HOAc |
| M 10 | | $C_{38}H_{75}N_3O_6 \times C_2H_4O_2$ | |
| M 12 | | $C_{42}H_{83}N_3O_6 \times C_2H_4O_2$ | |

| Verb. | $R_F$ | Elementaranalyse | $[\alpha]_D$ |
|---|---|---|---|
| M 13 | 0,22 | $C_{38}H_{75}N_3O_6 \times C_2H_4O_2$ | +17,3°; C=1,0; THF |
| M 16 | 0,23 | $C_{44}H_{87}N_3O_6 \times C_2H_4O_2$ | +12,5°; C=1,0; THF |
| M 17 | 0,19 | $C_{33}H_{65}N_3O_6 \times C_2H_4O_2$ | |
| M 18 | | $C_{35}H_{69}N_3O_6 \times C_2H_4O_2$ | |
| M 20 | 0,21 | $C_{39}H_{77}N_3O_6 \times C_2H_4O_2$ | +20,0°; C=1,0; THF |
| M 21 | | $C_{35}H_{69}N_3O_6 \times C_2H_4O_2$ | |
| M 23 | | $C_{39}H_{77}N_3O_6 \times C_2H_4O_2$ | |
| M 25 | 0,21 | $C_{37}H_{73}N_3O_6 \times C_2H_4O_2$ | |
| M 29 | | $C_{39}H_{77}N_3O_6 \times C_2H_4O_2$ | +19,7°; C=1,0; THF |
| M 31 | 0,23 | $C_{43}H_{85}N_3O_6 \times C_2H_4O_2$ | |
| M 34 | | $C_{39}H_{77}N_3O_6 \times C_2H_4O_2$ | +12,6°; C=1,0; THF |
| M 36 | | $C_{41}H_{81}N_3O_6 \times C_2H_4O_2$ | |
| M 37 | 0,17 | $C_{39}H_{77}N_3O_6 \times C_2H_4O_2$ | +18,3°; C=1,0; THF |
| M 38 | | $C_{41}H_{81}N_3O_6 \times C_2H_4O_2$ | |
| M 41 | 0,63 | $C_{45}H_{81}N_3O_6 \times C_2H_4O_2$ | + 9,5°; C=1,0; THF |
| M 43 | | $C_{47}H_{85}N_3O_6 \times C_2H_4O_2$ | |
| M 45 | 0,63 | $C_{47}H_{85}N_3O_6 \times C_2H_4O_2$ | +10,1°; C=1,0; THF |
| M 47 | 0,51 | $C_{35}H_{69}N_3O_6 \times C_2H_4O_2$ | |
| M 50 | | $C_{43}H_{89}N_3O_6 \times C_2H_4O_2$ | |
| M 51 | 0,49 | $C_{41}H_{85}N_3O_6 \times C_2H_4O_2$ | +17,3°; C=1,0; DMF |
| M 54 | 0,47 | $C_{36}H_{71}N_3O_6 \times C_2H_4O_2$ | +17,6°; C=1,0; THF |
| M 55 | 0,47 | $C_{38}H_{75}N_3O_6 \times C_2H_4O_2$ | |
| M 57 | | $C_{42}H_{83}N_3O_6 \times C_2H_4O_2$ | |
| M 58 | 0,49 | $C_{38}H_{75}N_3O_6 \times C_2H_4O_2$ | +16,2°; C=1,0; THF |
| M 60 | | $C_{42}H_{83}N_3O_6 \times C_2H_4O_2$ | |
| M 61 | 0,52 | $C_{44}H_{87}N_3O_6 \times C_2H_4O_2$ | +16,4°; C=1,0; THF |
| M 63 | | $C_{42}H_{83}N_3O_6 \times C_2H_4O_2$ | |
| M 66 | 0,51 | $C_{42}H_{83}N_3O_6 \times C_2H_4O_2$ | +13,5°; C=1,0; THF |
| M 69 | | $C_{48}H_{95}N_3O_6 \times C_2H_4O_2$ | |

| Verb. | $R_F$ | Elementaranalyse | $[\alpha]_D$ |
|---|---|---|---|
| M 71 | | $C_{39}H_{77}N_3O_6$ x $C_2H_4O_2$ | |
| M 74 | 0,27 | $C_{39}H_{77}N_3O_6$ x $C_2H_4O_2$ | + 9,6°; C=1,0; THF |
| M 75 | | $C_{41}H_{81}N_3O_6$ x $C_2H_4O_2$ | |
| M 77 | 0,19 | $C_{33}H_{65}N_3H_7$ x $C_2H_4O_2$ | + 3,8°; C=1,0; THF |
| M 79 | | $C_{35}H_{69}N_3O_7$ x $C_2H_4O_2$ | |
| M 80 | 0,22 | $C_{41}H_{81}N_3O_7$ x $C_2H_4O_2$ | |
| M 84 | 0,01 | $C_{36}H_{72}N_4O_6$ x 2($C_2H_4O_2$) | +15,7°; C=1,0; THF |
| M 86 | | $C_{38}H_{76}N_4O_6$ x 2($C_2H_4O_2$ | |
| M 88 | 0,01 | $C_{43}H_{84}N_4O_6$ x 2($C_2H_4O_2$) | +15,5°; C=1,0; FHF |
| M 90 | | $C_{48}H_{96}N_4O_6$ x 2($C_2H_4O_2$) | |
| M 91 | | $C_{34}H_{65}N_3O_8$ | |
| M 92 | 0,05 | $C_{49}H_{77}N_3O_8$ | +20,1°; C=1,0; HOAc |
| M 96 | 0,04 | $C_{42}H_{81}N_3O_8$ | |
| M 98 | 0,02 | $C_{35}H_{67}N_3O_8$ | +15,9°; C=1,0; HOAc |
| M 99 | | $C_{41}H_{79}N_3O_8$ | |
| M 102 | 0,02 | $C_{41}H_{79}N_3O_8$ | +15,4°; C=1,0; HOAc |
| M 103 | | $C_{32}H_{83}N_3O_8$ | |
| M 105 | 0,20 | $C_{32}H_{63}N_3O_6$ x $C_2H_4O_2$ | +28,6°; C=1,0; THF |
| M 109 | 0,21 | $C_{38}H_{75}N_3O_6$ x $C_2H_4O_2$ | |
| M 166 | 0,21 | $C_{39}H_{77}N_3O_6$ x $C_2H_4O_2$ | +30,4°; C=1,0; THF |
| M 122 | 0,45 | $C_{44}H_{87}N_3O_6$ x $C_2H_4O_2$ | |
| M 123 | 0,45 | $C_{32}H_{83}N_3O_6$ x $C_2H_4O_2$ | +24,1°; C=1,0; THF |
| M 124 | 0,45 | $C_{44}H_{87}N_3O_6$ x $C_2H_4O_2$ | |

Verbindungen der allgemeinen Struktur I (X = -O-)

| Verb. | $R_F$ | Elementaranalyse | $[\alpha]_D$ |
|---|---|---|---|
| M 127 | 0,20 | $C_{35}H_{69}N_3O_7$ x $C_2H_4O_2$ | +18,1°; C=1,0; THF |
| M 131 | 0,22 | $C_{39}H_{77}N_3O_7$ x $C_2H_4O_2$ | |
| M 133 | 0,22 | $C_{45}H_{89}N_3O_7$ x $C_2H_4O_2$ | |
| M 135 | 0,17 | $C_{36}H_{71}N_3O_7$ x $C_2H_4O_2$ | +17,4°; C=1,0; THF |
| M 139 | 0,19 | $C_{40}H_{79}N_3O_7$ x $C_2H_4O_2$ | |
| M 143 | 0,44 | $C_{39}H_{77}N_3O_7$ x $C_2H_4O_2$ | +16,4°; C=1,0; THF |
| M 147 | 0,45 | $C_{43}H_{85}N_3O_7$ x $C_2H_4O_2$ | |
| M 149 | 0,45 | $C_{38}H_{97}N_3O_7$ x $C_2H_4O_2$ | |
| M 151 | 0,20 | $C_{35}H_{69}N_3O_7$ x $C_2H_4O_2$ | +25,3°; C=1,0; THF |
| M 152 | 0,20 | $C_{39}H_{77}N_3O_7$ x $C_2H_4O_2$ | |
| M 158 | 0,19 | $C_{33}H_{66}N_4O_6$ x $C_2H_4O_2$ | +14,2°; C=1,0; THF |
| M 160 | 0,20 | $C_{39}H_{78}N_4O_6$ x $C_2H_4O_2$ | |
| M 162 | 0,20 | $C_{41}H_{82}N_4O_6$ x $C_2H_4O_2$ | |
| M 170 | 0,20 | $C_{42}H_{84}N_4O_6$ x $C_2H_4O_2$ | |
| M 174 | 0,39 | $C_{37}H_{74}N_4O_6$ x $C_2H_4O_2$ | |
| M 176 | 0,43 | $C_{43}H_{85}N_4O_6$ x $C_2H_4O_2$ | |
| M 178 | 0,43 | $C_{45}H_{90}N_4O_6$ x $C_2H_4O_2$ | |
| M 184 | 0,16 | $C_{39}H_{78}N_4O_6$ x $C_2H_4O_2$ | |
| M 186 | 0,16 | $C_{41}H_{82}N_4O_6$ x $C_2H_4O_2$ | |

## Patentansprüche

**1.** Verbindungen der allgemeinen Formel I

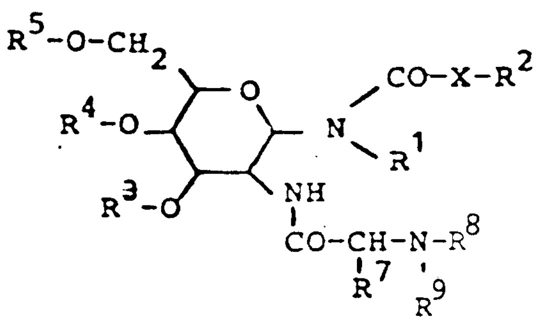

(I)

in der

| | |
|---|---|
| $R^1$ | einen gesättigten oder einfach oder mehrfach ungesättigten Alkylrest mit bis zu 50 Kohlenstoffatomen bedeutet, |
| X | für $-CH_2-$, -O- oder -NH- steht, |
| $R^2$ | Wasserstoff oder einen gesättigten oder einfach oder mehrfach ungesättigten Alkylrest mit bis zu 50 Kohlenstoffatomen bedeutet, |
| $R^3$, $R^4$ und $R^5$ | unabhängig voneinander Wasserstoff oder Acyl $-CO-R^6$ bedeutet, wobei unter $R^6$ ein Alkylrest mit bis zu 10 Kohlenstoffatomen zu verstehen ist, |
| $R^7$ | Wasserstoff, $C_1$-$C_7$-Alkyl-, Hydroxymethyl, 1-Hydroxyethyl, Mercaptomethyl, 2-(Methylthio)-ethyl, 3-Aminopropyl, 3-Ureidopropyl, 3-Guanidylpropyl, 4-Aminobutyl, Carboxymethyl, Carbamoylmethyl, 2-Carboxyethyl, 2-Carbamoylethyl, Benzyl, 4-Hydroxybenzyl, 3-Indolylmethyl oder 4-Imidazolylmethyl bedeutet, und |
| $R^8$ | für Wasserstoff oder Methyl steht und |
| $R^9$ | für Wasserstoff, Acetyl, Benzoyl, Trichloracetyl, Trifluoracetyl, Methoxycarbonyl, t-Butyloxycarbonyl oder Benzyloxycarbonyl steht und |
| $R^7$ und $R^8$ | zusammen $-CH_2-CH_2-CH_2-$ bedeuten können. |

**2.** Verbindungen nach Anspruch 1, in denen $R^1$ für einen geradkettigen oder verzweigten, gesättigten oder ungesättigten Alkylrest mit 1 bis 20 C-Atomen steht.

**3.** Verbindungen nach den Ansprüchen 1 und 2, in denen $R^2$ für Wasserstoff oder für einen geradkettigen oder verzweigten, gesättigten oder ungesättigten Alkylrest mit 1 bis 20 C-Atomen steht.

**4.** Verbindungen nach den Ansprüchen 1 bis 3, in denen die Zuckerreste 2-Amino-2-desoxy-D-glucose oder 2-Amino-2-desoxy-D-galactose sind.

**5.** Verbindungen nach den Ansprüchen 1 bis 4, in denen die 2-Aminogruppe des Aminozuckers mit Glycin, Sarcosin, Hippursäure, Alanin, Valin, Leucin, Isoleucin, Serin, Threonin, Cystein, Methionin, Ornithin, Citrullin, Arginin, Asparaginsäure, Asparagin, Glutaminsäure, Glutamin, Phenylalanin, Tyrosin, Prolin, Tryptophan oder Histidin in der D- oder L-Form oder mit Aminocarbonsäuren wie α-Aminobuttersäure, α-Aminovaleriansäure,α-Aminocapronsäure oder α-Aminoheptansäure in der D- oder in L-Form verbunden ist.

**6.** Verfahren zur Herstellung von Verbindungen der allgemeinen Formel (I)

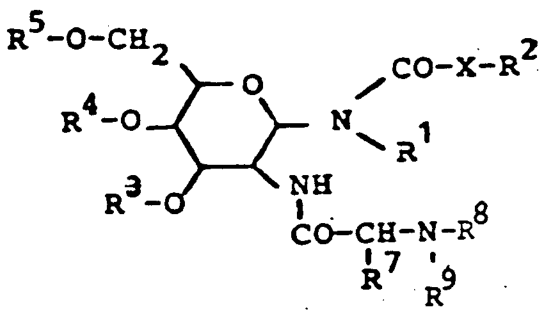

(I)

in der

| | |
|---|---|
| $R^1$ | einen gesättigten oder einfach oder mehrfach ungesättigten Alkylrest mit bis zu 50 Kohlenstoffatomen bedeutet, |
| X | für $-CH_2-$, -O- oder -NH- steht, |
| $R^2$ | Wasserstoff oder einen gesättigten oder einfach oder mehrfach ungesättigten Alkylrest mit bis zu 50 Kohlenstoffatomen bedeutet, |
| $R^3$, $R^4$ und $R^5$ | unabhängig voneinander Wasserstoff oder Acyl $-CO-R^6$ bedeutet, wobei unter $R^6$ ein Alkylrest mit bis zu 10 Kohlenstoffatomen zu verstehen ist, |
| $R^7$ | Wasserstoff, $C_1$-$C_7$-Alkyl; Hydroxymethyl, 1-Hydroxyethyl, Mercaptomethyl, 2-(Methylthio)-ethyl, 3-Aminopropyl, 3-Ureidopropyl, 3-Guanidylpropyl, 4-Amino- butyl, Carboxymethyl, Carbamoylmethyl,2-Carboxyethyl, 2-Carbamoylethyl, Benzyl, 4-Hydroxybenzyl, 3-Indolylmethyl oder 4-Imidazolylmethyl bedeutet, und |
| $R^8$ | für Wasserstoff oder Methyl steht und |
| $R^9$ | für Wasserstoff, Acetyl, Benzoyl, Trichloracetyl, Trifluoracetyl, Methoxycarbonyl, t-Butyloxycarbonyl oder Benzyloxycarbonyl steht und |
| $R^7$ und $R^8$ | zusammen $-CH_2-CH_2-CH_2-$ bedeuten können, |

dadurch gekennzeichnet, daß man ein 2-Amino-2-desoxy-glycopyranosederivat der Formel (II),

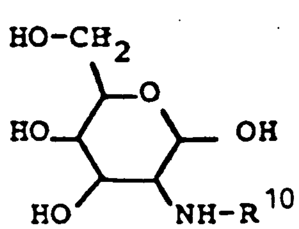

(II)

in dem

| | |
|---|---|
| $R^{10}$ | eine für den Schutz von Aminogruppen aus der Synthese von Peptiden her bekannte und gegebenenfalls selektiv abspaltbare Schutzgruppe darstellt, |

in einem ersten Reaktionsschritt mit Aminen der Formel (III),

$H_2N-R^1$ (III)

wobei
$R^1$ einen gesättigten oder einfach oder mehrfach ungesättigten Alkylrest mit 1 bis 50 C-Atomen bedeutet,
zu Glycosylaminen der Formel (IV)

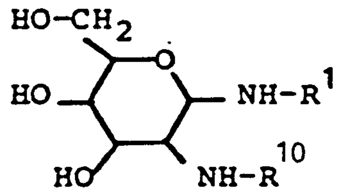

(IV)

umgesetzt und anschließend die Glycosylamine der Formel (IV)
a) mit geeigneten Carbonsäurederivaten der Formel (V)

$R^{11}-CO-CH_2-R^2$, (V)

wobei
$R^2$ Wasserstoff oder einen gesättigten oder einfach oder mehrfach ungesättigten Alkylrest mit 1 bis 50 C-Atomen bedeutet, und
$R^{11}$ für Halogen oder für $-O-CO-R^2$ oder für $-O-CO-O-(C_1-C_5)$-Alkyl steht,
oder
b) mit einem Halogenameisensäurester (VII)

$R^{12}-CO-O-R^2$ (VII)

wobei
$R^{12}$ für Halogen steht und
$R^2$ die oben angegebene Bedeutung hat,
oder
c) mit einem Isocyanat (VIII)

$R^2$ - NCO (VIII)

in dem
$R^2$ die oben angegebene Bedeutung hat,
umsetzt,

die erhaltenen Glycosylamide VI (X = $-CH_2-$), Glycosylcarbamate VI (X = -O-) oder Glycosylharnstoffe VI (X = -NH-) entweder chromatografisch reinigt oder an den Hydroxylgruppen der Zuckerreste verestert, kristallisiert und anschließend verseift,

aus den so erhaltenen Verbindungen der Formel (VI)

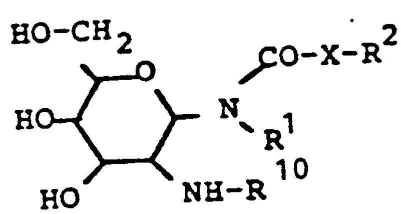

(VI)

mit den oben angegebenen Bedeutungen für $R^1$, $R^2$, X und $R^{10}$ die Schutzgruppe $-R^{10}$ abspaltet, um Verbindungen der Formel (X)

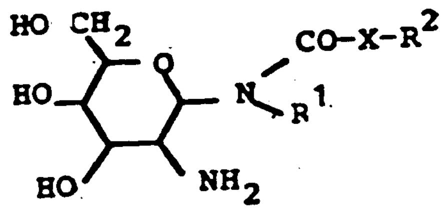

(X)

zu erhalten,

die Verbindungen der Formel (X) mit Aminosäurederivaten der Formel (XI)

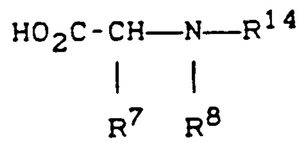

(XI)

in der

- $R^7$ die oben angegebene Bedeutung hat,
- $R^8$ für Wasserstoff oder Alkyl steht und
- $R^{14}$ eine in der Peptidsynthese gewöhnlich verwendete, selektiv unter Erhalt der Peptidbindung wieder abspaltbare Schutzgruppe darstellt,

umsetzt zu Verbindungen der Formel (XII),

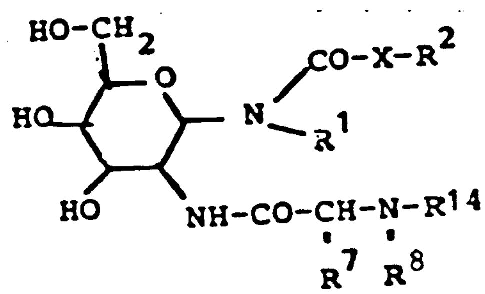

(XII)

die erhaltene Verbindung der Formel (XII) chromatographisch reinigt und dann unter Abspaltung von $-R^{14}$ unter Erhalt der Amid-, Urethan- oder Harnstoffunktion zu den Verbindungen der Formel (I) umsetzt oder zunächst an den Zuckerhydroxylgruppen verestert, kristallisiert, abseift und dann den Rest $-R^{14}$ unter Erhalt der Amid-, Urethan- oder Harnstoffunktion zur Herstellung der Verbindungen der Formel (I) abspaltet.

**7.** Verfahren zur Herstellung von Verbindungen der allgemeinen Formel (I) gemäß Anspruch 6, dadurch gekennzeichnet, daß eine Verbindung der Formel (XIII)

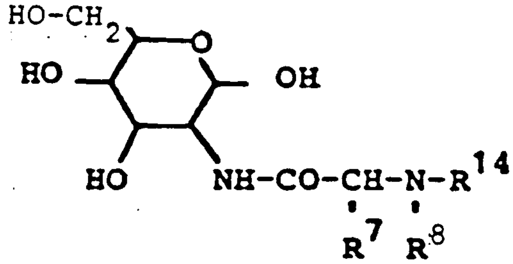

(XIII)

in der $R^7$, $R^8$ und $R^{14}$ die in Anspruch 6 angegebene Bedeutung haben, mit Verbindungen der Formel (III)

$H_2N - R^1$ (III)

in der

$R^1$ einen einfach oder mehrfach gesättigten oder ungesättigten Alkylrest mit bis zu 50 C-Atomen steht,

umsetzt zu Verbindungen der Formel (XIV)

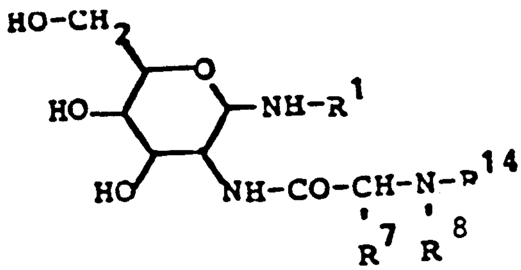

(XIV)

worin $R^1$, $R^7$, $R^8$ und $R^{14}$ die oben bzw. im Anspruch 6 angegebene Bedeutung haben, die Verbindungen der Formel (XIV)

a) mit geeigneten Carbonsäurederivaten der Formel (V)

$R^{11}$-CO-$CH_2$-$R^2$, (V)

wobei

$R^2$ Wasserstoff oder einen gesättigten oder einfach oder mehrfach ungesättigten Alkylrest mit 1 bis 50 C-Atomen bedeutet, und

$R^{11}$ für Halogen oder für -O-CO-$R^2$ oder für -O-CO-O-($C_1$-$C_5$)-Alkyl steht,

oder

b) mit einem Halogenameisensäurestern (VII)

$R^{12}$-CO-O-$R^2$ (VII

wobei

$R^{12}$ für Halogen steht und

$R^2$ die oben angegebene Bedeutung hat,

oder

c) mit einem Isocyanat (VIII)

$R^2$ - NCO (VIII)

in dem

$R^2$ die oben angegebene Bedeutung hat,

umsetzt und die so erhaltenen Verbindungen der Formel (XII)

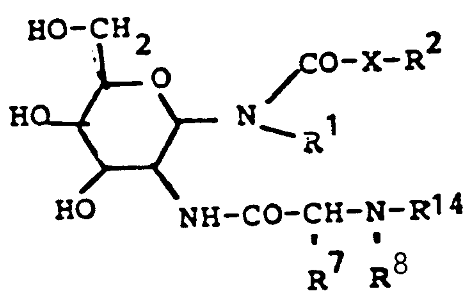

(XII)

worin $R^1$, $R^2$, X, $R^7$, $R^8$ und $R^{14}$ die oben bzw. im Anspruch 6 angegebene Bedeutung haben,

wie in Anspruch 6 angegeben zu den Verbindungen der Formel (I) umsetzt.

**8.** Verbindungen der allgemeinen Formel I

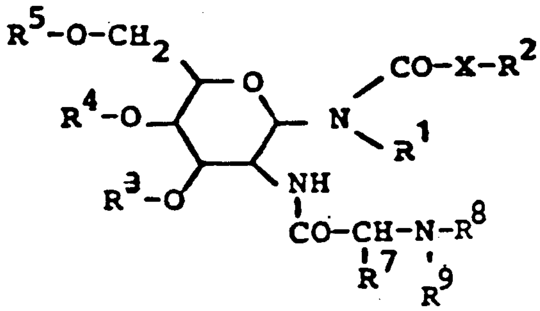

(I)

in der

| | |
|---|---|
| $R^1$ | einen gesättigten oder einfach oder mehrfach ungesättigten Alkylrest mit bis zu 50 Kohlenstoffatomen bedeutet, |
| X | für $-CH_2$, -O- oder -NH- steht, |
| $R^2$ | Wasserstoff oder einen gesättigten oder einfach oder mehrfach ungesättigten Alkylrest mit bis zu 50 Kohlenstoffatomen bedeutet, |
| $R^3$, $R^4$ und $R^5$ | unabhängig voneinander Wasserstoff oder Acyl $-CO-R^6$ bedeutet, wobei unter $R^6$ ein Alkylrest mit bis zu 10 Kohlenstoffatomen zu verstehen ist, |
| $R^7$ | Wasserstoff, $C_1$-$C_7$-Alkyl; Hydroxymethyl, 1-Hydroxyethyl, Mercaptomethyl, 2-(Methylthio)-ethyl, 3-Aminopropyl, 3-Ureidopropyl, 3-Guanidylpropyl, 4-Aminobutyl, Carboxymethyl, Carbamoylmethyl, 2-Carboxyethyl, 2-Carbamoylethyl, Benzyl, 4-Hydroxybenzyl, 3-Indolylmethyl oder 4-Imidazolylmethyl bedeutet, und |
| $R^8$ | für Wasserstoff oder Methyl steht und |
| $R^9$ | für Wasserstoff, Acetyl, Benzoyl, Trichloracetyl, Trifluoracetyl, Methoxycarbonyl, t-Butyloxycarbonyl oder Benzyloxycarbonyl steht und |
| $R^7$ und $R^8$ | zusammen $-CH_2-CH_2-CH_2-$ bedeuten können, |

zur prophylaktischen und therapeutischen Behandlung des menschlichen oder tierischen Körpers.

**9.** Verbindungen der allgemeinen Formel I

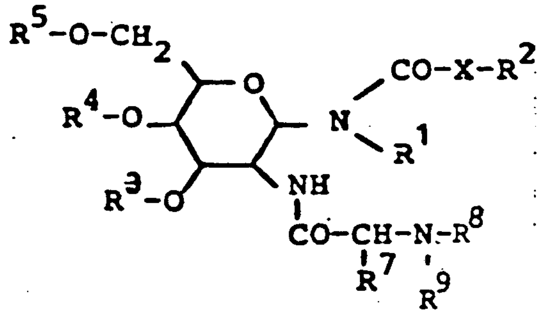

(I)

in der

| | |
|---|---|
| $R^1$ | einen gesättigten oder einfach oder mehrfach ungesättigten Alkylrest mit bis zu 50 Kohlenstoffatomen bedeutet, |
| X | für $-CH_2-$, -O- oder -NH- steht, |
| $R^2$ | Wasserstoff oder einen gesättigten oder einfach oder mehrfach ungesättigten Alkylrest mit bis zu 50 Kohlenstoffatomen bedeutet, |
| $R^3$, $R^4$ und $R^5$ | unabhängig voneinander Wasserstoff oder Acyl $-CO-R^6$ bedeutet, wobei unter $R^6$ ein Alkylrest mit bis zu 10 Kohlenstoffatomen zu verstehen ist, |
| $R^7$ | Wasserstoff, $C_1$-$C_7$-Alkyl; Hydroxymethyl, 1-Hydroxyethyl, Mercaptomethyl, 2- |

(Methylthio)-ethyl, 3-Aminopropyl, 3-Ureidopropyl, 3-Guanidylpropyl, 4-Aminobutyl, Carboxymethyl, Carbamoylmethyl,2-Carboxyethyl, 2-Carbamoylethyl, Benzyl, 4-Hydroxybenzyl, 3-Indolylmethyl oder 4-Imidazolylmethyl bedeutet, und

$R^8$ für Wasserstoff oder Methyl steht und

$R^9$ für Wasserstoff, Acetyl, Benzoyl, Trichloracetyl, Trifluoracetyl, Methoxycarbonyl, t-Butyloxycarbonyl oder Benzyloxycarbonyl steht und

$R^7$ und $R^8$ zusammen $-CH_2-CH_2-CH_2-$ bedeuten können,

in der L- oder D-Form oder in Form ihrer, gegebenenfalls inneren, pharmazeutisch verwendbaren Salze.

**10.** Verbindungen der Formel XII

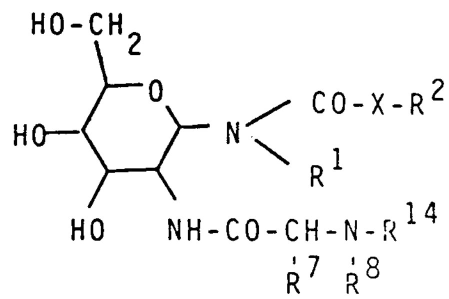

(XII)

in denen $R^1$, $R^2$, $R^7$ und X die in Anspruch 1 angegebene Bedeutung haben,

$R^8$ für Wasserstoff oder Methyl steht und

$R^{14}$ eine in die Peptidsynthese gewöhnlich verwendete selektiv unter Erhalt der Peptidbindung wieder abspaltbare Schutzgruppe darstellt.

**11.** Verbindungen der Formel XV

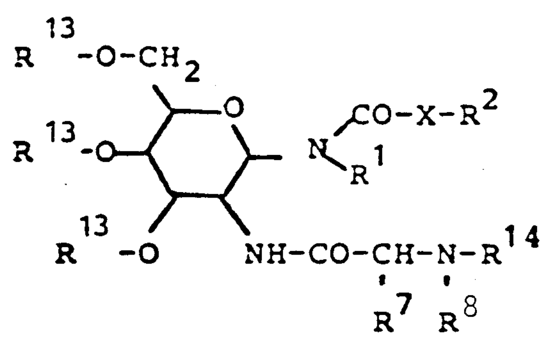

(XV)

in denen

$R^1$, $R^2$, $R^7$, $R^8$, $R^{13}$, $R^{14}$ und X die in Anspruch 10 angegebene Bedeutung haben.

**12.** Arzneimittel enthaltend Verbindungen der allgemeinen Formel I

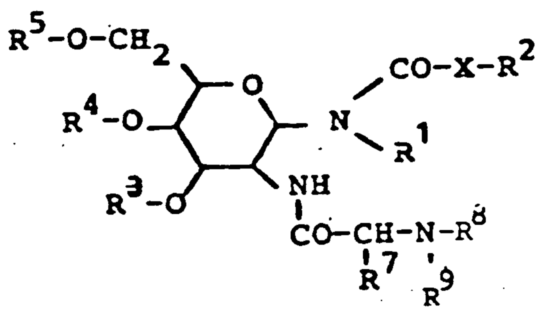

(I)

in der

| | |
|---|---|
| $R^1$ | einen gesättigten oder einfach oder mehrfach ungesättigten Alkylrest mit bis zu 50 Kohlenstoffatomen bedeutet, |
| X | für $-CH_2-$, -O- oder -NH- steht, |
| $R^2$ | Wasserstoff oder einen gesättigten oder einfach oder mehrfach ungesättigten Alkylrest mit bis zu 50 Kohlenstoffatomen bedeutet, |
| $R^3$, $R^4$ und $R^5$ | unabhängig voneinander Wasserstoff oder Acyl $-CO-R^6$ bedeutet, wobei unter $R^6$ ein Alkylrest mit bis zu 10 Kohlenstoffatomen zu verstehen ist, |
| $R^7$ | Wasserstoff, $C_1$-$C_7$-Alkyl; Hydroxymethyl, 1-Hydroxyethyl, Mercaptomethyl, 2-(Methylthio)-ethyl, 3-Aminopropyl, 3-Ureidopropyl, 3-Guanidylpropyl, 4-Amino- butyl, Carboxymethyl, Carbamoylmethyl, 2-Carboxyethyl, 2-Carbamoylethyl, Benzyl, 4-Hydroxybenzyl, 3-Indolylmethyl oder 4-Imidazolylmethyl bedeutet, und |
| $R^8$ | für Wasserstoff oder Methyl steht und |
| $R^9$ | für Wasserstoff, Acetyl, Benzoyl, Trichloracetyl, Trifluoracetyl, Methoxycarbonyl, t-Butyloxycarbonyl oder Benzyloxycarbonyl steht und |
| $R^7$ und $R^8$ | zusammen $-CH_2-CH_2-CH_2-$ bedeuten können. |

**13.** Verwendung von Verbindungen der allgemeinen Formel (I)

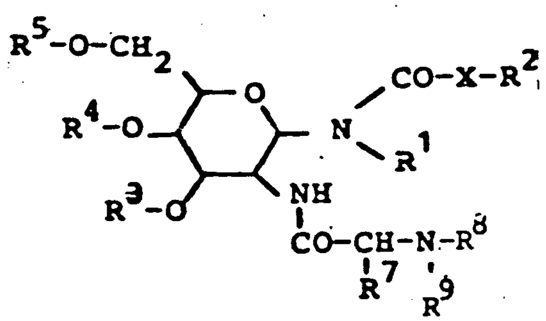

(I)

in der

| | |
|---|---|
| $R^1$ | einen gesättigten oder einfach oder mehrfach ungesättigten Alkylrest mit bis zu 50 Kohlenstoffatomen bedeutet, |
| X | für $-CH_2$, -O- oder -NH- steht, |
| $R^2$ | Wasserstoff oder einen gesättigten oder einfach oder mehrfach ungesättigten Alkylrest mit bis zu 50 Kohlenstoffatomen bedeutet, |
| $R^3$, $R^4$ und $R^5$ | unabhängig voneinander Wasserstoff oder Acyl $-CO-R^6$ bedeutet, wobei unter $R^6$ ein Alkylrest mit bis zu 10 Kohlenstoffatomen zu verstehen ist, |
| $R^7$ | Wasserstoff, $C_1$-$C_7$-Alkyl; Hydroxymethyl, 1-Hydroxyethyl, Mercaptomethyl, 2-(Methylthio)-ethyl, 3-Aminopropyl, 3-Ureidopropyl, 3-Guanidylpropyl, 4-Aminobutyl, Carboxymethyl, Carbamoylmethyl,2-Carboxyethyl, 2-Carbamoylethyl, Benzyl, 4-Hydroxybenzyl, 3-Indolylmethyl dodr 4-Imidazolylmethyl bedeutet, und |
| $R^8$ | für Wasserstoff oder Methyl steht und |
| $R^9$ | für Wasserstoff, Acetyl, Benzoyl, Trichloracetyl, Trifluoracetyl, Methoxycarbonyl, t-Butyloxycarbonyl oder Benzyloxycarbonyl steht und |
| $R^7$ und $R^8$ | zusammen $-CH_2-CH_2-CH_2-$ bedeuten können, |

zur Herstellung von Arzneimitteln.

**14.** Verwendung von Verbindungen der allgemeinen Formel I

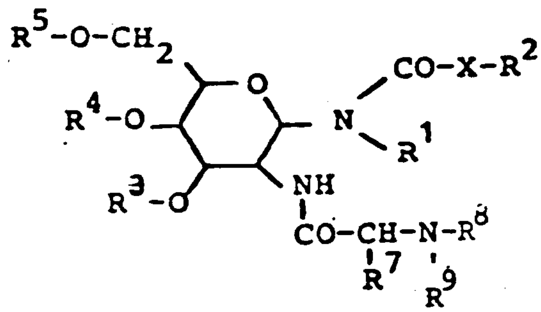

(I)

in der

| | |
|---|---|
| $R^1$ | einen gesättigten oder einfach oder mehrfach ungesättigten Alkylrest mit bis zu 50 Kohlenstoffatomen bedeutet, |
| X | für $-CH_2$, -O- oder -NH- steht, |
| $R^2$ | Wasserstoff oder einen gesättigten oder einfach oder mehrfach ungesättigten Alkylrest mit bis zu 50 Kohlenstoffatomen bedeutet, |
| $R^3$, $R^4$ und $R^5$ | unabhängig voneinander Wasserstoff oder Acyl $-CO-R^6$ bedeutet, wobei unter $R^6$ ein Alkylrest mit bis zu 10 Kohlenstoffatomen zu verstehen ist, |
| $R^7$ | Wasserstoff, $C_1$-$C_7$-Alkyl: Hydroxymethyl, 1-Hydroxyethyl, Mercaptomethyl, 2-(Methylthio)-ethyl, 3-Aminopropyl, 3-Ureidopropyl, 3-Guanidylpropyl, 4-Aminobutyl, Carboxymethyl, Carbamoylmethyl,2-Carboxyethyl, 2-Carbamoylethyl, Benzyl, 4-Hydroxybenzyl, 3-Indolylmethyl oder 4-Imidazolylmethyl bedeutet, und |
| $R^8$ | für Wasserstoff oder Methyl steht und |
| $R^9$ | für Wasserstoff, Acetyl, Benzoyl, Trichloracetyl, Trifluoracetyl, Methoxycarbonyl, t-Butyloxycarbonyl oder Benzyloxycarbonyl steht und |
| $R^7$ und $R^8$ | zusammen $-CH_2-CH_2-CH_2-$ bedeuten können, |

zur Herstellung von Arzneimitteln für die Bekämpfung von Krankheiten des menschlichen oder tierischen Körpers.

**15.** Verwendung der Verbindungen gemäß Anspruch 1 bei der Herstellung von Arzneimitteln für die Steigerung der körpereigenen Abwehr.

**16.** Verwendung von Verbindungen gemäß Anspruch 1 als Adjuvans mit Antigenen zur Herstellung von Antiseren für Diagnose und Therapie von Infektionskrankheiten.

**17.** Vaccinen enthaltend Verbindungen der allgemeinen Formel I

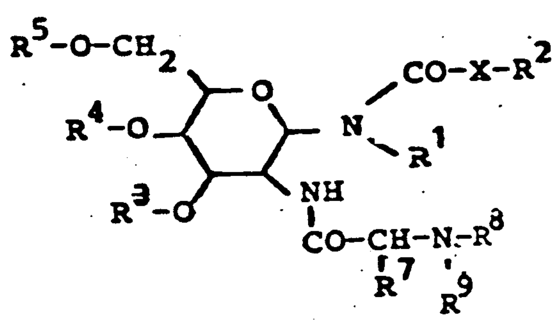

(I)

in der

| | |
|---|---|
| $R^1$ | einen gesättigten oder einfach oder mehrfach ungesättigten Alkylrest mit bis zu 50 Kohlenstoffatomen bedeutet, |
| X | für $-CH_2-$, -O- oder -NH- steht, |
| $R^2$ | Wasserstoff oder einen gesättigten oder einfach oder mehrfach ungesättigten Alkylrest mit bis zu 50 Kohlenstoffatomen bedeutet, |
| $R^3$, $R^4$ und $R^5$ | unabhängig voneinander Wasserstoff oder Acyl $-CO-R^6$ bedeutet, wobei unter $R^6$ ein Alkylrest mit bis zu 10 Kohlenstoffatomen zu verstehen ist, |

| | |
|---|---|
| $R^7$ | Wasserstoff, $C_1$-$C_7$-Alkyl; Hydroxymethyl, 1-Hydroxyethyl, Mercaptomethyl, 2-(Methylthio)-ethyl, 3-Aminopropyl, 3-Ureidopropyl, 3-Guanidylpropyl, 4-Aminobutyl, Carboxymethyl, Carbamoylmethyl,2-Carboxyethyl, 2-Carbamoylethyl, Benzyl, 4-Hydroxybenzyl, 3-Indolylmethyl oder 4-Imidazolylmethyl bedeutet, und |
| $R^8$ | für Wasserstoff oder Methyl steht und |
| $R^9$ | für Wasserstoff, Acetyl, Benzoyl, Trichloracetyl, Trifluoracetyl, Methoxycarbonyl, t-Butyloxycarbonyl oder Benzyloxycarbonyl steht und |
| $R^7$ und $R^8$ | zusammen $-CH_2-CH_2-CH_2-$ bedeuten können. |

**18.** Verwendung von Verbindungen der allgemeinen Formel I

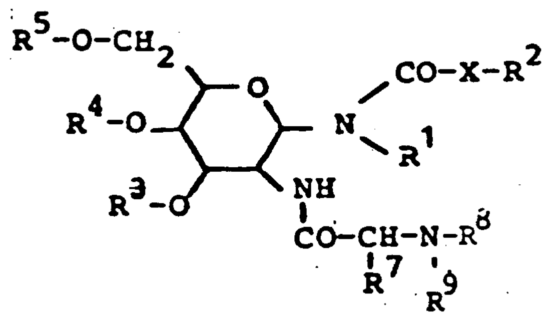

(I)

| | |
|---|---|
| $R^1$ | einen gesättigten oder einfach oder mehrfach ungesättigten Alkylrest mit bis zu 50 Kohlenstoffatomen bedeutet, |
| X | für $-CH_2$, -O- oder -NH- steht, |
| $R^2$ | Wasserstoff oder einen gesättigten oder einfach oder mehrfach ungesättigten Alkylrest mit bis zu 50 Kohlenstoffatomen bedeutet, |
| $R^3$, $R^4$ und $R^5$ | unabhängig voneinander Wasserstoff oder Acyl $-CO-R^6$ bedeutet, wobei unter $R^6$ ein Alkylrest mit bis zu 10 Kohlenstoffatomen zu verstehen ist, |
| $R^7$ | Wasserstoff, $C_1$-$C_7$-Alkyl; Hydroxymethyl, 1-Hydroxyethyl, Mercaptomethyl, 2-(Methylthio)-ethyl, 3-Aminopropyl, 3-Ureidopropyl, 3-Guanidylpropyl, 4-Aminobutyl, Carboxymethyl, Carbamoylmethyl,2-Carboxyethyl, 2-Carbamoylethyl, Benzyl, 4-Hydroxybenzyl, 3-Indolylmethyl oder 4-Imidazolylmethyl bedeutet, und |
| $R^8$ | für Wasserstoff oder Methyl steht und |
| $R^9$ | für Wasserstoff, Acetyl, Benzoyl, Trichloracetyl, Trifluoracetyl, Methoxycarbonyl, t-Butyloxycarbonyl oder Benzyloxycarbonyl steht und |
| $R^7$ und $R^8$ | zusammen $-CH_2-CH_2-CH_2-$ bedeuten können, als Adjuvantien bei der Herstellung von Virusvaccinen gegen Infektionen mit Influenza-, Mumps-, Masern-, Röteln-, Hepatitis-, Herpes- u.a. menschlichen Viren. |

**19.** Verwendung von Verbindungen der allgemeinen Formel I

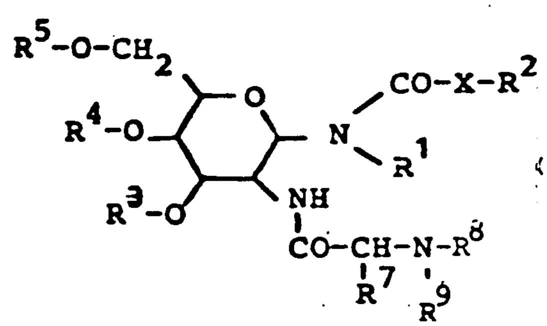

(I)

in der

| | |
|---|---|
| $R^1$ | einen gesättigten oder einfach oder mehrfach ungesättigten Alkylrest mit bis zu 50 Kohlenstoffatomen bedeutet, |
| X | für $-CH_2$, -O- oder -NH- steht, |

| | |
|---|---|
| $R^2$ | Wasserstoff oder einen gesättigten oder einfach oder mehrfach ungesättigten Alkylrest mit bis zu 50 Kohlenstoffatomen bedeutet, |
| $R^3$, $R^4$ und $R^5$ | unabhängig voneinander Wasserstoff oder Acyl -CO-$R^6$ bedeutet, wobei unter $R^6$ ein Alkylrest mit bis zu 10 Kohlenstoffatomen zu verstehen ist, |
| $R^7$ | Wasserstoff, $C_1$-$C_7$-Alkyl; Hydroxymethyl, 1-Hydroxyethyl, Mercaptomethyl, 2-(Methylthio)-ethyl, 3-Aminopropyl, 3-Ureidopropyl, 3-Guanidylpropyl, 4-Aminobutyl, Carboxymethyl, Carbamoylmethyl, 2-Carboxyethyl, 2-Carbamoylethyl, Benzyl, 4-Hydroxybenzyl, 3-Indolylmethyl oder 4-Imidazolylmethyl bedeutet, und |
| $R^8$ | für Wasserstoff oder Methyl steht und |
| $R^9$ | für Wasserstoff, Acetyl, Benzoyl, Trichloracetyl, Trifluoracetyl, Methoxycarbonyl, t-Butyloxycarbonyl oder Benzyloxycarbonyl steht und |
| $R^7$ und $R^8$ | zusammen $-CH_2-CH_2-CH_2-$ bedeuten können, |

als Adjuvantien bei der Herstellung von Virusvaccinen gegen Infektionen mit Pseudorabies-, Rhinopneumonitis-, Marek-, Maul- und Klauenseuche-, Rindergrippe u.a. tierische Viren.

## Claims

1. Compounds of the general formula I

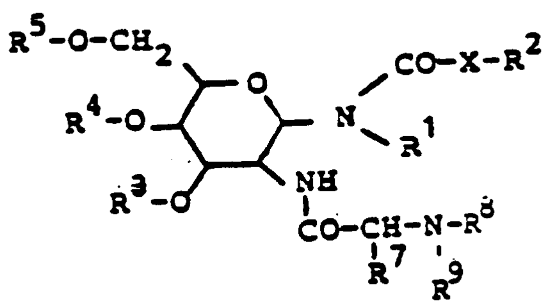

(I)

in which

| | |
|---|---|
| $R^1$ | denotes a saturated or singly or multiply unsaturated alkyl radical having up to 50 carbon atoms, |
| X | represents $-CH_2-$, -O- or -NH-, |
| $R^2$ | denotes hydrogen or a saturated or singly or multiply unsaturated alkyl radical having up to 50 carbon atoms, |
| $R^3$, $R^4$ and $R^5$, | independently of one another, denote hydrogen or acyl-CO-$R^6$, $R^6$ being understood to be an alkyl radical having up to 10 carbon atoms, |
| $R^7$ | denotes hydrogen, $C_1$-$C_7$-alkyl, hydroxymethyl, 1-hydroxyethyl, mercaptomethyl, 2-(methylthio)-ethyl, 3-aminopropyl, 3-ureidopropyl, 3-guanidylpropyl, 4-aminobutyl, carboxymethyl, carbamoylmethyl, 2-carboxyethyl, 2-carbamoylethyl, benzyl, 4-hydroxybenzyl, 3-indolylmethyl or 4-imidazolylmethyl, and |
| $R^8$ | represents hydrogen or methyl, and |
| $R^9$ | represents hydrogen, acetyl, benzoyl, trichloroacetyl, trifluoroacetyl, methoxycarbonyl, t-butyloxycarbonyl or benzyloxycarbonyl and |
| $R^7$ and $R^8$ | together can denote $-CH_2-CH_2-CH_2-$. |

2. Compounds according to Claim 1, in which $R^1$ represents a straight-chain or branched, saturated or unsaturated alkyl radical having 1 to 20 C atoms.

3. Compounds according to Claims 1 and 2, in which $R^2$ represents hydrogen or represents a straight-chain or branched, saturated or unsaturated alkyl radical having 1 to 20 C atoms.

4. Compounds according to Claims 1 to 3, in which the sugar residues are 2-amino-2-deoxy-D-glucose or 2-amino-2-deoxy-D-galactose.

5. Compounds according to Claims 1 to 4, in which the 2-amino group of the aminosugar is bonded to

glycine, sarcosine, hippuric acid, alanine, valine, leucine, isoleucine, serine, threonine, cysteine, methionine, ornithine, citrulline, arginine, aspartic acid, asparagine, glutamic acid, glutamine, phenylalanine, tyrosine, proline, tryptophan or histidine, in the D- or L-form, or to amino carboxylic acids such as α-aminobutyric acid, α-aminovaleric acid, α-aminocaproic acid or α-aminoheptanoic acid, in the D- or L-form.

**6.** Process for the preparation of compounds of the general formula (I)

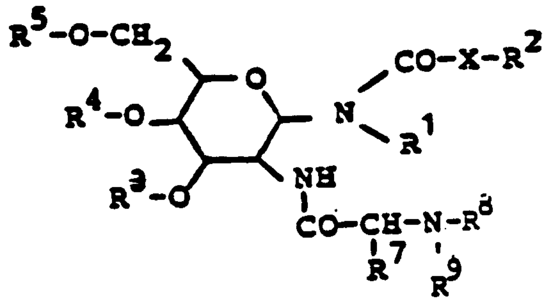

(I)

in which

| | |
|---|---|
| $R^1$ | denotes a saturated or singly or multiply unsaturated alkyl radical having up to 50 carbon atoms, |
| X | represents $-CH_2-$, -O- or -NH-, |
| $R^2$ | denotes hydrogen or a saturated or singly or multiply unsaturated alkyl radical having up to 50 carbon atoms, |
| $R^3$, $R^4$ and $R^5$, | independently of one another, denote hydrogen or acyl-CO-$R^6$, $R^6$ being understood to be an alkyl radical having up to 10 carbon atoms, |
| $R^7$ | denotes hydrogen, $C_1$-$C_7$-alkyl, hydroxymethyl, 1-hydroxyethyl, mercaptomethyl, 2-(methylthio)-ethyl, 3-aminopropyl, 3-ureidopropyl, 3-guanidylpropyl, 4-aminobutyl, carboxymethyl, carbamoylmethyl, 2-carboxyethyl, 2-carbamoylethyl, benzyl, 4-hydroxybenzyl, 3-indolylmethyl or 4-imidazolylmethyl, and |
| $R^8$ | represents hydrogen or methyl, and |
| $R^9$ | represents hydrogen, acetyl, benzoyl, trichloroacetyl, trifluoroacetyl, methoxycarbonyl, t-butyloxycarbonyl or benzyloxycarbonyl and |
| $R^7$ and $R^8$ | together can denote $-CH_2-CH_2-CH_2-$, |

characterised in that a 2-amino-2-deoxy-glycopyranose derivative of the formula (II),

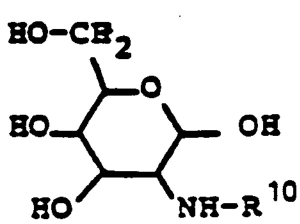

(II)

in which

$R^{10}$ represents a protective group for the protection of amino groups, which is known from the synthesis of peptides and can, where appropriate, be selectively eliminated,

is reacted, in a first reaction step, with amines of the formula (III)

$H_2N-R^1$ (III)

$R^1$ denoting a saturated or singly or multiply unsaturated alkyl radical having 1 to 50 C atoms,

to give glycosylamines of the formula (IV)

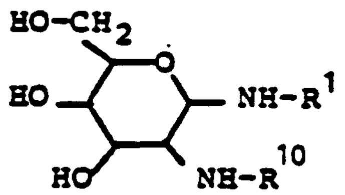

(IV)

and then the glycosylamines of the formula (IV) are reacted

a) with suitable carboxylic acid derivatives of the formula (V)

$R^{11}$-CO-$CH_2$-$R^2$ (V)

- $R^2$ denoting hydrogen or a saturated or singly or multiply unsaturated alkyl radical having 1 to 50 C atoms, and
- $R^{11}$ represents halogen or represents -O-CO-$R^2$ or represents -O-CO-O-($C_1$-$C_5$)-alkyl, or

b) with a haloformic ester (VII)

$R^{12}$-CO-O-$R^2$ (VII)

- $R^{12}$ representing halogen, and
- $R^2$ having the abovementioned meaning, or

c) with an isocyanate (VIII)

$R^2$-NCO (VIII)

in which

$R^2$ has the abovementioned meaning, and the resulting glycosylamides VI (X = -$CH_2$-), glycosyl carbamates VI (X = -O-) or glycosylureas VI (X = -NH-) are either purified by chromatography or esterified on the hydroxyl groups of the sugar residues, crystallised and then hydrolysed, and from the compounds of the formula (VI) thus obtained

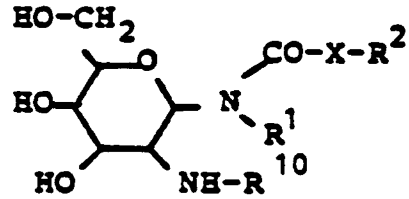

(VI)

with the abovementioned meanings for $R^1$, $R^2$, X and $R^{10}$, the protective group -$R^{10}$ is eliminated in order to obtain compounds of the formula (X)

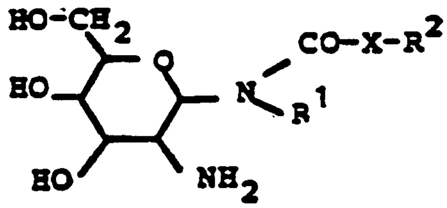

(X)

and the compounds of the formula (X) are reacted with amino acid derivatives of the formula (XI)

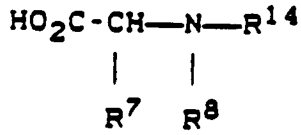

(XI)

in which

$R^7$ has the abovementioned meaning,

$R^8$ represents hydrogen or alkyl, and

$R^{14}$ represents a protective group which is customarily used in peptide synthesis and can be eliminated again selectively while retaining the peptide bond,

to give compounds of the formula (XII),

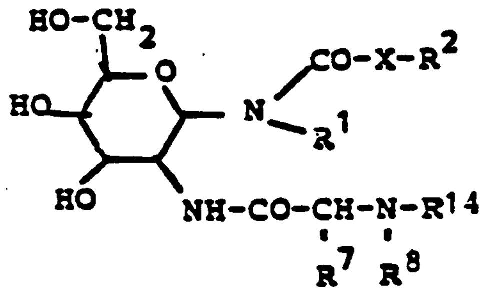

(XII)

and the resulting compound of the formula (XII) is purified by chromatography and then converted into the compounds of the formula (I) with elimination of $-R^{14}$ while retaining the amide, urethane or urea group, or is initially esterified on the sugar hydroxyl groups, and crystallised, hydrolysed and then the radical $-R^{14}$ is eliminated, while retaining the amide, urethane or urea group, to prepare the compounds of the formula (I).

**7.** Process for the preparation of compounds of the general formula (I) according to Claim 6, characterised in that a compound of the formula (XIII)

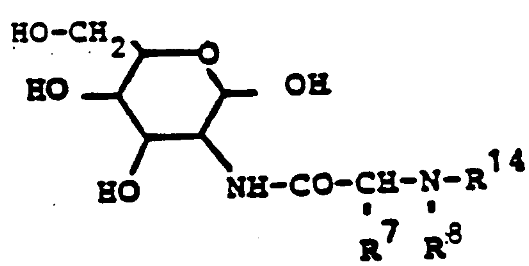

(XIII)

in which $R^7$, $R^8$ and $R^{14}$ have the meaning indicated in Claim 6, is reacted with compounds of the formula (III)

$H_2N - R^1$ (III)

in which

$R^1$ represents a saturated or singly or multiply unsaturated alkyl radical having up to 50 C atoms,

to give compounds of the formula (XIV)

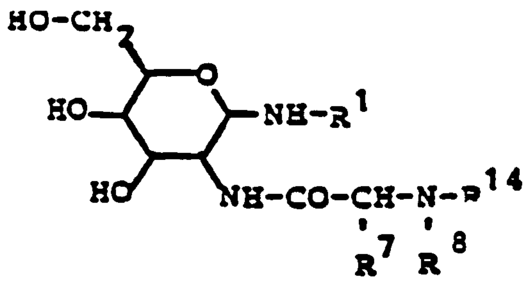

(XIV)

in which $R^1$, $R^7$, $R^8$ and $R^{14}$ have the meaning indicated above or in Claim 6, and the compounds of the formula (XIV) are reacted

a) with suitable carboxylic acid derivatives of the formula (V)

$R^{11}$-CO-$CH_2$-$R^2$ (V)

$R^2$ denoting hydrogen or a saturated or singly or multiply unsaturated alkyl radical having 1 to 50 C atoms, and

$R^{11}$ represents halogen or represents -O-CO-$R^2$ or represents -O-CO-O-($C_1$-$C_5$)-alkyl, or

b) with a haloformic ester (VII)

$R^{12}$-CO-O-$R^2$ (VII)

$R^{12}$ representing halogen, and

$R^2$ having the abovementioned meaning, or

c) with an isocyanate (VIII)

$R^2$-NCO (VIII)

in which

$R^2$ has the abovementioned meaning,

and the compounds of the formula (XII) thus obtained

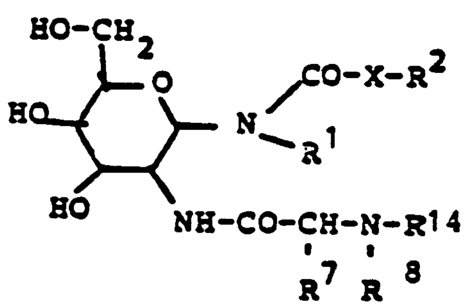

(XII)

in which

$R^1$, $R^2$, X, $R^7$, $R^8$ and $R^{14}$ have the meaning indicated above or in Claim 6, are reacted as indicated in Claim 6 to give the compounds of the formula (I).

**8.** Compounds of the general formula I

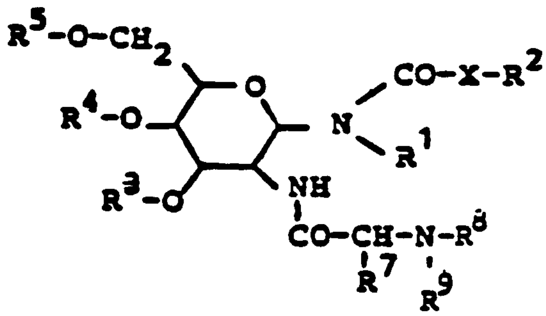

(I)

in which

| | |
|---|---|
| $R^1$ | denotes a saturated or singly or multiply unsaturated alkyl radical having up to 50 carbon atoms, |
| X | represents $-CH_2$, -O- or -NH-, |
| $R^2$ | denotes hydrogen or a saturated or singly or multiply unsaturated alkyl radical having up to 50 carbon atoms, |
| $R^3$, $R^4$ and $R^5$, | independently of one another, denote hydrogen or acyl-CO-$R^6$, $R^6$ being understood to be an alkyl radical having up to 10 carbon atoms, |
| $R^7$ | denotes hydrogen, $C_1$-$C_7$-alkyl, hydroxymethyl, 1-hydroxyethyl, mercaptomethyl, 2-(methylthio)-ethyl, 3-aminopropyl, 3-ureidopropyl, 3-guanidylpropyl, 4-aminobutyl, carboxymethyl, carbamoylmethyl, 2-carboxyethyl, 2-carbamoylethyl, benzyl, 4-hydroxybenzyl, 3-indolylmethyl or 4-imidazolylmethyl, and |
| $R^8$ | represents hydrogen or methyl, and |
| $R^9$ | represents hydrogen, acetyl, benzoyl, trichloroacetyl, trifluoroacetyl, methoxycarbonyl, t-butyloxycarbonyl or benzyloxycarbonyl and |
| $R^7$ and $R^8$ | together can denote $-CH_2-CH_2-CH_2-$, |

for the prophylactic and therapeutic treatment of the human or animal body.

**9.** Compounds of the general formula I

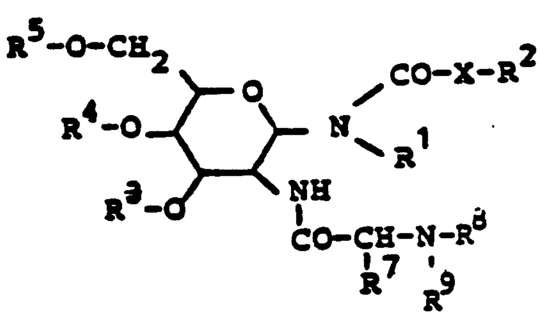

(I)

in which

| | |
|---|---|
| $R^1$ | denotes a saturated or singly or multiply unsaturated alkyl radical having up to 50 carbon atoms, |
| X | represents $-CH_2-$, -O- or -NH-, |
| $R^2$ | denotes hydrogen or a saturated or singly or multiply unsaturated alkyl radical having up to 50 carbon atoms, |
| $R^3$, $R^4$ and $R^5$, | independently of one another, denote hydrogen or acyl-CO-$R^6$, $R^6$ being understood to be an alkyl radical having up to 10 carbon atoms, |
| $R^7$ | denotes hydrogen, $C_1$-$C_7$-alkyl, hydroxymethyl, 1-hydroxyethyl, mercaptomethyl, 2-(methylthio)-ethyl, 3-aminopropyl, 3-ureidopropyl, 3-guanidylpropyl, 4-aminobutyl, carboxymethyl, carbamoylmethyl, 2-carboxyethyl, 2-carbamoylethyl, benzyl, 4-hydroxybenzyl, 3-indolylmethyl or 4-imidazolylmethyl, and |
| $R^8$ | represents hydrogen or methyl, and |
| $R^9$ | represents hydrogen, acetyl, benzoyl, trichloroacetyl, trifluoroacetyl, methoxycarbonyl, t-butyloxycarbonyl or benzyloxycarbonyl and |
| $R^7$ and $R^8$ | together can denote $-CH_2-CH_2-CH_2-$, |

in the L- or D-form or in the form of their, optionally internal, pharmaceutically utilisable salts.

**10.** Compounds of the formula XII

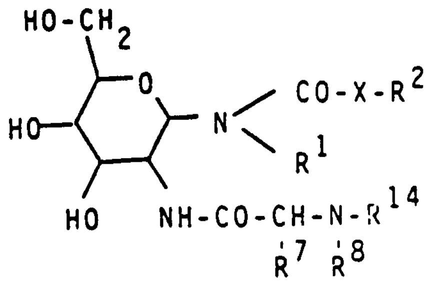

(XII)

in which $R^1$, $R^2$, $R^7$ and X have the meaning indicated in Claim 1,

$R^8$ represents hydrogen or methyl, and

$R^{14}$ represents a protective group which is customarily used in peptide synthesis and can be selectively eliminated again while retaining the peptide bond.

**11.** Compounds of the formula XV

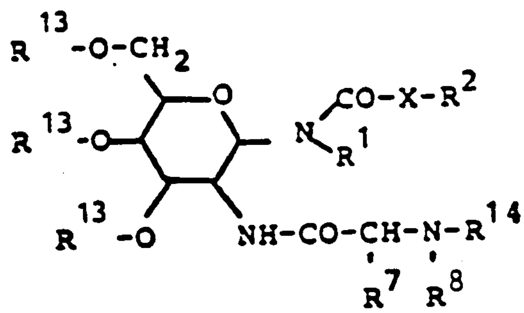

(XV)

in which

$R^1$, $R^2$, $R^7$, $R^8$, $R^{13}$, $R^{14}$ and X have the meaning indicated in Claim 10.

**12.** Medicament containing compounds of the general formula I

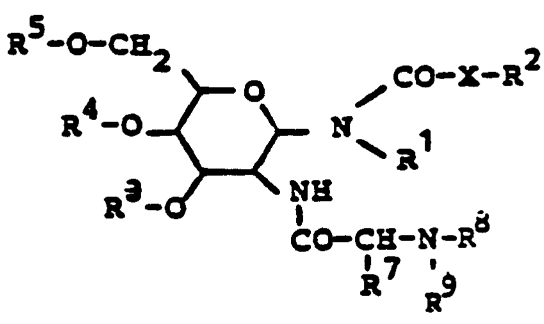

(I)

in which

$R^1$ denotes a saturated or singly or multiply unsaturated alkyl radical having up to 50 carbon atoms,

X represents $-CH_2-$, -O- or -NH-,

$R^2$ denotes hydrogen or a saturated or singly or multiply unsaturated alkyl radical having up to 50 carbon atoms,

$R^3$, $R^4$ and $R^5$, independently of one another, denote hydrogen or acyl-CO-$R^6$, $R^6$ being understood to be an alkyl radical having up to 10 carbon atoms,

| | |
|---|---|
| $R^7$ | denotes hydrogen, $C_1$-$C_7$-alkyl, hydroxymethyl, 1-hydroxyethyl, mercaptomethyl, 2-(methylthio)-ethyl, 3-aminopropyl, 3-ureidopropyl, 3-guanidylpropyl, 4-aminobutyl, carboxymethyl, carbamoylmethyl, 2-carboxyethyl, 2-carbamoylethyl, benzyl, 4-hydroxybenzyl, 3-indolylmethyl or 4-imidazolylmethyl, and |
| $R^8$ | represents hydrogen or methyl, and |
| $R^9$ | represents hydrogen, acetyl, benzoyl, trichloroacetyl, trifluoroacetyl, methoxycarbonyl, t-butyloxycarbonyl or benzyloxycarbonyl and |
| $R^7$ and $R^8$ | together can denote $-CH_2-CH_2-CH_2-$. |

**13.** Use of compounds of the general formula (I)

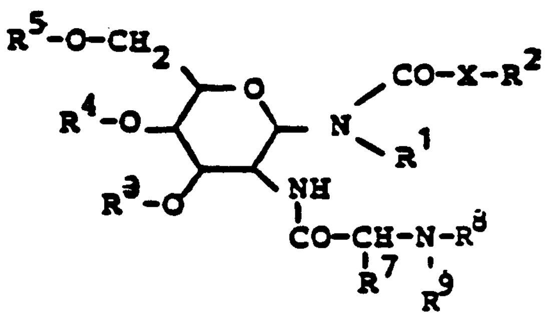

(I)

in which

| | |
|---|---|
| $R^1$ | denotes a saturated or singly or multiply unsaturated alkyl radical having up to 50 carbon atoms, |
| X | represents $-CH_2$, -O- or -NH-, |
| $R^2$ | denotes hydrogen or a saturated or singly or multiply unsaturated alkyl radical having up to 50 carbon atoms, |
| $R^3$, $R^4$ and $R^5$, | independently of one another, denote hydrogen or acyl-CO-$R^6$, $R^6$ being understood to be an alkyl radical having up to 10 carbon atoms, |
| $R^7$ | denotes hydrogen, $C_1$-$C_7$-alkyl, hydroxymethyl, 1-hydroxyethyl, mercaptomethyl, 2-(methylthio)-ethyl, 3-aminopropyl, 3-ureidopropyl, 3-guanidylpropyl, 4-aminobutyl, carboxymethyl, carbamoylmethyl, 2-carboxyethyl, 2-carbamoylethyl, benzyl, 4-hydroxybenzyl, 3-indolylmethyl or 4-imidazolylmethyl, and |
| $R^8$ | represents hydrogen or methyl, and |
| $R^9$ | represents hydrogen, acetyl, benzoyl, trichloroacetyl, trifluoroacetyl, methoxycarbonyl, t-butyloxycarbonyl or benzyloxycarbonyl and |
| $R^7$ and $R^8$ | together can denote $-CH_2-CH_2-CH_2-$, |

for the production of medicaments.

**14.** Use of compounds of the general formula (I)

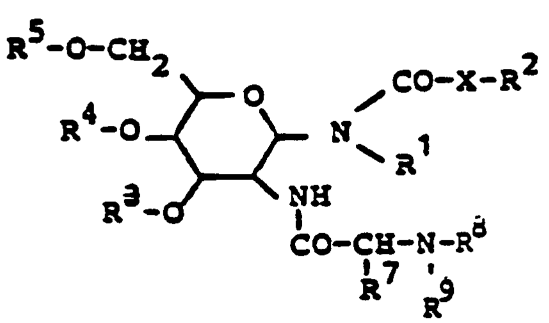

(I)

in which

| | |
|---|---|
| $R^1$ | denotes a saturated or singly or multiply unsaturated alkyl radical having up to 50 carbon atoms, |
| X | represents $-CH_2$, -O- or -NH-, |

| | |
|---|---|
| $R^2$ | denotes hydrogen or a saturated or singly or multiply unsaturated alkyl radical having up to 50 carbon atoms, |
| $R^3$, $R^4$ and $R^5$, | independently of one another, denote hydrogen or acyl-CO-$R^6$, $R^6$ being understood to be an alkyl radical having up to 10 carbon atoms, |
| $R^7$ | denotes hydrogen, $C_1$-$C_7$-alkyl, hydroxymethyl, 1-hydroxyethyl, mercaptomethyl, 2-(methylthio)-ethyl, 3-aminopropyl, 3-ureidopropyl, 3-guanidylpropyl, 4-aminobutyl, carboxymethyl, carbamoylmethyl, 2-carboxyethyl, 2-carbamoylethyl, benzyl, 4-hydroxybenzyl, 3-indolylmethyl or 4-imidazolylmethyl, and |
| $R^8$ | represents hydrogen or methyl, and |
| $R^9$ | represents hydrogen, acetyl, benzoyl, trichloroacetyl, trifluoroacetyl, methoxycarbonyl, t-butyloxycarbonyl or benzyloxycarbonyl and |
| $R^7$ and $R^8$ | together can denote $-CH_2-CH_2-CH_2-$, |

for the production of medicaments for controlling diseases of the human or animal body.

**15.** Use of the compounds according to Claim 1 in the production of medicaments for increasing the body's own defences.

**16.** Use of compounds according to Claim 1 as adjuvant with antigens for the production of antisera for diagnosis and treatment of infectious diseases.

**17.** Vaccines containing compounds of the general formula I

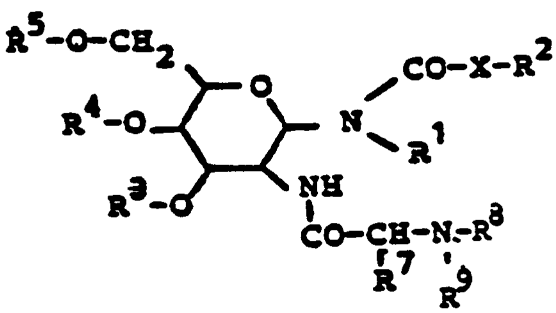

(I)

in which

| | |
|---|---|
| $R^1$ | denotes a saturated or singly or multiply unsaturated alkyl radical having up to 50 carbon atoms, |
| X | represents $-CH_2-$, -O- or -NH-, |
| $R^2$ | denotes hydrogen or a saturated or singly or multiply unsaturated alkyl radical having up to 50 carbon atoms, |
| $R^3$, $R^4$ and $R^5$, | independently of one another, denote hydrogen or acyl-CO-$R^6$, $R^6$ being understood to be an alkyl radical having up to 10 carbon atoms, |
| $R^7$ | denotes hydrogen, $C_1$-$C_7$-alkyl, hydroxymethyl, 1-hydroxyethyl, mercaptomethyl, 2-(methylthio)-ethyl, 3-aminopropyl, 3-ureidopropyl, 3-guanidylpropyl, 4-aminobutyl, carboxymethyl, carbamoylmethyl, 2-carboxyethyl, 2-carbamoylethyl, benzyl, 4-hydroxybenzyl, 3-indolylmethyl or 4-imidazolylmethyl, and |
| $R^8$ | represents hydrogen or methyl, and |
| $R^9$ | represents hydrogen, acetyl, benzoyl, trichloroacetyl, trifluoroacetyl, methoxycarbonyl, t-butyloxycarbonyl or benzyloxycarbonyl and |
| $R^7$ and $R^8$ | together can denote $-CH_2-CH_2-CH_2-$. |

**18.** Use of compounds of the general formula I

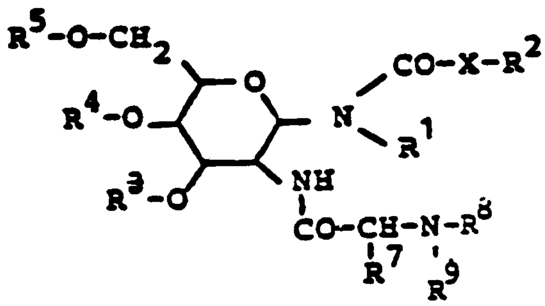

(I)

in which

| | |
|---|---|
| $R^1$ | denotes a saturated or singly or multiply unsaturated alkyl radical having up to 50 carbon atoms, |
| X | represents $-CH_2$, -O- or -NH-, |
| $R^2$ | denotes hydrogen or a saturated or singly or multiply unsaturated alkyl radical having up to 50 carbon atoms, |
| $R^3$, $R^4$ and $R^5$, | independently of one another, denote hydrogen or acyl-CO-$R^6$, $R^6$ being understood to be an alkyl radical having up to 10 carbon atoms, |
| $R^7$ | denotes hydrogen, $C_1$-$C_7$-alkyl, hydroxymethyl, 1-hydroxyethyl, mercaptomethyl, 2-(methylthio)-ethyl, 3-aminopropyl, 3-ureidopropyl, 3-guanidylpropyl, 4-aminobutyl, carboxymethyl, carbamoylmethyl, 2-carboxyethyl, 2-carbamoylethyl, benzyl, 4-hydroxybenzyl, 3-indolylmethyl or 4-imidazolylmethyl, and |
| $R^8$ | represents hydrogen or methyl, and |
| $R^9$ | represents hydrogen, acetyl, benzoyl, trichloroacetyl, trifluoroacetyl, methoxycarbonyl, t-butyloxycarbonyl or benzyloxycarbonyl and |
| $R^7$ and $R^8$ | together can denote $-CH_2-CH_2-CH_2-$, |

as adjuvants in the production of virus vaccines against infections with influenza, mumps, measles, rubella, hepatitis, herpes and other human viruses.

**19.** Use of compounds of the general formula I

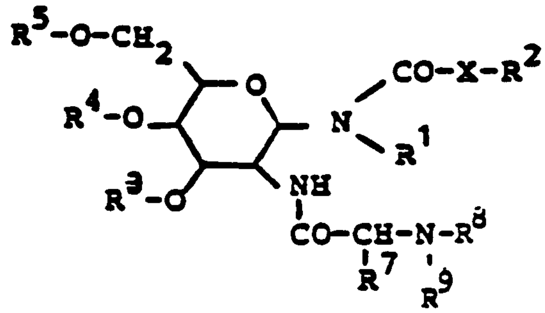

(I)

in which

| | |
|---|---|
| $R^1$ | denotes a saturated or singly or multiply unsaturated alkyl radical having up to 50 carbon atoms, |
| X | represents $-CH_2$, -O- or -NH-, |
| $R^2$ | denotes hydrogen or a saturated or singly or multiply unsaturated alkyl radical having up to 50 carbon atoms, |
| $R^3$, $R^4$ and $R^5$, | independently of one another, denote hydrogen or acyl-CO-$R^6$, $R^6$ being understood to be an alkyl radical having up to 10 carbon atoms, |
| $R^7$ | denotes hydrogen, $C_1$-$C_7$-alkyl, hydroxymethyl, 1-hydroxyethyl, mercaptomethyl, 2-(methylthio)-ethyl, 3-aminopropyl, 3-ureidopropyl, 3-guanidylpropyl, 4-aminobutyl, carboxymethyl, carbamoylmethyl, 2-carboxyethyl, 2-carbamoylethyl, benzyl, 4-hydroxybenzyl, 3-indolylmethyl or 4-imidazolylmethyl, and |
| $R^8$ | represents hydrogen or methyl, and |
| $R^9$ | represents hydrogen, acetyl, benzoyl, trichloroacetyl, trifluoroacetyl, methoxycar- |

bonyl, t-butyloxycarbonyl or benzyloxycarbonyl and

$R^7$ and $R^8$ together can denote $-CH_2-CH_2-CH_2-$,

as adjuvants in the production of virus vaccines against infections with pseudorabies, rhinopneumonitis, Marek, foot-and-mouth, bovine influenza and other animal viruses.

## Revendications

**1.** Composés de formule générale I

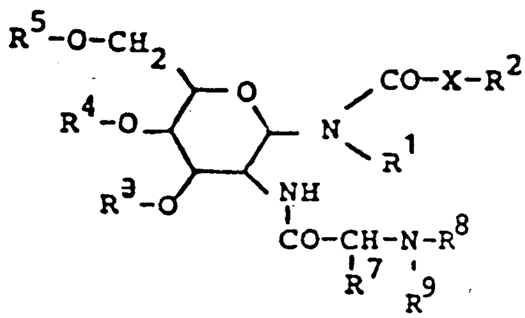

(I)

dans laquelle

| | |
|---|---|
| $R^1$ | représente un reste alkyle saturé ou insaturé une ou plusieurs fois et comportant 1 à 50 atomes de carbone, |
| X | représente $-CH_2-$, -O- ou -NH-, |
| $R^2$ | représente un atome d'hydrogène ou un reste alkyle saturé ou insaturé une ou plusieurs fois et comportant jusqu'à 50 atomes de carbone, |
| $R^3$, $R^4$ et $R^5$ | représentent chacun, indépendamment l'un de l'autre, un atome d'hydrogène ou un reste acyl-CO-$R^6$, dans lequel on entend désigner par $R^6$ un reste alkyle ayant jusqu'à 10 atomes de carbone, |
| $R^7$ | représente un atome d'hydrogène, un groupe alkyle en $C_1$-$C_7$, hydroxyméthyle, 1-hydroxyéthyle, mercaptométhyle, 2-(méthylthio)-éthyle, 3-aminopropyle, 3-uréidopropyle, 3-guanidylpropyle, 4-aminobutyle, carboxyméthyle, carbamoylméthyle, 2-carboxyéthyle, 2-carbamoyléthyle, benzyle, 4-hydroxybenzyle, 3-indolylméthyle ou 4-imidazolylméthyle, et |
| $R^8$ | représente un atome d'hydrogène ou un groupe méthyle, et |
| $R^9$ | représente un atome d'hydrogène, un groupe acétyle, benzoyle, trichloracétyle, trifluoracétyle, méthoxycarbonyle, t-butyloxycarbonyle ou benzyloxycarbonyle, et |
| $R^7$ et $R^8$ | peuvent, quand ils sont pris ensemble, représenter un groupe $-CH_2-CH_2-CH_2-$. |

**2.** Composés selon la revendication 1, dans lesquels $R^1$ représente un reste alkyle linéaire ou ramifié, saturé ou insaturé, comportant 1 à 20 atomes de carbone.

**3.** Composés selon les revendications 1 et 2, dans lesquels $R^2$ représente un atome d'hydrogène ou un reste alkyle linéaire ou ramifié, saturé ou insaturé, ayant 1 à 20 atomes de carbone.

**4.** Composés selon les revendications 1 à 3, dans lesquels les restes de sucre représentent un reste 2-amino-2-désoxy-D-glucose ou 2-amino-2-désoxy-D-galactose.

**5.** Composés selon les revendications 1 à 4, dans lesquels le groupe 2-amino de l'amino-sucre est lié à la glycine, la sarcosine, l'acide hippurique, l'alanine, la valine, la leucine, l'isoleucine, la sérine, la thréonine, la cystéine, la méthionine, l'ornithine, la citrulline, l'arginine, l'acide asparagique, l'asparagine, l'acide glutamique, la glutamine, la phénylalanine, la tyrosine, la proline, le tryptophanne ou l'histidine sous forme D ou L ou avec des acides aminocarboxyliques comme l'acide $\alpha$-aminobutyrique, l'acide $\alpha$-aminovalérianique, l'acide $\alpha$-aminocaproïque ou l'acide $\alpha$-aminoheptanoïque sous forme D ou sous forme L.

**6.** Procédé pour préparer des composés de formule générale (I)

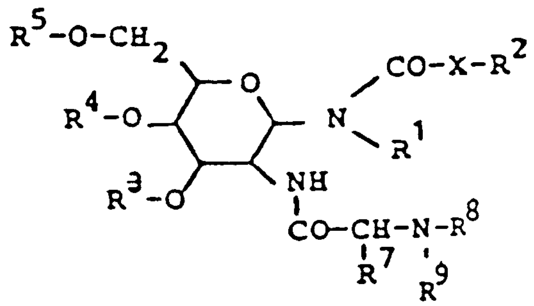

(I)

dans laquelle

$R^1$ représente un reste alkyle saturé ou insaturé une ou plusieurs fois et comportant 1 à 50 atomes de carbone,

X représente $-CH_2-$, -O- ou -NH-,

$R^2$ représente un atome d'hydrogène ou un reste alkyle saturé ou insaturé une ou plusieurs fois et comportant jusqu'à 50 atomes de carbone,

$R^3$, $R^4$ et $R^5$ représentent chacun, indépendamment l'un de l'autre, un atome d'hydrogène ou un reste acyl-CO-$R^6$, dans lequel on entend désigner par $R^6$ un reste alkyle ayant jusqu'à 10 atomes de carbone,

$R^7$ représente un atome d'hydrogène, un groupe alkyle en $C_1$-$C_7$, hydroxyméthyle, 1-hydroxyéthyle, mercaptométhyle, 2-(méthylthio)-éthyle, 3-aminopropyle, 3-uréidopropyle, 3-guanidylpropyle, 4-aminobutyle, carboxyméthyle, carbamoylméthyle, 2-carboxyéthyle, 2-carbamoyléthyle, benzyle, 4-hydroxybenzyle, 3-indolylméthyle ou 4-imidazolylméthyle, et

$R^8$ représente un atome d'hydrogène ou un groupe méthyle, et

$R^9$ représente un atome d'hydrogène, un groupe acétyle, benzoyle, trichloracétyle, trifluoracétyle, méthoxycarbonyle, t-butyloxycarbonyle ou benzyloxycarbonyle, et

$R^7$ et $R^8$ peuvent, quand ils sont pris ensemble, représenter un groupe $-CH_2-CH_2-CH_2-$,

procédé caractérisé en ce qu'on fait réagir un dérivé de 2-amino-2-désoxy-glycopyrannose de formule (II),

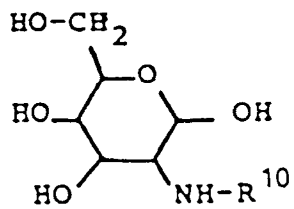

(II)

(dans laquelle

$R^1$ $^0$ représente un groupe protecteur de groupes amino, qui est connu dans la synthèse des peptides et qui peut éventuellement être sélectivement scindé),

dans une première étape de réaction, avec des amines de formule (III),

$H_2N-R^1$ (III)

(dans laquelle

$R^1$ représente un reste alkyle saturé ou insaturé une ou plusieurs fois, comportant 1 à 50 atomes de carbone),

pour obtenir des glycosylamines de formule (IV)

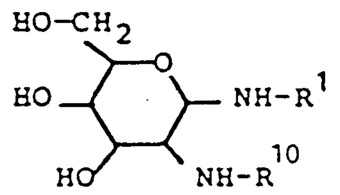

(IV)

et l'on fait ensuite réagir les glycosylamines de formule (IV)

a) avec des dérivés d'acides carboxyliques convenables, de formule (V)

$R^{11}$-CO-$CH_2$-$R^2$, (V)

dans laquelle

$R^2$ représente un atome d'hydrogène ou un reste alkyle saturé ou insaturé une ou plusieurs fois et comportant 1 à 50 atomes de carbone, et

$R^{11}$ représente un atome d'halogène ou un groupe -O-CO-$R^2$ ou -O-CO-O-alkyle (en $C_1$-$C_5$),

ou

b) avec un ester d'acide halogénoformique (VII)

$R^{12}$-CO-O-$R^2$ (VII)

dans laquelle

$R^{12}$ représente un atome d'halogène et

$R^2$ a le sens précité,

ou

c) avec un isocyanate (VIII)

$R^2$-NCO (VIII)

formule dans laquelle $R^2$ a le sens précité,

on purifie les glycosylamides VI (X = -$CH_2$-), les glycosylcarbamates VI (X = -O-) ou les glycosylurées VI (X = -NH-) ainsi obtenus en les soumettant à une chromatographie ou bien on les estérifie sur les groupes hydroxyles des restes sucres puis on les saponifie, on soumet les composés ainsi obtenus, de formule (VI)

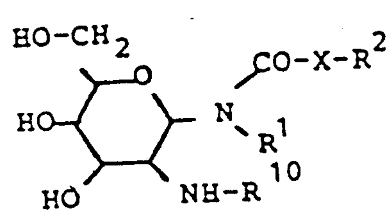

(VI)

(dans laquelle les symboles $R^1$, $R^2$, X et $R^{10}$ ont les sens indiqués ci-dessus) à un enlèvement du groupe protecteur -$R^{10}$, pour obtenir les composés de formule (X)

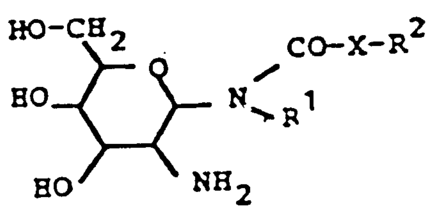

(X)

on fait réagir les composés de formule (X) avec des dérivés d'aminoacides de formule (XI)

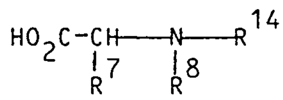

(XI)

(dans laquelle
$R^7$ a le sens indiqué ci-dessus,
$R^8$ représente un atome d'hydrogène ou un groupe alkyle, et
$R^{14}$ représente un groupe protecteur, utilisé couramment dans la synthèse des peptides et pouvant être à nouveau scindé sélectivement en donnant la liaison peptide),
pour obtenir les composés de formule (XII)

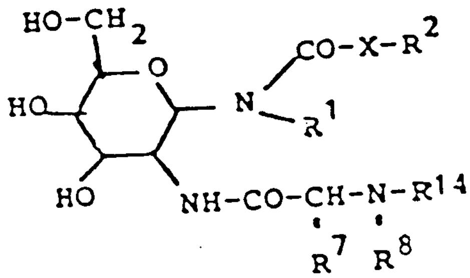

(XII)

on purifie par chromatographie le composé de formule (XII) ainsi obtenu, puis on fait réagir en scindant $-R^{14}$, et en obtenant ainsi la fonction amide, uréthanne ou urée, pour obtenir les composés de formule (I), ou bien on estérifie tout d'abord sur des groupes hydroxyles de sucre, on fait cristalliser, on saponifie puis l'on scinde le reste $-R^{14}$ en obtenant la fonction amide, uréthanne ou urée pour préparer les composés de formule (I).

**7.** Procédé pour préparer des composés de formule générale (I) selon la revendication 6, caractérisé en ce qu'on fait réagir un composé de formule (XIII)

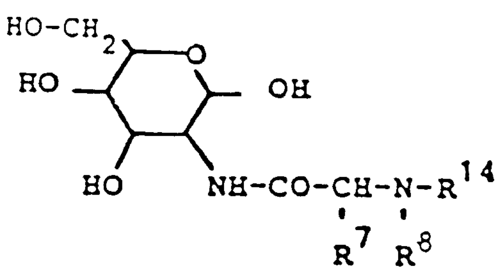

(XIII)

(dans laquelle $R^7$, $R^8$ et $R^{14}$ ont le sens indiqué à la revendication 6) avec des composés de formule (III)

$H_2N-R^1$ (III)

(dans laquelle
$R^1$ représente un reste alkyle saturé ou insaturé une ou plusieurs fois et comportant jusqu'à 50 atomes de carbone) pour obtenir des composés de formule (XIV)

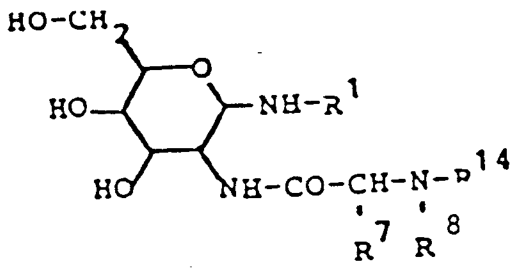

(XIV)

(dans laquelle $R^1$, $R^7$, $R^8$ et $R^{14}$ ont le sens indiqué ci-dessus ou dans la revendication 6), on fait réagir les composés de formule (XIV)

a) avec des dérivés d'acides carboxyliques convenables de formule (V)

$R^{11}$-CO-$CH_2$-$R^2$ (V)

dans laquelle

$R^2$ représente un atome d'hydrogène ou un reste alkyle saturé ou insaturé une ou plusieurs fois et comportant 1 à 50 atomes de carbone, et

$R^{11}$ représente un atome d'halogène ou un reste -O-CO-$R^2$ ou -O-CO-O-alkyle (en $C_1$-$C_5$),

ou

b) avec un ester d'acide halogénoformique (VII)

$R^{12}$-CO-O-$R^2$ (VII)

formule dans laquelle

$R^{12}$ représente un atome d'halogène, et

$R^2$ a le sens indiqué ci-dessus, ou

c) avec un isocyanate (VIII)

$R^2$-NCO (VIII)

dans lequel

$R^2$ a le sens indiqué ci-dessus,

et l'on fait réagir les composés ainsi obtenus, de formule (XII)

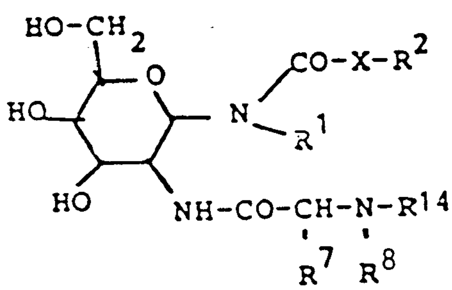

(XII)

dans laquelle $R^1$, $R^2$, X, $R^7$, $R^8$ et $R^{14}$ ont le sens indiqué ci-dessus ou à la revendication 6, en opérant comme indiqué à la revendication 6 pour obtenir les composés de formule (I).

**8.** Composés de formule générale I

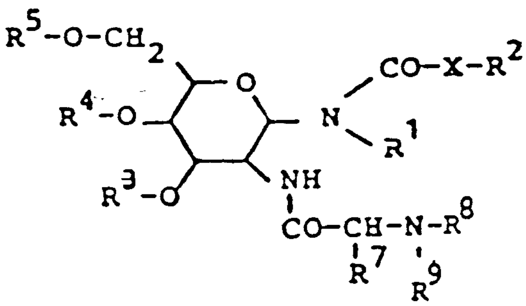

(I)

(dans laquelle

$R^1$ représente un reste alkyle saturé ou insaturé une ou plusieurs fois et comportant 1 à 50 atomes de carbone,

X représente $-CH_2-$, -O- ou -NH-,

$R^2$ représente un atome d'hydrogène ou un reste alkyle saturé ou insaturé une ou plusieurs fois et comportant jusqu'à 50 atomes de carbone,

$R^3$, $R^4$ et $R^5$ représentent chacun, indépendamment l'un de l'autre, un atome d'hydrogène ou un reste acyl-CO-$R^6$, dans lequel on entend désigner par $R^6$ un reste alkyle ayant jusqu'à 10 atomes de carbone,

$R^7$ représente un atome d'hydrogène, un groupe alkyle en $C_1$-$C_7$, hydroxyméthyle, 1-hydroxyéthyle, mercaptométhyle, 2-(méthylthio)-éthyle, 3-aminopropyle, 3-uréidopropyle, 3-guanidylpropyle, 4-aminobutyle, carboxyméthyle, carbamoylméthyle, 2-carboxyéthyle, 2-carbamoyléthyle, benzyle, 4-hydroxybenzyle, 3-indolylméthyle ou 4-imidazolylméthyle, et

$R^8$ représente un atome d'hydrogène ou un groupe méthyle, et

$R^9$ représente un atome d'hydrogène, un groupe acétyle, benzoyle, trichloracétyle, trifluoracétyle, méthoxycarbonyle, t-butyloxycarbonyle ou benzyloxycarbonyle, et

$R^7$ et $R^8$ peuvent, quand ils sont pris ensemble, représenter un groupe $-CH_2-CH_2-CH_2-$),

pour le traitement prophylactique et thérapeutique du corps humain ou animal.

**9.** Composés de formule générale I

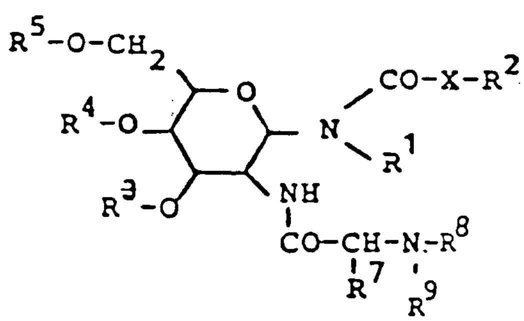

(I)

(dans laquelle

$R^1$ représente un reste alkyle saturé ou insaturé une ou plusieurs fois et comportant 1 à 50 atomes de carbone,

X représente $-CH_2-$, -O- ou -NH-,

$R^2$ représente un atome d'hydrogène ou un reste alkyle saturé ou insaturé une ou plusieurs fois et comportant jusqu'à 50 atomes de carbone,

$R^3$ $R^4$ et $R^5$ représentent chacun, indépendamment l'un de l'autre, un atome d'hydrogène ou un reste acyl-CO-$R^6$, dans lequel on entend désigner par $R^6$ un reste alkyle ayant jusqu'à 10 atomes de carbone,

$R^7$ représente un atome d'hydrogène, un groupe alkyle en $C_1$-$C_7$, hydroxyméthyle, 1-hydroxyéthyle, mercaptométhyle, 2-(méthylthio)-éthyle, 3-aminopropyle, 3-uréidopropyle, 3-guanidylpropyle, 4-aminobutyle, carboxyméthyle, carbamoylméthyle, 2-carboxyéthyle, 2-carbamoyléthyle, benzyle, 4-hydroxybenzyle, 3-indolylméthyle ou 4-

imidazolylméthyle, et

$R^8$ représente un atome d'hydrogène ou un groupe méthyle, et

$R^9$ représente un atome d'hydrogène, un groupe acétyle, benzoyle, trichloracétyle, trifluoracétyle, méthoxycarbonyle, t-butyloxycarbonyle ou benzyloxycarbonyle, et

$R^7$ et $R^8$ peuvent, quand ils sont pris ensemble, représenter un groupe $-CH_2-CH_2-CH_2-$),

sous forme L ou D ou sous forme de leurs sels, éventuellement internes, pharmaceutiquement utilisables.

**10.** Composés de formule XII

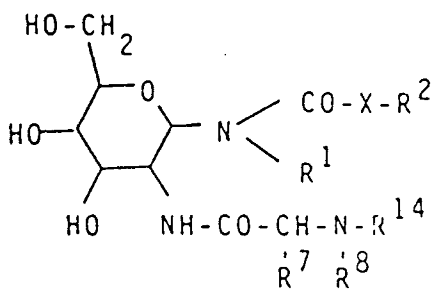

(XII)

dans laquelle $R^1$, $R^2$, $R^7$ et X ont le sens indiqué à la revendication 1,

$R^8$ représente un atome d'hydrogène ou un groupe méthyle, et

$R^{14}$ représente un groupe protecteur, utilisé habituellement dans la synthèse des peptides et pouvant être à nouveau scindé sélectivement en donnant la liaison peptide.

**11.** Composés de formule XV

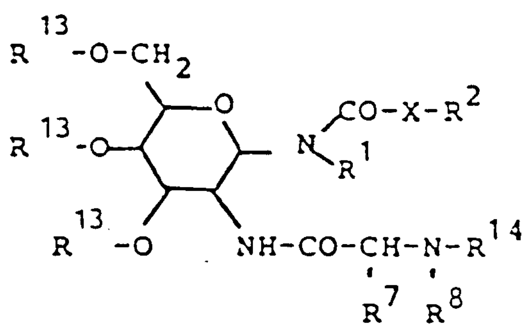

(XV)

dans laquelle

$R^1$, $R^2$, $R^7$, $R^8$, $R^{13}$, $R^{14}$ et X ont le sens indiqué à la revendication 10.

**12.** Médicaments contenant des composés de formule générale I

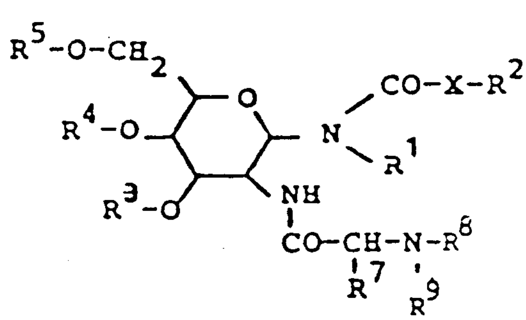

(I)

dans laquelle

$R^1$ représente un reste alkyle saturé ou insaturé une ou plusieurs fois et comportant 1 à 50 atomes de carbone,

X représente $-CH_2-$, -O- ou -NH-,

$R^2$ représente un atome d'hydrogène ou un reste alkyle saturé ou insaturé une ou plusieurs fois et comportant jusqu'à 50 atomes de carbone,

$R^3$, $R^4$ et $R^5$ représentent chacun, indépendamment l'un de l'autre, un atome d'hydrogène ou un reste acyl-CO-$R^6$, dans lequel on entend désigner par $R^6$ un reste alkyle ayant jusqu'à 10 atomes de carbone,

$R^7$ représente un atome d'hydrogène, un groupe alkyle en $C_1$-$C_7$, hydroxyméthyle, 1-hydroxyéthyle, mercaptométhyle, 2-(méthylthio)-éthyle, 3-aminopropyle, 3-uréidopropyle, 3-guanidylpropyle, 4-aminobutyle, carboxyméthyle, carbamoylméthyle, 2-carboxyéthyle, 2-carbamoyléthyle, benzyle, 4-hydroxybenzyle, 3-indolylméthyle ou 4-imidazolylméthyle, et

$R^8$ représente un atome d'hydrogène ou un groupe méthyle, et

$R^9$ représente un atome d'hydrogène, un groupe acétyle, benzoyle, trichloracétyle, trifluoracétyle, méthoxycarbonyle, t-butyloxycarbonyle ou benzyloxycarbonyle, et

$R^7$ et $R^8$ peuvent, quand ils sont pris ensemble, représenter un groupe $-CH_2-CH_2-CH_2-$.

**13.** Utilisation de composés de formule générale (I)

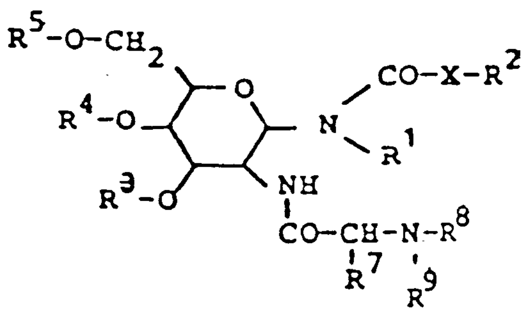

(I)

(dans laquelle

$R^1$ représente un reste alkyle saturé ou insaturé une ou plusieurs fois et comportant 1 à 50 atomes de carbone,

X représente $CH_2-$, -O- ou -NH-,

$R^2$ représente un atome d'hydrogène ou un reste alkyle saturé ou insaturé une ou plusieurs fois et comportant jusqu'à 50 atomes de carbone,

$R^3$, $R^4$ et $R^5$ représentent chacun, indépendamment l'un de l'autre, un atome d'hydrogène ou un reste acyl-CO-$R^6$, dans lequel on entend désigner par $R^6$ un reste alkyle ayant jusqu'à 10 atomes de carbone,

$R^7$ représente un atome d'hydrogène, un groupe alkyle en $C_1$-$C_7$, hydroxyméthyle, 1-hydroxyéthyle, mercaptométhyle, 2-(méthylthio)-éthyle, 3-aminopropyle, 3-uréidopropyle, 3-guanidylpropyle, 4-aminobutyle, carboxyméthyle, carbamoylméthyle, 2-carboxyéthyle, 2-carbamoyléthyle, benzyle, 4-hydroxybenzyle, 3-indolylméthyle ou 4-imidazolylméthyle, et

$R^8$ représente un atome d'hydrogène ou un groupe méthyle, et

$R^9$ représente un atome d'hydrogène, un groupe acétyle, benzoyle, trichloracétyle, trifluoracétyle, méthoxycarbonyle, t-butyloxycarbonyle ou benzyloxycarbonyle, et

$R^7$ et $R^8$ peuvent, quand ils sont pris ensemble, représenter un groupe $-CH_2-CH_2-CH_2-$),

pour préparer des médicaments.

**14.** Utilisation de composés de formule générale I

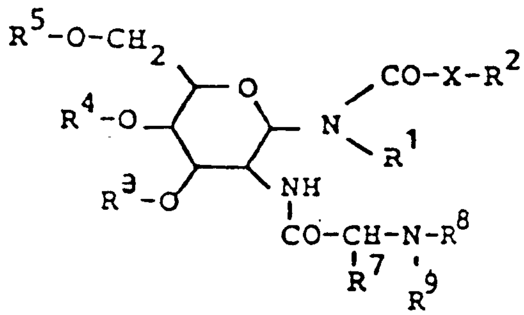

(I)

(dans laquelle

$R^1$ représente un reste alkyle saturé ou insaturé une ou plusieurs fois et comportant 1 à 50 atomes de carbone,

X représente $-CH_2-$, -O- ou -NH-,

$R^2$ représente un atome d'hydrogène ou un reste alkyle saturé ou insaturé une ou plusieurs fois et comportant jusqu'à 50 atomes de carbone,

$R^3$, $R^4$ et $R^5$ représentent chacun, indépendamment l'un de l'autre, un atome d'hydrogène ou un reste acyl-CO-$R^6$, dans lequel on entend désigner par $R^6$ un reste alkyle ayant jusqu'à 10 atomes de carbone,

$R^7$ représente un atome d'hydrogène, un groupe alkyle en $C_1$-$C_7$, hydroxyméthyle, 1-hydroxyéthyle, mercaptométhyle, 2-(méthylthio)-éthyle, 3-aminopropyle, 3-uréidopropyle, 3-guanidylpropyle, 4-aminobutyle, carboxyméthyle, carbamoylméthyle, 2-carboxyéthyle, 2-carbamoyléthyle, benzyle, 4-hydroxybenzyle, 3-indolylméthyle ou 4-imidazolylméthyle, et

$R^8$ représente un atome d'hydrogène ou un groupe méthyle, et

$R^9$ représente un atome d'hydrogène, un groupe acétyle, benzoyle, trichloracétyle, trifluoracétyle, méthoxycarbonyle, t-butyloxycarbonyle ou benzyloxycarbonyle, et

$R^7$ et $R^8$ peuvent, quand ils sont pris ensemble, représenter un groupe $-CH_2-CH_2-CH_2-$),

pour préparer des médicaments pour combattre des maladies du corps humain ou animal.

**15.** Utilisation des composés selon la revendication 1 pour la préparation de médicaments pour augmenter la défense propre du corps.

**16.** Utilisation de composés selon la revendication 1, comme adjuvants avec des antigènes pour préparer des antisérums pour le diagnostic et le traitement thérapeutique de maladies infectieuses.

**17.** Vaccins contenant des composés de formule générale I

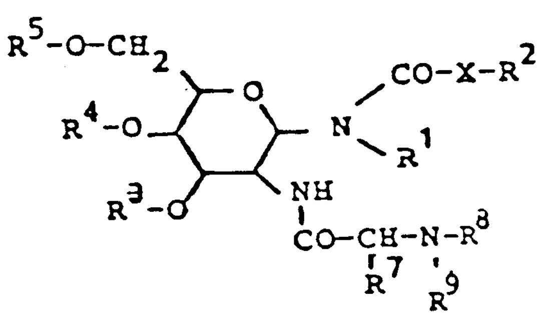

(I)

dans laquelle

$R^1$ représente un reste alkyle saturé ou insaturé une ou plusieurs fois et comportant 1 à 50 atomes de carbone,

X représente $-CH_2-$, -O- ou -NH-,

$R^2$ représente un atome d'hydrogène ou un reste alkyle saturé ou insaturé une ou plusieurs fois et comportant jusqu'à 50 atomes de carbone,

$R^3$, $R^4$ et $R^5$ représentent chacun, indépendamment l'un de l'autre, un atome d'hydrogène ou un reste acyl-CO-$R^6$, dans lequel on entend désigner par $R^6$ un reste alkyle ayant jusqu'à 10 atomes de carbone,

$R^7$ représente un atome d'hydrogène, un groupe alkyle en $C_1$-$C_7$, hydroxyméthyle, 1-hydroxyéthyle, mercaptométhyle, 2-(méthylthio)-éthyle, 3-aminopropyle, 3-uréidopropyle, 3-guanidylpropyle, 4-aminobutyle, carboxyméthyle, carbamoylméthyle, 2-carboxyéthyle, 2-carbamoyléthyle, benzyle, 4-hydroxybenzyle, 3-indolylméthyle ou 4-imidazolylméthyle, et

$R^8$ représente un atome d'hydrogène ou un groupe méthyle, et

$R^9$ représente un atome d'hydrogène, un groupe acétyle, benzoyle, trichloracétyle, trifluoracétyle, méthoxycarbonyle, t-butyloxycarbonyle ou benzyloxycarbonyle, et

$R^7$ et $R^8$ peuvent, quand ils sont pris ensemble, représenter un groupe $-CH_2-CH_2CH_2-$.

**18.** Utilisation de composés de formule générale I

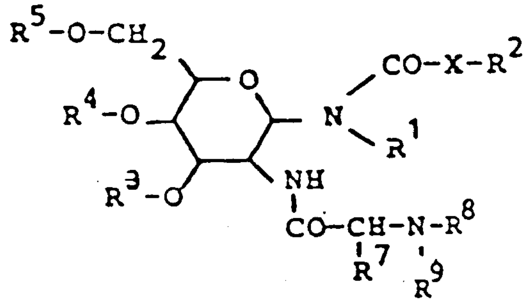

(I)

(dans laquelle

$R^1$ représente un reste alkyle saturé ou insaturé une ou plusieurs fois et comportant 1 à 50 atomes de carbone,

X représente $-CH_2-$, -O- ou -NH-,

$R^2$ représente un atome d'hydrogène ou un reste alkyle saturé ou insaturé une ou plusieurs fois et comportant jusqu'à 50 atomes de carbone,

$R^3$, $R^4$ et $R^5$ représentent chacun, indépendamment l'un de l'autre, un atome d'hydrogène ou un reste acyl-CO-$R^6$, dans lequel on entend désigner par $R^6$ un reste alkyle ayant jusqu'à 10 atomes de carbone,

$R^7$ représente un atome d'hydrogène, un groupe alkyle en $C_1$-$C_7$, hydroxyméthyle, 1-hydroxyéthyle, mercaptométhyle, 2-(méthylthio)-éthyle, 3-aminopropyle, 3-uréidopropyle, 3-guanidylpropyle, 4-aminobutyle, carboxyméthyle, carbamoylméthyle, 2-carboxyéthyle, 2-carbamoyléthyle, benzyle, 4-hydroxybenzyle, 3-indolylméthyle ou 4-imidazolylméthyle, et

$R^8$ représente un atome d'hydrogène ou un groupe méthyle, et

$R^9$ représente un atome d'hydrogène, un groupe acétyle, benzoyle, trichloracétyle, trifluoracétyle, méthoxycarbonyle, t-butyloxycarbonyle ou benzyloxycarbonyle, et

$R^7$ et $R^8$ peuvent, quand ils sont pris ensemble, représenter un groupe $-CH_2-CH_2-CH_2-$),

comme adjuvants dans la préparation de vaccins à base de virus contre des infections dues au virus de la grippe ou influenza, des oreillons, de la rougeôle, de la rubéole, de l'hépatite, de l'herpes et autres virus de maladies humaines.

**19.** Utilisation de composés de formule générale I

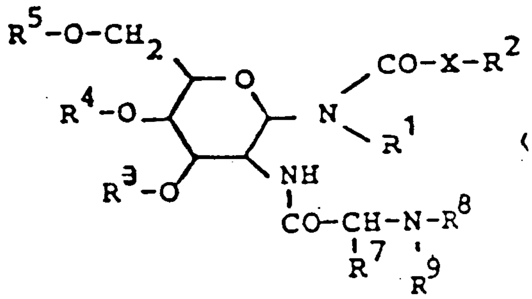

(I)

dans laquelle

$R^1$ représente un reste alkyle saturé ou insaturé une ou plusieurs fois et comportant 1 à 50 atomes de carbone,

X représente $-CH_2-$, -O- ou -NH-,

$R^2$ représente un atome d'hydrogène ou un reste alkyle saturé ou insaturé une ou plusieurs fois et comportant jusqu'à 50 atomes de carbone,

$R^3$, $R^4$ et $R^5$ représentent chacun, indépendamment l'un de l'autre, un atome d'hydrogène ou un reste acyl-CO-$R^6$, dans lequel on entend désigner par $R^6$ un reste alkyle ayant jusqu'à 10 atomes de carbone,

$R^7$ représente un atome d'hydrogène, un groupe alkyle en $C_1$-$C_7$, hydroxyméthyle, 1-hydroxyéthyle, mercaptométhyle, 2-(méthylthio)-éthyle, 3-aminopropyle, 3-uréidopropyle, 3-guanidylpropyle, 4-aminobutyle, carboxyméthyle, carbamoylméthyle, 2-carboxyéthyle, 2-carbamoyléthyle, benzyle, 4-hydroxybenzyle, 3-indolylméthyle ou 4-imidazolylméthyle, et

$R^8$ représente un atome d'hydrogène ou un groupe méthyle, et

$R^9$ représente un atome d'hydrogène, un groupe acétyle, benzoyle, trichloracétyle, trifluoracétyle, méthoxycarbonyle, t-butyloxycarbonyle ou benzyloxycarbonyle, et

$R^7$ et $R^8$ peuvent, quand ils sont pris ensemble, représenter un groupe $-CH_2-CH_2-CH_2-$,

comme adjuvants pour la préparation de vaccins à virus contre des infections dues à des virus de maladies animales du type virus pseudorabique, de rhinopneumonie, de Marek, de fièvre aphteuse, de grippe du gros bétail.